# EUROPEAN PATENT APPLICATION

(11) **EP 4 843 622 A2**
(43) Date of publication of application: **25.11.2026**
(21) Application number: 26210391.4
(22) Date of filing: 28.10.2021
(51) Int. Cl.: A61P 9/00

(54) **DRY POWDER COMPOSITIONS OF TREPROSTINIL PRODRUGS AND METHODS OF USE THEREOF**

(30) Priority: 28.10.2020 US 202063106818 P
(62) Document number(s) of the earlier application(s) in accordance with Art. 76 EPC: 21887529.2 / 4 236 934
(71) Applicant: Insmed Incorporated, Bridgewater, NJ 08807 (US)
(72) Inventor: DU, Ju, Bridgewater, 08807-1704 (US); PLAUNT, Adam, Bridgewater, 08807-1704 (US); MALININ, Vladimir, Bridgewater, 08807-1704 (US); PARIKH, Maulikkumar, Bridgewater, 08807-1704 (US); AMIN, Harshh, Bridgewater, 08807-1704 (US); PALWAI, Naveen, Bridgewater, 08807-1704 (US); O'BRIEN, Gerald, Bridgewater, 08807-1704 (US); ISMAT, Fraz, Bridgewater, 08807-1704 (US); TEPER, Ariel, Bridgewater, 08807-1704 (US); SULLIVAN, Eugene, Bridgewater, 08807-1704 (US); FERNANDEZ, Carlos, Bridgewater, 08807-1704 (US)
(74) Representative: Cooley (UK) LLP

(57) **Abstract**

The present disclosure provides dry powder compositions of treprostinil prodrugs and methods of treating pulmonary hypertension (e.g., pulmonary arterial hypertension or PH associated with interstitial lung disease), in a patient in need thereof with the same. The dry powder composition includes (a) from about 0.5 wt% to about 5 wt% of a compound of a stereoisomer thereof, or a pharmaceutically acceptable salt thereof, (b) from about 10 wt% to about 61 wt% of leucine, and the balance being (c) a sugar selected from the group consisting of trehalose and mannitol. The entirety of (a), (b), and (c) is 100 wt%, and R1 is tetradecyl, pentadecyl, hexadecyl, heptadecyl, or octadecyl. The method of treating PH includes administering an effective amount of the dry powder composition to the lungs of the patient by inhalation via a dry powder inhaler, during an administration period.

## Description

### CROSS REFERENCE TO RELATED APPLICATION

This application claims priority from U.S. Provisional Application Serial No. 63/106,818, filed October 28, 2020, the disclosure of which is incorporated by reference herein in its entirety.

### BACKGROUND OF THE INVENTION

Pulmonary hypertension (PH) is characterized by an abnormally high blood pressure in the lung vasculature. It is a progressive, lethal disease that leads to heart failure and can occur in the pulmonary artery, pulmonary vein, or pulmonary capillaries. Symptomatic patients experience shortness of breath, dizziness, fainting, and other symptoms, all of which are made worse by exertion. There are multiple causes, and can be of unknown origin, idiopathic, and can lead to hypertension in other systems, for example, portopulmonary hypertension in which patients have both portal and pulmonary hypertension.

Pulmonary hypertension has been classified into five groups by the World Health Organization (WHO). Group 1 is called pulmonary arterial hypertension (PAH), and includes PAH that has no known cause (idiopathic), inherited PAH (*i.e*., familial PAH or FPAH), PAH that is caused by drugs or toxins, and PAH caused by conditions such as connective tissue diseases, HIV infection, liver disease, and congenital heart disease. Group 2 pulmonary hypertension is characterized as pulmonary hypertension associated with left heart disease. Group 3 pulmonary hypertension is characterized as PH associated with lung diseases, such as chronic obstructive pulmonary disease and interstitial lung diseases, as well as PH associated with sleep-related breathing disorders (*e.g*., sleep apnea). Group 4 PH is PH due to chronic thrombotic and/or embolic disease, *e.g*., PH caused by blood clots in the lungs or blood clotting disorders. Group 5 includes PH caused by other disorders or conditions, *e.g.*, blood disorders (*e.g*., polycythemia vera, essential thrombocythemia), systemic disorders (*e.g*., sarcoidosis, vasculitis), and metabolic disorders (*e.g*., thyroid disease, glycogen storage disease).

Pulmonary arterial hypertension (PAH) afflicts approximately 200,000 people globally with approximately 30,000-40,000 of those patients in the United States. PAH patients experience constriction of pulmonary arteries which leads to high pulmonary arterial pressures, making it difficult for the heart to pump blood to the lungs. Patients suffer from shortness of breath and fatigue which often severely limits the ability to perform physical activity.

The New York Heart Association (NYHA) has categorized PAH patients into four functional classes to rate the severity of the disease. Class I PAH patients as categorized by the NYHA do not have a limitation of physical activity, as ordinary physical activity does not cause undue dyspnoea or fatigue, chest pain, or near syncope. Class II PAH patients as categorized by the NYHA have a slight limitation on physical activity. These patients are comfortable at rest, but ordinary physical activity causes undue dyspnoea or fatigue, chest pain or near syncope. Class III PAH patients as categorized by the NYHA have a marked limitation of physical activity. Although comfortable at rest, class III PAH patients experience undue dyspnoea or fatigue, chest pain or near syncope as a result of less than ordinary physical activity. Class IV PAH patients as categorized by the NYHA are unable to carry out any physical activity without symptoms. Class IV PAH patients might experience dyspnoea and/or fatigue at rest, and discomfort is increased by any physical activity. Signs of right heart failure are often manifested by class IV PAH patients.

Patients with PAH are treated with an endothelin receptor antagonist (ERA), phosphodiesterase type 5 (PDE-5) inhibitor, a guanylate cyclase stimulator, a prostanoid (*e.g*., prostacyclin), or a combination thereof. ERAs include abrisentan (Letairis^{®}), sitaxentan, bosentan (Tracleer^{®}), and macitentan (Opsumit^{®}). PDE-5 inhibitors indicated for the treatment of PAH include sildenafil (Revatio^{®}) and tadalafil (Adcirca^{®}). Prostanoids indicated for the treatment of PAH include iloprost, epoprosentol and treprostinil (Remodulin^{®}, Tyvaso^{®}). The one approved guanylate cyclase stimulator is riociguat (Adempas^{®}). Additionally, patients are often treated with combinations of the aforementioned compounds.

The present invention addresses the need for novel treatment options for pulmonary hypertension (PH) (including pulmonary arterial hypertension (PAH) and PH associated with interstitial lung disease), portopulmonary hypertension (PPH), and pulmonary fibrosis by providing dry powder compositions of treprostinil prodrugs useful for pulmonary administration, and methods for administering the same to patients in need of treatment.

### SUMMARY OF THE INVENTION

In one aspect, the present disclosure relates to a dry powder composition comprising (a) from about 0.5 wt% to about 5 wt% of a compound of Formula (I): 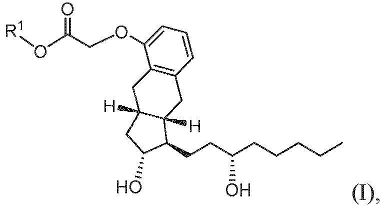 a stereoisomer thereof, or a pharmaceutically acceptable salt thereof, wherein R¹ is tetradecyl, pentadecyl, hexadecyl, heptadecyl, or octadecyl; (b) from about 10 wt% to about 61 wt% of leucine, and the balance being (c) a sugar selected from the group consisting of trehalose and mannitol. The entirety of (a), (b), and (c), is 100 wt%. In a further embodiment, the composition includes from about 29 wt% to about 61 wt% of leucine. In even a further embodiment, the composition comprises 0.5 wt% to about 4 wt% of the compound of Formula (I), a stereoisomer thereof, or a pharmaceutically acceptable salt thereof.

The stereoisomer, in one embodiment, is a diastereomer of a compound of Formula (I), or a pharmaceutically acceptable salt thereof. In a further embodiment, the stereoisomer is a diastereomer of a compound of Formula (I). In another embodiment, the stereoisomer is a diastereomer of a pharmaceutically acceptable salt of a compound of Formula (I).

In one embodiment, R¹ is tetradecyl. In a further embodiment, R¹ is linear tetradecyl.

In one embodiment, R¹ is pentadecyl. In a further embodiment, R¹ is linear pentadecyl.

In one embodiment, R¹ is heptadecyl. In a further embodiment, R¹ is linear heptadecyl.

In one embodiment, R¹ is octadecyl. In a further embodiment, R¹ is linear octadecyl.

In one embodiment, R¹ is hexadecyl. In a further embodiment, R¹ is linear hexadecyl.

In one embodiment, the compound of Formula (I), a stereoisomer thereof, or a pharmaceutically acceptable salt thereof, is present at from about 0.5 wt% to about 4 wt% of the total weight of the dry powder composition. In a further embodiment, R¹ is hexadecyl. In even a further embodiment, R¹ is linear hexadecyl. In even a further embodiment, the compound of Formula (I), or a pharmaceutically acceptable salt thereof is present at from about 2 wt% to about 4 wt% of the total weight of the dry powder composition.

In one embodiment, the compound of Formula (I), a stereoisomer thereof, or a pharmaceutically acceptable salt thereof, is present at from about 1 wt% to about 4 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 1 wt% to about 3.5 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 1 wt% to about 3 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 1.5 wt% to about 4 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 0.8 wt% to about 4 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 0.8 wt% to about 3.3 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at from about 1 wt% to about 2 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at from about 1 wt% to about 1.5 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at about 1 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at about 2 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at about 3 wt% of the total weight of the dry powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at about 4 wt% of the total weight of the dry powder composition. In a further embodiment, R¹ is hexadecyl. In even a further embodiment, R¹ is linear hexadecyl.

In one embodiment, the leucine is present at from about 20 wt% to about 40 wt% of the total weight of the dry powder composition. In a further embodiment, R¹ is hexadecyl. In even a further embodiment, R¹ is linear hexadecyl. In even a further embodiment, the compound of Formula (I), a stereoisomer thereof, or a pharmaceutically acceptable salt thereof, is present at from about 1 wt% to about 4 wt% of the total weight of the dry powder composition.

In another embodiment, the leucine is present at from about 29 wt% to about 61 wt% of the total weight of the dry powder composition. In a further embodiment, R¹ is hexadecyl. In even a further embodiment, R¹ is linear hexadecyl. In even a further embodiment, the compound of Formula (I), a stereoisomer thereof, or a pharmaceutically acceptable salt thereof, is present at from about 1 wt% to about 4 wt% of the total weight of the dry powder composition.

In another embodiment, the leucine is present at from about 25 wt% to about 35 wt% of the total weight of the dry powder composition. In a further embodiment, R¹ is hexadecyl. In even a further embodiment, R¹ is linear hexadecyl. In even a further embodiment, the compound of Formula (I), a stereoisomer thereof, or a pharmaceutically acceptable salt thereof, is present at from about 1 wt% to about 4 wt% of the total weight of the dry powder composition.

In another embodiment, the leucine is present at about 40 wt% to 61 wt% of the total weight of the dry powder composition. In a further embodiment, R¹ is hexadecyl. In even a further embodiment, R¹ is linear hexadecyl. In even a further embodiment, the compound of Formula (I), a stereoisomer thereof, or a pharmaceutically acceptable salt thereof, is present at from about 1 wt% to about 4 wt% of the total weight of the dry powder composition. In a further embodiment, the leucine is present at about 45 wt% to 61 wt% of the total weight of the dry powder composition. In even a further embodiment, the leucine is present at about 55 wt% to 61 wt% of the total weight of the dry powder composition.

In another embodiment, the leucine is present at from about 28 wt% to about 33 wt% of the total weight of the dry powder composition. In a further embodiment, R¹ is hexadecyl. In even a further embodiment, R¹ is linear hexadecyl. In a further embodiment, the compound of Formula (I), or a pharmaceutically acceptable salt thereof, is present at from about 1 wt% to about 4 wt% of the total weight of the dry powder composition.

In another embodiment, the leucine is present at from about 25 wt% to about 33 wt% of the total weight of the dry powder composition, for example, at from about 27 wt% to about 33 wt%, from about 27 wt% to about 31 wt%, from about 27 wt% to about 30 wt%, from about 28 wt% to about 30 wt%, or at about 30 wt% of the total weight of the dry powder composition. In a further embodiment, R¹ is hexadecyl. In even a further embodiment, R¹ is linear hexadecyl.

In one embodiment, the dry powder composition provided herein has a leucine-to-mannitol weight ratio of about 0.40-to-1 (leucine-to-mannitol) to about 0.50-to-1 (leucine-to-mannitol). In another embodiment, the dry powder composition provided herein has a leucine-to-mannitol weight ratio of about 0.75-to-1 (leucine-to-mannitol) to about 0.90-to-1 (leucine-to-mannitol). In yet another embodiment, the dry powder composition provided herein has a leucine-to-mannitol weight ratio of about 0 about 1.5-to-1 (leucine-to-mannitol) to about 1.7-to-1 (leucine-to-mannitol).

In one embodiment, the sugar is mannitol. In a further embodiment, R¹ is hexadecyl. In a further embodiment, R¹ is linear hexadecyl.

In one embodiment, the dry powder composition includes (a) about 1 wt% of the compound of Formula (I), a stereoisomer thereof, or a pharmaceutically acceptable salt thereof, (b) about 29.3 wt% or about 29.6 wt% of the leucine, and the balance being (c) mannitol. In a further embodiment, R¹ is hexadecyl. In a further embodiment, R¹ is linear hexadecyl.

In one embodiment, the dry powder composition includes (a) about 3 wt% of the compound of Formula (I), a stereoisomer thereof, or a pharmaceutically acceptable salt thereof, (b) about 29.3 wt% or about 29.6 wt% of the leucine, and the balance being (c) mannitol. In a further embodiment, R¹ is hexadecyl. In a further embodiment, R¹ is linear hexadecyl.

In another aspect of the invention, a method for treating pulmonary hypertension (PH) in a patient in need thereof is provided. The method includes administering an effective amount of the dry powder composition disclosed herein to the lungs of the patient by inhalation via a dry powder inhaler.

In one embodiment, the PH is group 1 PH, as characterized by the World Health Organization (WHO).

The pulmonary hypertension, in one embodiment, is pulmonary arterial hypertension (PAH). The PAH, in one embodiment, is class I PAH, as characterized by the New York Heart Association (NYHA). In another embodiment, the PAH is class II PAH, as characterized by NYHA. In another embodiment, the PAH is class III PAH, as characterized by NYHA. In another embodiment, the PAH is class IV PAH, as characterized by NYHA.

In another embodiment, the PH is group 2 PH, as characterized by the WHO. In another embodiment, the PH is group 3 PH, as characterized by the WHO. In a further embodiment, the group 3 PH is PH associated with interstitial lung disease (ILD). In another embodiment, the PH is group 4 PH, as characterized by the WHO. In another embodiment, the PH is group 5 PH, as characterized by the WHO.

In one embodiment of the treatment methods described herein, the administering is conducted in a once-a-day or twice-a-day.

In still another aspect, the present disclosure relates to a system for treating PH. The system includes one of the dry powder compositions disclosed herein and a dry powder inhaler (DPI), which may be single dose or a multidose inhaler. In another embodiment, the DPI is pre-metered or device-metered.

Yet another aspect of the invention relates to a method of treating PH (e.g., PAH or PH-ILD) an adult human patient in need thereof, comprising administering once daily during an administration period, to the lungs of the patient by inhalation, a dry powder composition comprising from about 80 µg to about 675 µg of a compound of Formula (I): 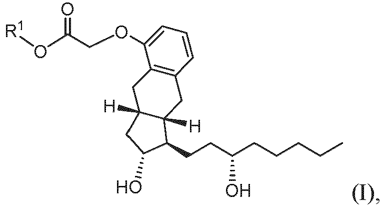 a stereoisomer thereof, or a pharmaceutically acceptable salt thereof,
wherein R¹ is tetradecyl, pentadecyl, hexadecyl, heptadecyl, or octadecyl,
wherein during the administration period, the patient has at least one of the following characteristics:
   (a) a treprostinil maximum plasma concentration (Cₘₐₓ) ranging from about 80% to about 125% of the range of from about 17 pg/mL to about 1150 pg/mL; or
   (b) a treprostinil area under the plasma concentration curve (AUC_{0-inf}) from about 80% to about 125% of the range of about 475 pg*h/mL to about 8000 pg*h/mL. In a further embodiment, R¹ is hexadecyl, e.g., linear hexadecyl.

In a further embodiment, the composition comprises a dose selected from the group consisting of 80 µg, 160 µg, 240 µg, 320 µg, 400 µg, 480 µg and 640 µg of a compound of Formula (I). The dose can be present, e.g., in one dry powder capsule, or multiple capsules.

In another aspect, the present relates to a dry powder composition, comprising from about 80 µg to about 675 µg of a compound of Formula (I): 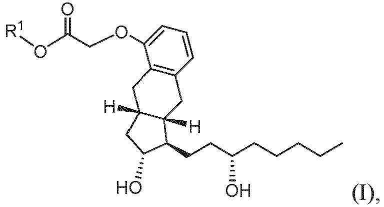 a stereoisomer thereof, or a pharmaceutically acceptable salt thereof. In this aspect, the dry powder composition provides at least one of the following characteristics:
(a) a maximum treprostinil plasma concentration (Cₘₐₓ) of from about 80% to about 125% of the range of from about 17 pg/mL to about 1150 pg/mL; or
(b) an area under the plasma concentration curve (AUC_{0-inf}) from about 80% to about 125% of the range of about 475 pg*h/mL to about 8000 pg*h/mL.

In a further embodiment, the composition comprises a dose selected from the group consisting of 80 µg, 160 µg, 240 µg, 320 µg, 400 µg, 480 µg and 640 µg of a compound of Formula (I). The dose can be present, e.g., in one dry powder capsule, or multiple capsules.

In some embodiments, the dry powder composition described herein and used in the methods described herein comprises from about 1 wt% to about 5 wt% of a compound of Formula (I) or (II), or a pharmaceutically acceptable salt thereof, with the balance being one or more pharmaceutically acceptable excipients which are suitable for use in a dry powder inhaler. In some embodiments, the one or more pharmaceutically acceptable excipients which are suitable for use in a dry powder inhaler comprise sugar, amino acid, and optionally distearoyl phosphoethanoamine-polyethylene glycol 2000 (DPSE-PEG2000). In some embodiments of the dry powder compositions or methods described herein, the dry powder composition comprises from about 25 wt% to about 61 wt% of leucine, with the balance being one or more sugars. In some embodiments, the one or more sugars are selected from trehalose and mannitol. In some embodiments of the dry powder compositions or methods described herein, the dry powder composition does not include distearoylphosphoethanoamine-polyethylene glycol 2000 (DPSE-PEG2000).

### BRIEF DESCRIPTION OF THE FIGURES

Figure 1 is a graph showing the concentration of treprostinil palmitil (TP) in the lung after TPIP-A or TPIP-B is inhaled.
Figure 2 is a graph showing the concentration of TRE in the lung after TPIP-A or TPIP-B is inhaled.
Figure 3 is a graph showing the concentration of treprostinil palmitil (TP) equivalent in the lung after TPIP-A or TPIP-B is inhaled.
Figure 4 is a graph showing the concentration of TRE in plasma after TPIP-A or TPIP-B is inhaled.
Figure 5 is a graph showing the concentration of TP in BAL cell fraction after TPIP-A or TPIP-B is inhaled.
Figure 6 is a graph showing the concentration of TRE in BAL cell fraction after TPIP-A or TPIP-B is inhaled.
Figure 7 is a graph showing the concentration of TP equivalent in BAL cell fraction after TPIP-A or TPIP-B is inhaled.
Figure 8 is a graph showing the concentration of TP in BAL fluid after TPIP-A or TPIP-B is inhaled.
Figure 9 is a graph showing the concentration of TRE in BAL fluid after TPIP-A or TPIP-B is inhaled.
Figure 10 is a graph showing the concentration of TP equivalent in BAL fluid after TPIP-A or TPIP-B is inhaled.

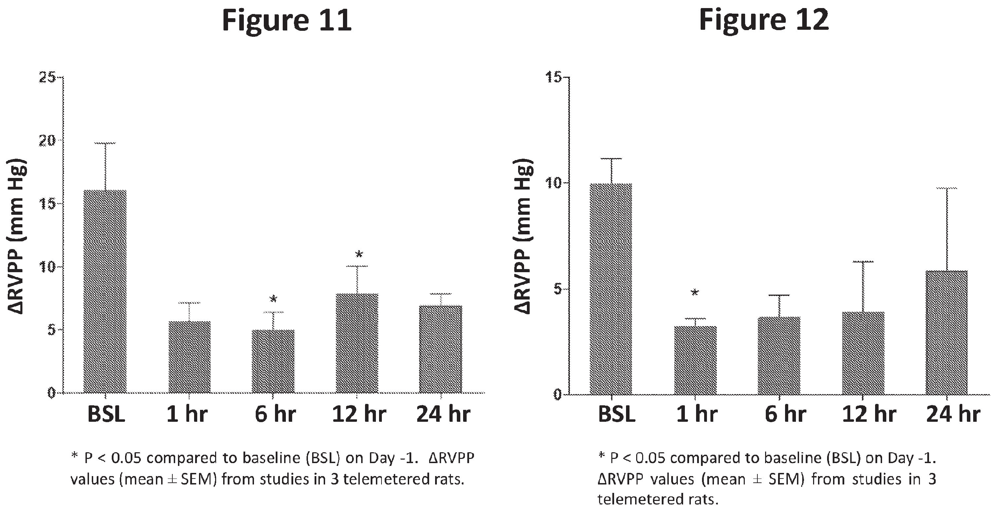

Figure 11 is a graph showing the ΔRVPP response to hypoxic challenge in rats exposed to inhaled TPIP-B at 6 µg/kg.
Figure 12 is a graph showing the ΔRVPP response to hypoxic challenge in rats exposed to inhaled TPIP-B at 23 µg/kg.

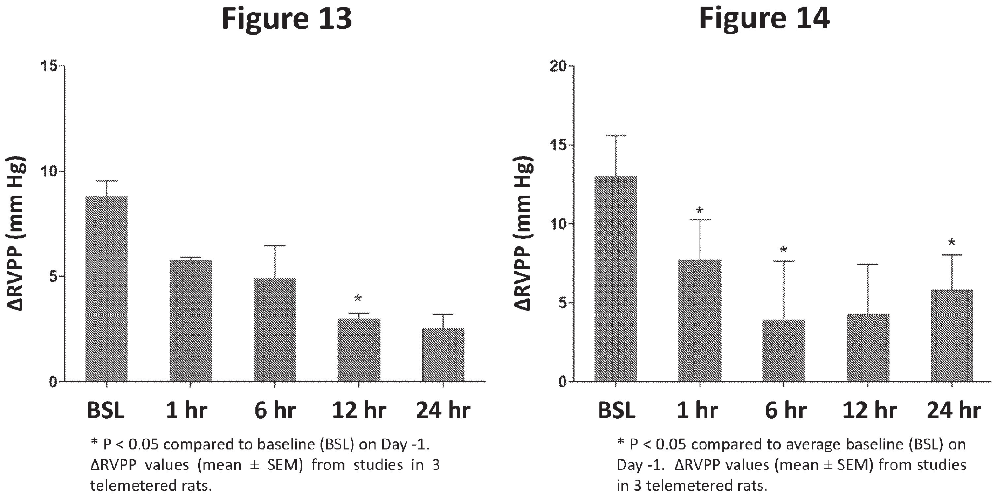

Figure 13 is a graph showing the ΔRVPP response to hypoxic challenge in rats exposed to inhaled TPIP-B at 57 µg/kg.
Figure 14 is a graph showing the ΔRVPP response to hypoxic challenge in rats exposed to inhaled TPIP-B at 138 µg/kg.

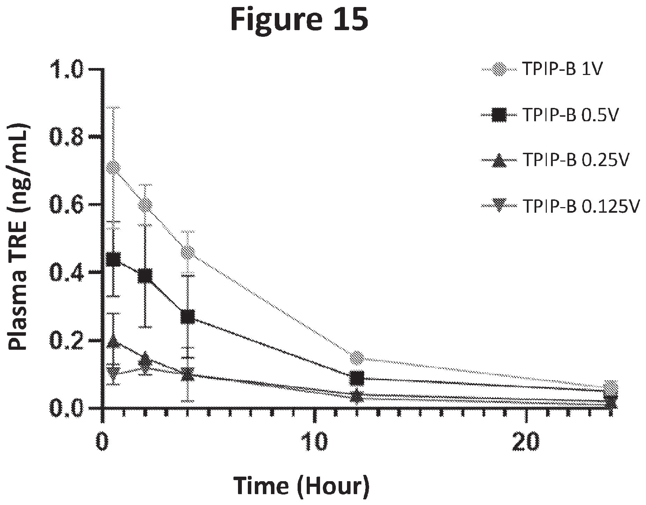

Figure 15 is a graph showing the TRE concentration in plasma after TPIP-B is inhaled.

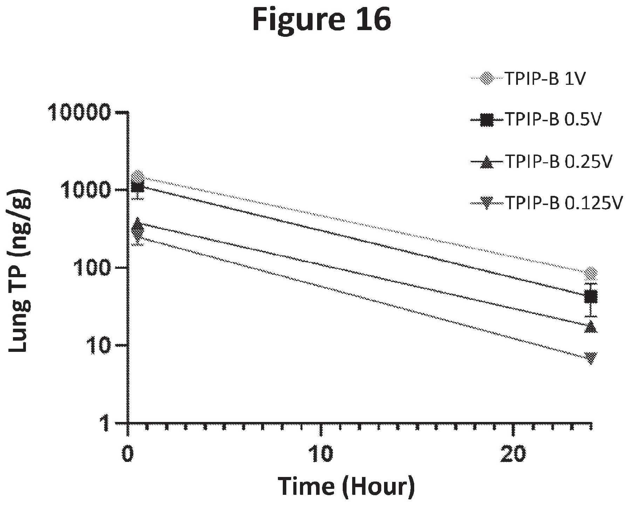

Figure 16 is a graph showing the TP concentration in the lung after TPIP-B is inhaled.

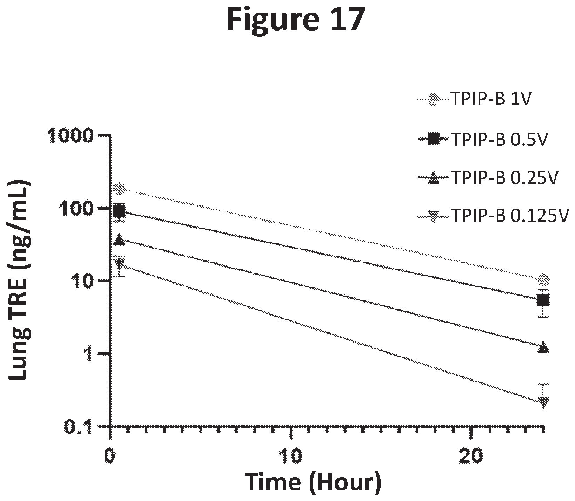

Figure 17 is a graph showing the TRE concentration in the lung after TPIP-B is inhaled.

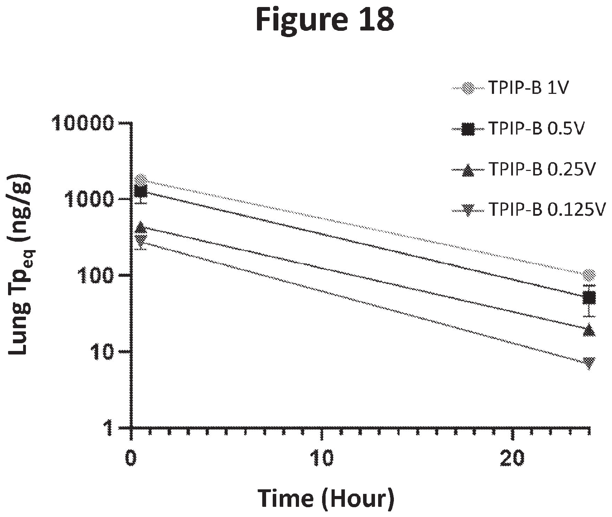

Figure 18 is a graph showing the TP equivalent concentration in the lung after TPIP-B is inhaled.
Figure 19 is a schematic of the study design for testing the pharmacokinetic (PK) profile of single and multiple daily dosing of TPIP-B in healthy adults. D: day; PK: pharmacokinetic; QD: once daily; Scn: screening; TPIP: treprostinil palmitil inhalation powder.

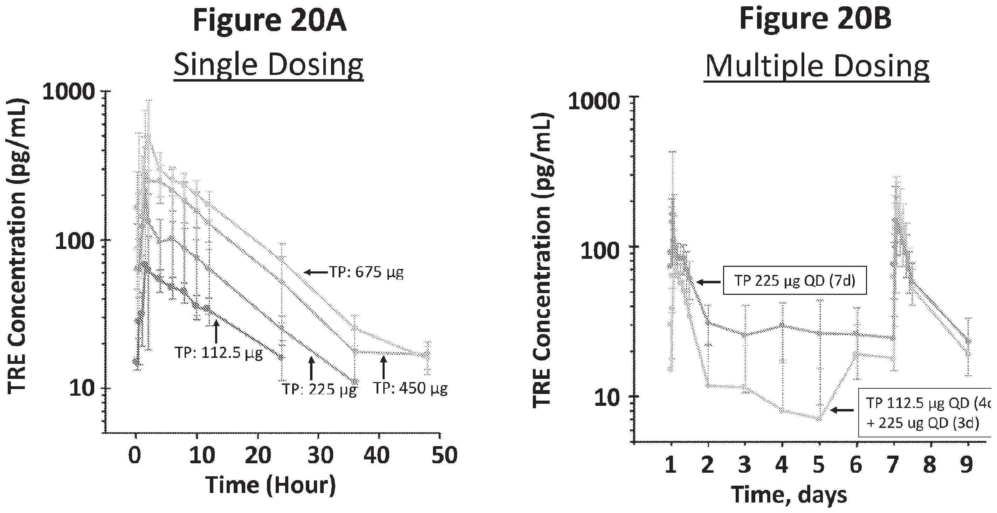

Figure 20A is a graph showing the PK results of TPIP-A in healthy adults (Single Dose).
Figure 20B is a graph showing the PK findings of TPIP-A in healthy adults (Multiple Doses).
Figure 21, top, shows one embodiment of a dose titration schedule of a compound of Formula (I) or (II). Figure 21, bottom, shows the capsule doses used according to the titration schedule in the top portion of Figure 21.

### DETAILED DESCRIPTION OF THE INVENTION

Throughout the present disclosure, the term "about" may be used in conjunction with numerical values and/or ranges. The term "about" is understood to mean those values near to a recited value. For example, "about 40 [units]" may mean within ± 25% of 40 (e.g., from 30 to 50), within ± 20%, ± 15%, ± 10%, ± 9%, ± 8%, ± 7%, ± 6%, ± 5%, ± 4%, ± 3%, ± 2%, ± 1 %, less than ± 1%, or any other value or range of values therein or there below.

The term "pharmaceutically acceptable salt" refers to salts prepared from pharmaceutically acceptable non-toxic bases or acids including inorganic or organic bases and inorganic or organic acids. The nature of the salt is not critical, provided that it is pharmaceutically acceptable. Suitable pharmaceutically acceptable acid addition salts may be prepared from an inorganic acid or from an organic acid. Exemplary pharmaceutical salts are disclosed in Stahl, P.H., Wermuth, C.G., Eds. Handbook of Pharmaceutical Salts: Properties, Selection and Use; Verlag Helvetica Chimica Acta/Wiley-VCH: Zurich, 2002, the contents of which are hereby incorporated by reference in their entirety. Specific non-limiting examples of inorganic acids are hydrochloric, hydrobromic, hydroiodic, nitric, carbonic, sulfuric and phosphoric acid. Appropriate organic acids include, without limitation, aliphatic, cycloaliphatic, aromatic, arylaliphatic, and heterocyclyl containing carboxylic acids and sulfonic acids, for example formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, *p*-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, cyclohexylaminosulfonic, algenic, 3-hydroxybutyric, galactaric or galacturonic acid. Suitable pharmaceutically acceptable salts of free acid-containing compounds disclosed herein include, without limitation, metallic salts and organic salts. Exemplary metallic salts include, but are not limited to, appropriate alkali metal (group Ia) salts, alkaline earth metal (group IIa) salts, and other physiological acceptable metals. Such salts can be made from aluminum, calcium, lithium, magnesium, potassium, sodium and zinc. Exemplary organic salts can be made from primary amines, secondary amines, tertiary amines and quaternary ammonium salts, for example, tromethamine, diethylamine, tetra-*N-*methylammonium, *N,N*'-dibenzylethylenediamine, chloroprocaine, choline, diethanolamine, ethylenediamine, meglumine (*N*-methylglucamine) and procaine.

The term "stereoisomer" as used herein refers to two molecules having the same molecular formula and sequence of bonded atoms, but differ in three-dimensional orientations of their atoms in space. One preferred stereoisomer according to the present invention is a diastereomer. The stereoisomer, in one embodiment, is a diastereomer of a compound of Formula (I), or a pharmaceutically acceptable salt thereof. In a further embodiment, the stereoisomer is a diastereomer of a compound of Formula (I). In another embodiment, the stereoisomer is a diastereomer of a pharmaceutically acceptable salt of a compound of Formula (I). In yet another embodiment, the stereoisomer is a diastereomer of a compound of Formula (II). In even another embodiment, the stereoisomer is a diastereomer of a pharmaceutically acceptable salt of a compound of Formula (II).

Throughout the present specification, numerical ranges are provided for certain quantities. It is to be understood that these ranges comprise all subranges therein. Thus, the range "50-80" includes all possible ranges therein (e.g., 51-79, 52-78, 53-77, 54-76, 55-75, 60-70, etc.). Furthermore, all values within a given range may be an endpoint for the range encompassed thereby (e.g., the range 50-80 includes the ranges with endpoints such as 55-80, 50-75, etc.).

Throughout the present specification, numerical ranges are described as encompassing "about 80% to about 125%" or "about 80-125%" of a range of values. It is to be understood that these comprise 80% of the lowest endpoint of the range up to 125% of the highest endpoint of the range, and all values therein.

The term "Cₘₐₓ" means the maximum (or peak) treprostinil serum concentration measured after a compound of Formula (I) or (II), a stereoisomer thereof, or a pharmaceutically acceptable salt thereof is administered to the lungs of a subject via a dry powder composition described herein. In addition, Cₘₐₓ may be measured after a single administration of a compound of Formula (I) or (II), a stereoisomer thereof, or a pharmaceutically acceptable salt thereof, described herein, or treprostinil Cₘₐₓ may be measured at steady state. Unless stated otherwise, Cₘₐₓ refers to the average treprostinil Cₘₐₓ measured after a single administration among a population of subjects (e.g., a population of PH patients).

The term "AUC" means the area under the plasma concentration time curve for treprostinil, measured from time 0 to a certain time post-administration to the lungs of a subject, calculated by a combination of linear and logarithmic trapezoidal methods (Linear up/log down method). In some embodiments, AUC may be measured from time 0 to 24 hours post-administration ("AUC₀₋₂₄") or AUC may be measured form from time 0 to extrapolated to infinity ("AUC₀₋ᵢₙₜ"). In addition, treprostinil AUC may be measured after a single administration or at steady state values. Unless stated otherwise, AUC refers to the average AUC measured after a single administration among a population of subjects (e.g., a population of PH patients).

The term "plasma trough concentration" refers to the treprostinil plasma concentration before administering a subsequent dose of the compounds of Formula (I) or (II), a stereoisomer thereof, or a pharmaceutically acceptable salt thereof. For example, treprostinil plasma trough concentration may be measured within 2 hours, 1 hour, or 30 minutes of administering a subsequent dose. Plasma trough concentrations may be measured after a single administration or may be measured at steady state. Unless stated otherwise, plasma trough levels refer to the average treprostinil trough level measured among a population of subjects (e.g., a population of PH patients).

The term "adult" refers to a human subject, e.g., a human patient that is at least 18 years of age or older. In some embodiments, the adult is 18-100 years of age, e.g., 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, including all values and ranges in between.

In one aspect of the present invention, a dry powder composition of a treprostinil prodrug is provided. The dry powder composition comprises:
(a) a compound of Formula (I) or a pharmaceutically acceptable salt thereof, present at from about 0.5 wt% to about 5 wt% of the total weight of the dry powder composition: 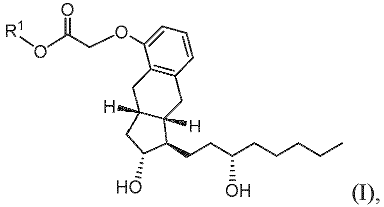 wherein R¹ is tetradecyl, pentadecyl, hexadecyl, heptadecyl, or octadecyl;
(b) from about 10 wt% to about 61 wt% of leucine, and the balance being
(c) a sugar selected from the group consisting of trehalose and mannitol. The entirety of (a), (b), and (c) is 100 wt%.

In a further embodiment, the composition comprises from about 25 wt% to about 61 wt% of leucine. In even a further embodiment, the composition comprises from about 25 wt% to about 45 wt% of leucine. In another embodiment, the composition comprises from about 45 wt% to about 61 wt% of leucine.

In some embodiments, the compound of Formula (I), a stereoisomer thereof, or a pharmaceutically acceptable salt thereof is present at about 0.4 wt% about 0.5 wt%, about 1 wt%, about 1.1 wt%, about 1.2 wt%, about 1.3 wt%, about 1.5 wt%, about 1.7 wt%, about 2.0 wt%, about 2.3 wt%, about 2.5 wt%, about 2.6 wt%, about 2.7 wt%, about 2.8 wt%, about 2.9 wt%, about 3 wt%, about 3.1 wt%, about 3.2 wt%, about 3.3 wt%, about 3.4 wt%, about 3.5 wt%, about 4 wt%, about 3.5 wt%, or about 5 wt% of the total weight of the dry powder composition.

The compound of Formula (I) and pharmaceutically acceptable salts thereof are treprostinil prodrugs as disclosed in International Application Publication WO 2015/061720, the disclosure of which is incorporated herein by reference in its entirety. In some embodiments, the leucine is present at about 25 wt%, about 30 wt%, about 35 wt%, about 40 wt%, about 45 wt%, about 50 wt%, about 55 wt%, or about 60 wt% of the total weight of the dry powder composition.

In one embodiment of the compound of Formula (I), a stereoisomer thereof, or a pharmaceutically acceptable salt thereof, R¹ is tetradecyl. In a further embodiment, R¹ is linear tetradecyl.

In another embodiment of the compound of Formula (I), a stereoisomer thereof, or a pharmaceutically acceptable salt thereof, R¹ is pentadecyl. In a further embodiment, R¹ is linear pentadecyl.

In another embodiment of the compound of Formula (I), a stereoisomer thereof, or a pharmaceutically acceptable salt thereof, R¹ is heptadecyl. In a further embodiment, R¹ is linear heptadecyl.

In another embodiment of the compound of Formula (I), a stereoisomer thereof, or a pharmaceutically acceptable salt thereof, R¹ is octadecyl. In a further embodiment, R¹ is linear octadecyl.

In another embodiment of the compound of Formula (I), a stereoisomer thereof, or a pharmaceutically acceptable salt thereof, R¹ is hexadecyl. In a further embodiment, R¹ is linear hexadecyl, i.e., the compound of Formula (I), a stereoisomer thereof, or a pharmaceutically acceptable salt thereof, is a compound of Formula (II): 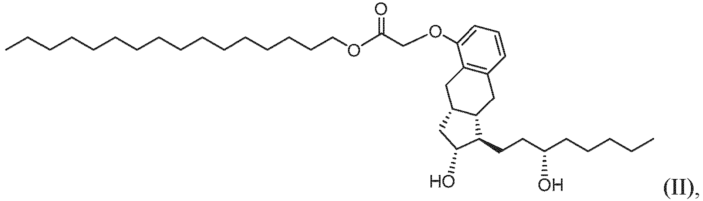 a stereoisomer thereof, or a pharmaceutically acceptable salt thereof. In a further embodiment, the compound of Formula (I) is a compound of Formula (II). The compound of Formula (I) where R¹ is linear hexadecyl is also referred to herein as C16TR or its international nonproprietary name, treprostinil palmitil. In the present application, C16TR and treprostinil palmitil are used interchangeably. Similarly, a compound of Formula (II) is equivalent to a compound of Formula (I), wherein R¹ is linear hexadecyl.

In one embodiment, (a) is a compound of Formula (I) or a pharmaceutically acceptable salt thereof. In a further embodiment, (a) is a compound of Formula (II) or a pharmaceutically acceptable salt thereof. In a further embodiment, (a) is a compound of Formula (II).

In one embodiment, the compound of Formula (I) or (II), or a pharmaceutically acceptable salt thereof, is present at from about 1 wt% to about 5 wt% of the total weight of the dry powder composition. In some embodiments, the compound of Formula (I) or (II), or a pharmaceutically acceptable salt thereof, is present at from about 1 wt% to about 4.5 wt% of the total weight of the dry powder composition. In some embodiments, the compound of Formula (I) or (II) is present at from about 1 wt% to about 4 wt% of the total weight of the dry powder composition.

In one embodiment, the compound of Formula (I) or (II), or a pharmaceutically acceptable salt thereof, is present at from about 1 wt% to about 3.5 wt% of the total weight of the dry powder composition. In another embodiment, the compound of Formula (I) or (II), or a pharmaceutically acceptable salt thereof, is present at from about 1 wt% to about 3 wt% of the total weight of the dry powder composition.

In one embodiment, the compound of Formula (I) or (II), or a pharmaceutically acceptable salt thereof, is present at from about 1 wt% to about 5 wt%, from about 1 wt% to about 4.5 wt%, from about 1 wt% to about 4 wt%, at about 2 wt%, at about 3 wt%, at about 4 wt%, or at about 5 wt%, of the total weight of the dry powder composition. In some embodiments, the compound of Formula (I) or (II), or a pharmaceutically acceptable salt thereof, is present at from about 1 wt% to about 5 wt%, from about 1 wt% to about 4.5 wt%, from about 1 wt% to about 4 wt%, from about 1 wt% to about 2 wt%, about 2 wt%, or about 4 wt%, of the total weight of the dry powder composition.

In one embodiment, the compound of Formula (I) or (II), or a pharmaceutically acceptable salt thereof is present at from about 0.8 wt% to about 3.3 wt%, or from about 1 wt% to about 3 wt%, or from about 1 wt% to about 2 wt%, or from about 1 wt% to about 1.5% of the total weight of the dry powder composition.

In one embodiment, the compound of Formula (I) or (II), or a pharmaceutically acceptable salt thereof is present at about 1 wt% of the total weight of the dry powder composition. In another embodiment, the compound of Formula (I) or (II), or a pharmaceutically acceptable salt thereof is present at about 1.5 wt% of the total weight of the dry powder composition.

In one embodiment, the compound of Formula (I) or (II), or a pharmaceutically acceptable salt thereof, is present from about 0.8 wt% to about 1.5 wt% of the total weight of the dry powder composition. In another embodiment, the compound of Formula (I) or (II), or a pharmaceutically acceptable salt thereof is present from about 2.7 wt% to about 4 wt% of the total weight of the dry powder composition. In one embodiment, the compound of Formula (I) or (II), or a pharmaceutically acceptable salt thereof is present from about 2.7 wt% to about 3.5 wt%, for example, from about 2.8 wt% to about 3.2 wt%, or from about 2.9 wt% to about 3.1 wt% of the total weight of the dry powder composition.

In one embodiment, the leucine is present at from about 25 wt% to about 61 wt% of the total weight of the dry powder composition. In a further embodiment, the leucine is present at from about 25 wt% to about 50 wt% of the total weight of the dry powder composition. In a further embodiment, the leucine is present at from about 25 wt% to about 40 wt% of the total weight of the dry powder composition. In a further embodiment, the leucine is present at from about 20 wt% to about 33 wt%, e.g., about 20 wt%, about 25 wt%, about 26 wt%, about 27 wt%, about 28 wt%, about 29 wt%, about 30 wt%, about 31 wt%, about 32 wt%, or about 33 wt% of the total weight of the dry powder composition. In a further embodiment, the leucine is present at from about 25 wt% to about 33 wt% of the total weight of the dry powder composition. In a further embodiment, the leucine is present at from about 27 wt% to about 33 wt% of the total weight of the dry powder composition. In a further embodiment, the leucine is present at from about 27 wt% to about 31 wt% of the total weight of the dry powder composition. In a further embodiment, the leucine is present at from about 27 wt% to about 30 wt% of the total weight of the dry powder composition. In a further embodiment, the leucine is present at from about 28 wt% to about 30 wt% of the total weight of the dry powder composition.

In another embodiment, the leucine is present at about 30 wt% of the total weight of the dry powder composition.

In yet another embodiment, the leucine is present at from about 45 wt% to about 61 wt% of the total weight of the dry powder composition, for example at from about 45 wt% to about 55 wt%, or from about 50 wt% to about 55 wt%. In a further embodiment, the compound of Formula (I), or a pharmaceutically acceptable salt thereof, is present at about 3 wt% to about 4 wt% of the total weight of the dry powder composition. In even a further embodiment, R¹ is hexadecyl, e.g., linear hexadecyl.

In some embodiments, the sugar in the dry powder composition is trehalose. In another embodiment, the sugar in the dry powder composition is mannitol.

In one embodiment, the composition has the weight percentages set forth in Table A, below. In another embodiment, the composition has the weight percentages set forth in Table A, below, ± 5% for each component. In yet another embodiment, the composition has a leucine-to-mannitol weight ratio ("leucine : mannitol" or "leucine-to-mannitol") set forth in Table A.

| **Table A.** Exemplary TPIP compositions. | | | | |
|---|---|---|---|---|
| | **TP (%w)** | **Leucine (%w)** | **Mannitol (%w)** | **Leucine-to-Mannitol Weight Ratio** |
| **1** | 0.5 | 60.0 | 39.5 | 1.52-to-1 |
| **2** | 2.0 | 61.2 | 36.8 | 1.66-to-1 |
| **3** | 3.0 | 60.7 | 36.3 | 1.67-to-1 |
| **4** | 4.0 | 60.0 | 36.0 | 1.67-to-1 |
| **5** | 0.4 | 45.0 | 54.6 | 0.82-to-1 |
| **6** | 1.5 | 44.4 | 54.1 | 0.82-to-1 |
| **7** | 2.0 | 45.0 | 53.0 | 0.85-to-1 |
| **8** | 3.0 | 44.5 | 52.5 | 0.85-to-1 |
| **9** | 4.0 | 45.0 | 51.0 | 0.88-to-1 |
| **10** | 0.5 | 30.0 | 69.5 | 0.43-to-1 |
| **11** | 1.0 | 29.3 | 69.7 | 0.42-to-1 |
| **12** | 1.5 | 29.6 | 68.9 | 0.43-to-1 |
| **13** | 1.5 | 29.3 | 69.2 | 0.42-to-1 |
| **14** | 2.0 | 28.8 | 69.2 | 0.42-to-1 |
| **15** | 3.0 | 28.6 | 68.4 | 0.42-to-1 |
| **16** | 4.0 | 30.0 | 66.0 | 0.45-to-1 |

In one embodiment, the dry powder composition has the components and weight percentages set forth in Table B.

| **Table B.** Exemplary TPIP composition. | |
|---|---|
| | **Quantity (%w)** |
| **TP (drug substance)** | 1.0 |
| **Leucine (dispersion enhancer)** | 29.3 |
| **Mannitol (bulking agent)** | 69.7 |
| **Filled Capsule Weight** | 100 |

The leucine-to-sugar (i.e., mannitol or trehalose) weight ratio in a composition provided herein, in one embodiment, is from about 0.4-to-1 (leucine-to-mannitol or -trehalose) to about 1.7-to-1 (leucine-to-mannitol -trehalose). In a further embodiment, the composition comprises a compound of Formula (I), or a pharmaceutically acceptable salt thereof, at from about 1 wt% to about 4 wt% of the total weight of the dry powder composition. In a further embodiment, the leucine-to-sugar weight ratio is from about 0.4:1 (leucine-to-mannitol or - trehalose) to 0.9:1 (leucine-to-mannitol or -trehalose). In even a further embodiment, the leucine-to-sugar weight ratio is from about 0.4:1 (leucine-to-mannitol or -trehalose) to 0.5:1 (leucine-to-mannitol or -trehalose). In a further embodiment, the sugar is mannitol. The leucine, in one embodiment, is L-leucine.

In another embodiment, the sugar is mannitol and the leucine-to-mannitol weight ratio is from about 0.75-to-1 (leucine-to-mannitol) to 0.9-to-1 (leucine-to-mannitol). In a further embodiment, the composition comprises a compound of Formula (I), or a pharmaceutically acceptable salt thereof, at from about 1 wt% to about 4 wt% of the total weight of the dry powder composition. In a further embodiment, the leucine-to-mannitol weight ratio is from about 0.8:1 (leucine-to-mannitol) to 0.9:1 (leucine-to-mannitol). In another embodiment, the sugar is trehalose and the leucine-to-trehalose weight ratio is from about 0.75:1 (leucine-to-trehalose) to 0.9:1 (leucine-to-trehalose). In a further embodiment, the composition comprises a compound of Formula (I), or a pharmaceutically acceptable salt thereof, at from about 1 wt% to about 4 wt% of the total weight of the dry powder composition. In a further embodiment, the leucine-to- trehalose weight ratio is from about 0.8:1 (leucine-to-trehalose) to 0.9:1 (leucine-to-trehalose). The leucine, in one embodiment, is L-leucine.

In yet another embodiment, the sugar is mannitol and the leucine-to-mannitol weight ratio is from about 1.5:1 (leucine-to-mannitol) to 1.7:1 (leucine-to-mannitol). In a further embodiment, the composition comprises a compound of Formula (I), or a pharmaceutically acceptable salt thereof, at from about 1 wt% to about 4 wt% of the total weight of the dry powder composition. In a further embodiment, the leucine-to-mannitol weight ratio is from about 1.6:1 (leucine-to-mannitol) to 1.7:1 (leucine-to-mannitol). In yet another embodiment, the sugar is trehalose and the leucine-to-trehalose weight ratio is from about 1.5:1 (leucine-to-trehalose) to 1.7:1 (leucine-to-trehalose). In a further embodiment, the composition comprises a compound of Formula (I), or a pharmaceutically acceptable salt thereof, at from about 1 wt% to about 4 wt% of the total weight of the dry powder composition. In a further embodiment, the leucine-to-mannitol weight ratio is from about 1.6:1 (leucine-to-trehalose) to 1.7:1 (leucine-to-trehalose).

In another embodiment, the dry powder composition includes (a) about 1-2 wt% of the compound of Formula (I) or (II), a stereoisomer thereof, or a pharmaceutically acceptable salt thereof, (b) about 29 wt% of the leucine, and the balance being (c) mannitol. In a further embodiment, (a) in the dry powder composition is about 1 wt% of the compound of Formula (I) or (II), or a pharmaceutically acceptable salt thereof. In another embodiment, (a) in the dry powder composition is at about 2 wt% of the compound of Formula (I) or (II), or a pharmaceutically acceptable salt thereof.

In another embodiment, the dry powder composition includes (a) about 1.5 wt% of the compound of Formula (I) or (II), or a pharmaceutically acceptable salt thereof, (b) about 29.6 wt% of the leucine, and the balance being (c) mannitol. In a further embodiment, R¹ is linear hexadecyl in the compound of Formula (I).

In another embodiment, the dry powder composition includes (a) about 3 wt% of the compound of Formula (I) or (II), a stereoisomer thereof, or a pharmaceutically acceptable salt thereof, (b) about 29 wt% of the leucine, and the balance being (c) mannitol. In a further embodiment, R¹ is linear hexadecyl in the compound of Formula (I).

In another embodiment, the dry powder composition includes (a) about 3 wt% of the compound of Formula (I) or (II), a stereoisomer thereof, or a pharmaceutically acceptable salt thereof, (b) about 29 wt% of the leucine, and the balance being (c) mannitol. In a further embodiment, R¹ is linear hexadecyl in the compound of Formula (I).

In another embodiment, the dry powder composition includes (a) about 1 wt% of the compound of Formula (I) or (II), a stereoisomer thereof, or a pharmaceutically acceptable salt thereof, (b) about 29 wt% of leucine, and the balance being (c) mannitol. In a further embodiment, R¹ is linear hexadecyl in the compound of Formula (I).

In another embodiment, the dry powder composition includes (a) about 1 wt% of the compound of Formula (I) or (II), a stereoisomer thereof, or a pharmaceutically acceptable salt thereof, (b) about 29.6 wt% of the leucine, and the balance being (c) mannitol. In a further embodiment, R¹ is linear hexadecyl in the compound of Formula (I).

In some embodiments, the dry powder composition does not include distearoyl phosphoethanoamine-polyethylene glycol 2000 (DPSE-PEG2000).

In one embodiment, the dry powder composition comprises from about 80 µg to about 700 µg of a compound of Formula (I) or (II), for example, about 80 µg, about 100 µg, about 110 µg, about 112.5 µg, about 120 µg, about 130 µg, about 140 µg, about 150 µg, about 160 µg, about 170 µg, about 180 µg, about 190 µg, about 200 µg, about 210 µg, about 220 µg, about 225 µg, about 230 µg, about 240 µg, about 250 µg, about 260 µg, about 270 µg, about 280 µg, about 290 µg, about 300 µg, about 310 µg, about 320 µg, about 330 µg, about 340 µg, about 350 µg, about 360 µg, about 370 µg, about 380 µg, about 390 µg, about 400 µg, about 410 µg, about 420 µg, about 430 µg, about 440 µg, about 450 µg, about 460 µg, about 470 µg, about 480 µg, about 490 µg, about 500 µg, about 510 µg, about 520 µg, about 530 µg, about 540 µg, about 550 µg, about 560 µg, about 570 µg, about 580 µg, about 590 µg, about 600 µg, about 610 µg, about 620 µg, about 630 µg, about 640 µg, about 650 µg, about 660 µg, about 670 µg, about 675 µg, about 680 µg, about 690 µg, or about 700 µg of a compound of Formula (I), a stereoisomer thereof, or a pharmaceutically acceptable salt thereof, including all values and ranges therein. In one embodiment, the dry powder composition comprises from about 80 µg to about 640 µg of a compound of Formula (I) or (II). In one embodiment, the composition comprises about 80 µg, about 160 µg, about 240 µg, about 320 µg, about 400 µg, about 480 µg or about 640 µg of a compound of Formula (I). The composition may be present, in one embodiment, in one dry powder capsule or a plurality (two or more) dry powder capsules. When present in multiple capsules, one of the aforementioned doses of the compound of Formula (I) is divided amongst the capsules. The capsule, in one embodiment, is a size #3 HPMC capsule.

Embodiments of a TPIP composition at different unit strengths are provided in Table C, below. It should be understood that the unit strengths of the components provided herein can be calculated based on the weight percentages of the component and the desired dosage. For example, for an 80 µg dose of TP, each component is multiplied by 80 to obtain the unit strength of each component.

| **Table C.** TPIP filled capsule embodiments. | | | | |
|---|---|---|---|---|
| **Component** | **Quantity (%w)** | **Unit Strength (µg** / **capsule)** | | |
| | | 80 | 160 | 320 |
| TP (drug substance) | 1.0 | 80 | 160 | 320 |
| Leucine (dispersion enhancer) | 29.3 | 2344 | 4688 | 9376 |
| Mannitol (bulking agent) | 69.7 | 5576 | 11152 | 22304 |
| Filled Capsule Weight | 100 | 8 mg | 16 mg | 32 mg |

In one embodiment, the dry powder composition comprises about 80 µg of the compound of Formula (I) or a pharmaceutically acceptable salt thereof. In a further embodiment, R¹ is hexadecyl. In even a further embodiment, R¹ is linear hexadecyl.

In one embodiment, the dry powder composition comprises about 160 µg of the compound of Formula (I) or a pharmaceutically acceptable salt thereof. In a further embodiment, R¹ is hexadecyl. In even a further embodiment, R¹ is linear hexadecyl.

In another embodiment, the dry powder composition comprises about 240 µg of the compound of Formula (I) or a pharmaceutically acceptable salt thereof. In a further embodiment, R¹ is hexadecyl. In even a further embodiment, R¹ is linear hexadecyl.

In one embodiment, the dry powder composition comprises about 320 µg of the compound of Formula (I), a stereoisomer thereof, or a pharmaceutically acceptable salt thereof. In a further embodiment, R¹ is hexadecyl. In even a further embodiment, R¹ is linear hexadecyl.

In another embodiment, the dry powder composition comprises about 400 µg of the compound of Formula (I) or a pharmaceutically acceptable salt thereof. In a further embodiment, R¹ is hexadecyl. In even a further embodiment, R¹ is linear hexadecyl.

In another embodiment, the dry powder composition comprises about 480 µg of the compound of Formula (I) or a pharmaceutically acceptable salt thereof. In a further embodiment, R¹ is hexadecyl. In even a further embodiment, R¹ is linear hexadecyl.

In one embodiment, the dry powder composition comprises about 640 µg of the compound of Formula (I) or a pharmaceutically acceptable salt thereof. In a further embodiment, R¹ is hexadecyl. In even a further embodiment, R¹ is linear hexadecyl.

In a preferred embodiment of the dry powder composition provided herein, the leucine is L-leucine.

In another aspect, the present disclosure provides a dry powder composition comprising a compound of Formula (I) or (II), or a pharmaceutically acceptable salt thereof, which provides a particular pharmacokinetic profile following once daily administration. Advantageously, pharmacokinetic profile has a lower Cₘₐₓ and longer half-life compared to the current treprostinil inhaled solution, Tyvaso^{®}.

In one embodiment, the dry powder composition exhibiting one of the pharmacokinetic profiles described herein is a composition described in U.S. Patent Application Publication No. 2020/0338005, incorporated by reference herein in its entirety for all purposes.

In another embodiment, the dry powder composition exhibiting one of the pharmacokinetic profiles described herein comprises (a) a compound of Formula (I) or (II) at from about 1 wt% to about 5 wt% of the total weight of the dry powder composition; (b) from about 25 wt% to about 61 wt% of leucine, and the balance being (c) a sugar selected from trehalose and mannitol. The entirety of (a), (b), and (c) is 100 wt%.

As discussed in Example 5, the pharmacokinetic (PK) profile measured for the compound of Formula (I) or (II), a stereoisomer thereof, or a pharmaceutically acceptable salt thereof, was linear over the dose range of 112.5 µg to 675 µg. Based on this data, the skilled artisan can determine the pharmacokinetic parameters of doses outside of the range, or doses inside this range that were not specifically tested in the Example 5. For example, in order to find a pharmacokinetic parameter at a dose, Cmax and AUC associated with specific doses (112.5 µg, 225 µg, 450 µg, and/or 675 µg) may be plotted. The scatter plot may be fit to a straight line, y= mx + b, where m is the slope of the line, b is the y intercept, and the value of an unknown pharmacokinetic parameter (y) may be calculated by plugging in the dose for x. In addition, the dose range of 112.5 µg to 675 µg was based on the molecular weight of the compound of Formula (I) when R¹ is hexadecyl (i.e., the compound of Formula (II)). Equivalent doses for other treprostinil prodrugs (when R¹ is tetradecyl, pentadecyl, heptadecyl, or octadecyl) can be calculated using the molecular weight of the treprostinil prodrug of interest. For example, the dose of the compound of Formula (I) when R¹ is tetradecyl that is equivalent to 112.5 µg of the compound of Formula II (R¹ is hexadecyl) can be calculated by multiplying 112.5 µg by the ratio of the molecular weight of the compound of Formula (II) (614.95 µg/mol) to the molecular weight of the compound of Formula (I) when R¹ is tetradecyl (586.9 µg/mol).

In embodiments, the dry powder composition of the disclosure is formulated to administer once daily to the lungs of a subject by inhalation a dose ranging from about 80 µg to about 675 µg of the compound of Formula (I) or (II), a stereoisomer thereof, or a pharmaceutically acceptable salt thereof, and provide at least one of the following characteristics:
(a) a treprosintil maximum plasma concentration (Cₘₐₓ) ranging from about 14 pg/mL to about 1430 pg/mL; or
(b) a treprostinil area under the plasma concentration curve (AUC) ranging from about from about 500 pg*h/mL to about 10000 pg*h/mL.

In a further embodiment, the composition comprises about 80 µg, about 112.5 µg, about 160 µg, about 225 µg, about 240 µg, about 320 µg, about 400 µg, about 450 µg, about 480 µg, about 640 µg, or about 675 µg of a compound of Formula (I). In a further embodiment, the composition comprises about 80 µg, about 160 µg, about 240 µg, about 320 µg, about 400 µg, about 480 µg, or about 640 µg of a compound of Formula (I). In a further embodiment, R¹ is hexadecyl, e.g., linear hexadecyl. The composition may be present, in one embodiment, in one dry powder capsule or a plurality (two or more) dry powder capsules. When present in multiple capsules, one of the aforementioned doses of the compound of Formula (I) is divided amongst the capsules.

In embodiments, the dry powder composition of the disclosure is formulated to administer once daily to the lungs of the subject (e.g., patient) by inhalation a dose ranging from about 80 µg to about 640 µg of the compound of Formula (II), or a pharmaceutically acceptable salt thereof, and provide at least one of the following characteristics:
(a) a treprosintil maximum plasma concentration (Cₘₐₓ) ranging from about 14 pg/mL to about 1430 pg/mL; or
(b) a treprostinil area under the plasma concentration curve (AUC) ranging from about from about 380 pg*h/mL to about 10000 pg*h/mL.

In a further embodiment, the composition comprises about 80 µg, about 160 µg, about 240 µg, about 320 µg, about 400 µg, about 480 µg or about 640 µg of a compound of Formula (II). The composition may be present, in one embodiment, in one dry powder capsule or a plurality (two or more) dry powder capsules. When present in multiple capsules, one of the aforementioned doses of the compound of Formula (II) is divided amongst the capsules.

In one embodiment, the dry powder composition is formulated to administer once daily to the lungs of the subject (e.g., patient) by inhalation a dose ranging from about 112.5 µg to about 675 µg of the compound of Formula (II), a stereoisomer thereof, or a pharmaceutically acceptable salt thereof, and provide at least one of the following characteristics:
(a) a treprosintil maximum plasma concentration (Cₘₐₓ) ranging from about 17 pg/mL to about 1370 pg/mL; or
(b) a treprostinil area under the plasma concentration curve (AUC) ranging from about from about 700 pg*h/mL to about 7800 pg*h/mL.

In a further embodiment, the composition comprises about 80 µg, about 160 µg, about 240 µg, about 320 µg, about 400 µg, about 480 µg or about 640 µg of a compound of Formula (II). The composition may be present, in one embodiment, in one dry powder capsule or a plurality (two or more) dry powder capsules. When present in multiple capsules, one of the aforementioned doses of the compound of Formula (II) is divided amongst the capsules.

In embodiments, the dry powder composition comprises having one of the pharmacokinetic profiles described herein comprises about 80 µg to about 675 µg of a compound of Formula (I), for example from about 80 µg to about 640 µg or from about 112.5 µg to about 675 µg. In one embodiment, the dry powder composition having one of the pK profiles described herein comprises about 80 µg, about 100 µg, about 110 µg, about 112.5 µg, about 120 µg, about 130 µg, about 140 µg, about 150 µg, about 160 µg, about 170 µg, about 180 µg, about 190 µg, about 200 µg, about 210 µg, about 220 µg, about 225 µg, about 230 µg, about 240 µg, about 250 µg, about 260 µg, about 270 µg, about 280 µg, about 290 µg, about 300 µg, about 310 µg, about 320 µg, about 330 µg, about 340 µg, about 350 µg, about 360 µg, about 370 µg, about 380 µg, about 390 µg, about 400 µg, about 410 µg, about 420 µg, about 430 µg, about 440 µg, about 450 µg, about 460 µg, about 470 µg, about 480 µg, about 490 µg, about 500 µg, about 510 µg, about 520 µg, about 530 µg, about 540 µg, about 550 µg, about 560 µg, about 570 µg, about 580 µg, about 590 µg, about 600 µg, about 610 µg, about 620 µg, about 630 µg, about 640 µg, about 650 µg, about 660 µg, about 670 µg, about 675 µg, about 680 µg, about 690 µg, or about 700 µg, of a compound of Formula (I), or a pharmaceutically acceptable salt thereof, including all values and ranges therein. In a further embodiment, R¹ is hexadecyl, e.g., linear hexadecyl.

In some embodiments, following once daily administration of a dry powder composition comprising from about 80 µg to about 675 µg (e.g., about 80 µg to about 640 µg, or about 112.5 µg to about 675 µg) of the compound of Formula (I), a stereoisomer thereof (or an equivalent dose of a pharmaceutically acceptable salt thereof, (e.g., a compound of Formula (II)), the dry power composition or method of use thereof provides a maximum treprostinil plasma concentration (Cₘₐₓ) ranging from about 10 pg/mL to about 2000 pg/mL, for example, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 110 pg/mL, about 120 pg/mL, about 130 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, about 700 pg/mL, about 750 pg/mL, about 800 pg/mL, about 850 pg/mL, about 900 pg/mL, about 950 pg/mL, about 1000 pg/mL, about 1050 pg/mL, about 1100 pg/mL, about 1150 pg/mL, about 1200 pg/mL, about 1250 pg/mL, about 1300 pg/mL, about 1350 pg/mL, about 1400 pg/mL, about 1450 pg/mL, about 1500 pg/mL, about 1550 pg/mL, about 1600 pg/mL, about 1650 pg/mL, about 1700 pg/mL, about 1750 pg/mL, about 1800 pg/mL, about 1850 pg/mL, about 1900 pg/mL, or about 2000 pg/mL, including all values and ranges therein.

In some embodiments, following once daily administration of about 80 µg to about 675 µg (e.g., about 80 µg to about 640 µg, or about 112.5 µg to about 675 µg) of the compound of Formula (II), the dry power composition or method of the disclosure provides an area under the plasma concentration curve (AUC) ranging from about 300 pg*h/mL to about 11000 pg*h/mL, for example, about 300 pg*h/mL, about 400 pg*h/mL, about 500 pg*h/mL, about 600 pg*h/mL, about 700 pg*h/mL, about 800 pg*h/mL, about 900 pg*h/mL, about 1000 pg*h/mL, about 1100 pg*h/mL, about 1200 pg*h/mL, about 1300 pg*h/mL, about 1400 pg*h/mL, about 1500 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 ng*h/mL, about 2800 ng*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*hr/mL, about 5200 pg*hr/mL, about 5300 pg*hr/mL, about 5400 pg*hr/mL, about 5500 pg*hr/mL, about 5600 pg*hr/mL, about 5700 pg*hr/mL, about 5800 pg*hr/mL, about 5900 pg*hr/mL, about 6000 pg*hr/mL, about 6100 pg*hr/mL, about 6200 pg*hr/mL, about 6300 pg*h/mLg*hr/mL, about 6400 pg*h/mL, about 6500 pg*h/mLg*hr/mL, about 6600 pg*hr/mL, about 6700 pg*hr/mL, about 6800 pg*hr/mL, about 6900 pg*hr/mL, about 7000 pg*hr/mL, about 7100 pg*hr/mL, about 7200 pg*hr/mL, about 7300 pg*hr/mL, about 7400 pg*hr/mL, about 7500 pg*hr/mL, about 7600 pg*hr/mL, about 7700 pg*hr/mL, about 7800 pg*hr/mL, about 7900 pg*hr/mL, about 8000 pg*hr/mL, about 8100 pg*hr/mL, about 8200 pg*hr/mL, about 8300 pg*hr/mL, about 8400 pg*hr/mL, about 8500 pg*hr/mL, about 8600 pg*hr/mL, about 8700 pg*hr/mL, about 8800 pg*hr/mL, about 8900 pg*hr/mL, about 9000 pg*hr/mL, about 9100 pg*hr/mL, about 9200 pg*hr/mL, about 9300 pg*hr/mL, about 9400 pg*hr/mL, about 9500 pg*hr/mL, about 9600 pg*hr/mL, about 9700 pg*hr/mL, about 9800 pg*hr/mL, about 9900 pg*hr/mL, about 10000 pg*hr/mL, about 10100 pg*hr/mL, about 10200 pg*hr/mL, about 10300 pg*hr/mL, about 10400 pg*hr/mL, about 10500 pg*hr/mL, about 10600 pg*hr/mL, about 10700 pg*hr/mL, about 10800 pg*hr/mL, about 10900 pg*hr/mL, or about 11000 pg*hr/mL including all values and ranges therein.

In some embodiments, the dry powder composition or method of disclosure achieves treprostinil plasma trough concentration during an administration period of the dry powder composition. In some embodiments, the plasma trough levels are sufficient to provide a sustained therapeutic response during the administration period. In some embodiments, the dry powder composition comprises from about 80 µg to about 675 µg of the compound of Formula (I) or a stereoisomer thereof (e.g., where R¹ is hexadecyl, e.g., linear hexadecyl), and following once daily administration, the dry powder composition provides or the subject (e.g., patient) has a treprostinil plasma trough concentration of at least about 1 pg/mL, about 2 pg/mL, about 3 pg/mL, about 4 pg/mL, about 5 pg/mL, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 100 pg/mL, about 110 pg/mL, about 120 pg/mL, about 130 pg/mL, about 140 pg/mL, about 150 pg/mL, about 160 pg/mL, about 170 pg/mL, about 180 pg/mL, about 190 pg/mL, about 200 pg/mL, including all values and ranges therein. In some embodiments, the dry powder composition comprises from about 80 µg to about 640 µg of the compound of Formula (II), and the treprostinil plasma trough concentration ranges from about 3 pg/mL to about 125 pg/mL, for example about 3 pg/mL, about 4 pg/mL, about 5 pg/mL, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 100 pg/mL, about 110 pg/mL, about 120 pg/mL, including all values and ranges therein. In some embodiments, the dry powder composition comprises from about 80 µg to about 640 µg of the compound of Formula (II), and the treprostinil plasma trough concentration ranges from about 10 pg/mL to about 100 pg/mL.

In some embodiments, following once daily administration of a dry powder composition comprising from about 80 µg to about 675 µg of the compound of Formula (II) or a stereoisomer thereof (or an equivalent dose of a pharmaceutically acceptable salt thereof, or a compound of Formula (I), a stereoisomer thereof, or pharmaceutically acceptable salt thereof), the dry power composition provides or the subject (e.g., patient) has at least one of the following characteristics:
(a) a maximum treprostinil plasma concentration (Cₘₐₓ) within about 80% to about 125% of the range of from about 17 pg/mL to about 1150 pg/mL, for example, about 13 pg/mL, about 14 pg/mL, about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 110 pg/mL, about 120 pg/mL, about 130 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, about 700 pg/mL, about 750 pg/mL, about 800 pg/mL, about 850 pg/mL, about 900 pg/mL, about 950 pg/mL, about 1000 pg/mL, about 1050 pg/mL, about 1100 pg/mL, about 1150 pg/mL, about 1200 pg/mL, about 1250 pg/mL, about 1300 pg/mL, about 1350 pg/mL, about 1400 pg/mL, or about 1430 pg/mL, including all values and ranges therein; or
(b) a treprostinil area under the plasma concentration curve (AUC_{0-inf}) within about 80% to about 125% of the range of from about 475 pg*h/mL to about 8000 pg*h/mL, for example, about 370 pg*h/mL, about 400 pg*h/mL, about 450 pg*h/mL, about 500 pg*h/mL, about 550 pg*h/mL, about 600 pg*h/mL, about 650 pg*h/mL, about 700 pg*h/mL, about 800 pg*h/mL, about 900 pg*h/mL, about 1000 pg*h/mL, about 1100 pg*h/mL, about 1200 pg*h/mL, about 1300 pg*h/mL, about 1400 pg*h/mL, about 1500 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 ng*h/mL, about 2800 ng*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*hr/mL, about 5200 pg*hr/mL, about 5300 pg*hr/mL, about 5400 pg*hr/mL, about 5500 pg*hr/mL, about 5600 pg*hr/mL, about 5700 pg*hr/mL, about 5800 pg*hr/mL, about 5900 pg*hr/mL, about 6000 pg*hr/mL, about 6100 pg*hr/mL, about 6200 pg*hr/mL, about 6300 pg*h/mL, about 6400 pg*h/mL, about 6500 pg*h/mL, about 6600 pg*hr/mL, about 6700 pg*hr/mL, about 6800 pg*hr/mL, about 6900 pg*hr/mL, about 7000 pg*hr/mL, about 7100 pg*hr/mL, about 7200 pg*hr/mL, about 7300 pg*hr/mL, about 7400 pg*hr/mL, about 7500 pg*hr/mL, about 7600 pg*hr/mL, about 7700 pg*hr/mL, about 7800 pg*hr/mL, about 7900 pg*hr/mL, about 8000 pg*hr/mL, about 8100 pg*hr/mL, about 8200 pg*hr/mL, about 8300 pg*h/mL, about 8400 pg*h/mL, about 8500 pg*h/mL, about 8600 pg*hr/mL, about 8700 pg*hr/mL, about 8800 pg*hr/mL, about 8900 pg*hr/mL, about 9000 pg*hr/mL, about 9100 pg*hr/mL, about 9200 pg*hr/mL, about 9300 pg*hr/mL, about 9400 pg*hr/mL, about 9500 pg*hr/mL, about 9600 pg*hr/mL, about 9700 pg*hr/mL, about 9800 pg*hr/mL, about 9900 pg*hr/mL, or about 10000 pg*hr/mL, including all values and ranges therein.

In some embodiments, the dry powder composition comprises about 80 µg of the compound of Formula (II), is administered once daily, and provides a treprostinil Cₘₐₓ ranging from about 14 pg/mL to about 155 pg/mL, for example, about 14pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 105 pg/mL about 110 pg/mL, about 115 pg/mL, about 120 pg/mL, about 125 pg/mL, about 130 pg/mL, about 135 pg/mL, about 140 pg/mL, about 145 pg/mL, about 150 pg/mL, and about 155 pg/mL, including all values and ranges therein. In some embodiments, about 80 µg of the compound of Formula (II), or a stereoisomer thereof (or an equivalent dose of a pharmaceutically acceptable salt thereof, or a compound of Formula (I), a stereoisomer thereof, or pharmaceutically acceptable salt thereof), is administered once daily and provides a treprostinil Cₘₐₓ of about 80%-125% of a range from about 17 pg/mL to about 125 pg/mL. In some embodiments, about 80 µg of the compound of Formula (II), or a stereoisomer thereof (or an equivalent dose of a pharmaceutically acceptable salt thereof, or a compound of Formula (I), a stereoisomer thereof, or pharmaceutically acceptable salt thereof), is administered once daily and provides a treprostinil Cₘₐₓ of about 80%-125% of a range from about 35 pg/mL to about 105 pg/mL.

In some embodiments, the dry powder composition comprises about 112.5 µg of the compound of Formula (II) or a stereoisomer thereof (or an equivalent dose of a pharmaceutically acceptable salt thereof, or a compound of Formula (I), a stereoisomer thereof, or pharmaceutically acceptable salt thereof), and provides a treprostinil Cₘₐₓ (CV%) ranging from about 80% to about 125% of about 78.4 (72.9) pg/mL.

In some embodiments, the dry powder composition comprises about 160 µg of the compound of Formula (II), is administered once daily, and provides a treprostinil Cₘₐₓ ranging from about 30 pg/mL to about 335 pg/mL, for example, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 105 pg/mL about 110 pg/mL, about 115 pg/mL, about 120 pg/mL, about 125 pg/mL, about 130 pg/mL, about 135 pg/mL, about 140 pg/mL, about 145 pg/mL, about 150 pg/mL, about 155 pg/mL, about 160 pg/mL, about 165 pg/mL, about 170 pg/mL, about 175 pg/mL, about 180 pg/mL, about 1850 pg/mL, about 190 pg/mL, about 195 pg/mL, about 200 pg/mL, about 205 pg/mL, about 210 pg/mL, about 215 pg/mL, about 220 pg/mL, about 225 pg/mL, about 230 pg/mL, about 235 pg/mL, about 240 pg/mL, about 245 pg/mL, about 250 pg/mL, about 255 pg/mL, about 260 pg/mL, about 265 pg/mL, about 270 pg/mL, about 275 pg/mL, about 280 pg/mL, about 285 pg/mL, about 290 pg/mL, about 295 pg/mL, about 300 pg/mL, about 305 pg/mL, about 310 pg/mL, about 315 pg/mL, about 320 pg/mL, about 325 pg/mL, about 330 pg/mL, about 335 pg/mL, about 340 pg/mL, about 345 pg/mL, or about 350 pg/mL, including all values and ranges therein. In some embodiments, about 160 µg of the compound of Formula (II), a stereoisomer thereof (or an equivalent dose of a pharmaceutically acceptable salt thereof, or a compound of Formula (I), a stereoisomer thereof, or pharmaceutically acceptable salt thereof), is administered once daily and provides a treprostinil Cₘₐₓ from about 80%-125% of a range from about 35 pg/mL to about 270 pg/mL. In some embodiments, about 160 µg of the compound of Formula (II), or a stereoisomer thereof (or an equivalent dose of a pharmaceutically acceptable salt thereof, or a compound of Formula (I), a stereoisomer thereof, or pharmaceutically acceptable salt thereof), is administered once daily and provides a treprostinil Cₘₐₓ from about 80%-125% of a range from about 76 pg/mL to about 230 pg/mL.

In some embodiments, the dry powder composition comprises about 225 µg of the compound of Formula (II), is administered once daily, and provides a treprostinil Cₘₐₓ ranging from about 80% to about 125% of about 287 (46.6) pg/mL. In some embodiments, the dry powder composition comprises about 225 µg of the compound of Formula (II) or a stereoisomer thereof (or an equivalent dose of a pharmaceutically acceptable salt thereof, or a compound of Formula (I), a stereoisomer thereof, or pharmaceutically acceptable salt thereof), and provides a steady state treprostinil Cₘₐₓ ranging from about 80% to about 125% of about 193 (32.9) pg/mL. In some embodiments, the dry powder composition comprises about 225 µg of the compound of Formula (II), is administered once daily, and provides a steady state treprostinil Cₘₐₓ (CV%) ranging from about 80% to about 125% of about 228 (46.4) pg/mL.

In some embodiments, the dry powder composition comprises about 240 µg of the compound of Formula (II), is administered once daily, and provides a treprostinil Cₘₐₓ ranging from about 45 pg/mL to about 520 pg/mL, for example, about 45 pg/mL, about 50 pg/mL, about 60 pg/mL, about 70 pg/mL, about 80 pg/mL, about 90 pg/mL, about 100 pg/mL, about 110 pg/mL, about 120 pg/mL, about 130 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, or about 520 pg/mL, including all values and ranges therein. In some embodiments, about 240 µg of the compound of Formula (II), or a stereoisomer thereof (or an equivalent dose of a pharmaceutically acceptable salt thereof, or a compound of Formula (I), a stereoisomer thereof, or pharmaceutically acceptable salt thereof), is administered once daily and provides a treprostinil Cₘₐₓ from about 80%-125% of a range from about 55 pg/mL to about 415 pg/mL. In some embodiments, about 240 µg of the compound of Formula (II), or an a stereoisomer thereof (or an equivalent dose of a pharmaceutically acceptable salt thereof, or a compound of Formula (I), a stereoisomer thereof, or pharmaceutically acceptable salt thereof), is administered once daily and provides a treprostinil Cₘₐₓ from about 80%-125% of a range from about 115 pg/mL to about 355 pg/mL.

In some embodiments, the dry powder composition comprises about 320 µg of the compound of Formula (II), is administered once daily, and provides a treprostinil Cₘₐₓ ranging from about 60 pg/mL to about 700 pg/mL, for example, about 60 pg/mL, about 70 pg/mL, about 80 pg/mL, about 90 pg/mL, about 100 pg/mL, about 110 pg/mL, about 120 pg/mL, about 130 pg/mL, about 135 pg/mL, about 140 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, or about 700 pg/mL, including all values and ranges therein. In some embodiments, about 320 µg of the compound of Formula (II) is administered once daily and provides a treprostinil Cₘₐₓ from about 80%-125% of a range from about 80 pg/mL to about 560 pg/mL. In some embodiments, about 320 µg of the compound of Formula (II) is administered once daily and provides a treprostinil Cₘₐₓ from about 80%-125% of a range from about 160 pg/mL to about 480 pg/mL.

In some embodiments, the dry powder composition comprises about 400 µg of the compound of Formula (II), is administered once daily, and provides a treprostinil Cₘₐₓ ranging from about 80 pg/mL to about 885 pg/mL, for example, about 80 pg/mL, about 90 pg/mL, about 100 pg/mL, about 110 pg/mL, about 120 pg/mL, about 130 pg/mL, about 135 pg/mL, about 140 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, and about 700 pg/mL, about 710 pg/mL, about 720 pg/mL, about 730 pg/mL, about 740 pg/mL, about 750 pg/mL, about 760 pg/mL, about 770 pg/mL, about 780 pg/mL, about 790 pg/mL, about 800 pg/mL, about 810 pg/mL, about 820 pg/mL, about 830 pg/mL, about 840 pg/mL, about 850 pg/mL, about 860 pg/mL, about 870 pg/mL, or about 880 pg/mL, including all values and ranges therein. In some embodiments, about 400 µg of the compound of Formula (II), is administered once daily and provides a treprostinil Cₘₐₓ from about 80%-125% of a range of about 100 pg/mL to about 705 pg/mL. In some embodiments, about 400 µg of the compound of Formula (II) is administered once daily and provides a treprostinil Cₘₐₓ from about 80%-125% of a range from about 200 pg/mL to about 605 pg/mL.

In some embodiments, the dry powder composition comprises about 450 µg of the compound of Formula (II) or a stereoisomer thereof (or an equivalent dose of a pharmaceutically acceptable salt thereof, or a compound of Formula (I), a stereoisomer thereof, or pharmaceutically acceptable salt thereof), and provides a treprostinil Cₘₐₓ ranging from about 80% to about 125% of about 387 (38.6) pg/mL.

In some embodiments, the dry powder composition comprises about 480 µg of the compound of Formula (II), is administered once daily, and provides a treprostinil Cₘₐₓ ranging from about 95 pg/mL to about 1065 pg/mL, for example, about 95 pg/mL, about 100 pg/mL, about 110 pg/mL, about 120 pg/mL, about 130 pg/mL, about 135 pg/mL, about 140 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, and about 700 pg/mL, about 710 pg/mL, about 720 pg/mL, about 730 pg/mL, about 740 pg/mL, about 750 pg/mL, about 760 pg/mL, about 770 pg/mL, about 780 pg/mL, about 790 pg/mL, about 800 pg/mL, about 810 pg/mL, about 820 pg/mL, about 830 pg/mL, about 840 pg/mL, about 850 pg/mL, about 860 pg/mL, about 870 pg/mL, about 880 pg/mL, about 890 pg/mL, about 900 pg/mL, about 910 pg/mL, about 920 pg/mL, about 930 pg/mL, about 940 pg/mL, about 950 pg/mL, about 960 pg/mL, about 970 pg/mL, about 980 pg/mL, about 1000 pg/mL, about 1010 pg/mL, about 1020 pg/mL, about 1030 pg/mL, about 1040 pg/mL, about 1050 pg/mL, about 1060 pg/mL, or about 1065 pg/mL including all values and ranges therein. In some embodiments, about 480 µg of the compound of Formula (II) is administered once daily and provides a treprostinil Cₘₐₓ from about 80%-125% of about 120 pg/mL to about 855 pg/mL. In some embodiments, about 480 µg of the compound of Formula (II) is administered once daily and provides a treprostinil Cₘₐₓ from about 80%-125% of a range from about 240 pg/mL to about 730 pg/mL.

In some embodiments, the dry powder composition comprises about 640 µg of the compound of Formula (II), is administered once daily, and provides a treprostinil Cₘₐₓ ranging from about 130 pg/mL to about 1430 pg/mL, for example, about 130 pg/mL, about 135 pg/mL, about 140 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, and about 700 pg/mL, about 710 pg/mL, about 720 pg/mL, about 730 pg/mL, about 740 pg/mL, about 750 pg/mL, about 760 pg/mL, about 770 pg/mL, about 780 pg/mL, about 790 pg/mL, about 800 pg/mL, about 810 pg/mL, about 820 pg/mL, about 830 pg/mL, about 840 pg/mL, about 850 pg/mL, about 860 pg/mL, about 870 pg/mL, about 880 pg/mL, about 890 pg/mL, about 900 pg/mL, about 910 pg/mL, about 920 pg/mL, about 930 pg/mL, about 940 pg/mL, about 950 pg/mL, about 960 pg/mL, about 970 pg/mL, about 980 pg/mL, about 1000 pg/mL, about 1010 pg/mL, about 1020 pg/mL, about 1030 pg/mL, about 1040 pg/mL, about 1050 pg/mL, about 1060 pg/mL, about 1070 pg/mL, about 1080 pg/mL, about 1090 pg/mL, about 1100 pg/mL, about 1110 pg/mL, about 1120 pg/mL, about 1130 pg/mL, about 1140 pg/mL, about 1150 pg/mL, about 1160 pg/mL, about 1170 pg/mL, about 1180 pg/mL, about 1190 pg/mL, about 1200 pg/mL, about 1210 pg/mL, about 1220 pg/mL, about 1230 pg/mL, about 1240 pg/mL, about 1250 pg/mL, about 1260 pg/mL, about 1270 pg/mL, about 1280 pg/mL, about 1290 pg/mL, about 1300 pg/mL, about 1310 pg/mL, about 1320 pg/mL, about 1330 pg/mL, about 1340 pg/mL, about 1350 pg/mL, about 1360 pg/mL, about 1370 pg/mL, about 1380 pg/mL, about 1390 pg/mL, about 1400 pg/mL, about 1410 pg/mL, about 1420 pg/mL, or about 1430 pg/mL, including all values and ranges therein. In some embodiments, about 640 µg of the compound of Formula (II) is administered once daily and provides a treprostinil Cₘₐₓ from about 80%-125% of a range of about 160 pg/mL to about 1140 pg/mL. In some embodiments, about 640 µg of the compound of Formula (II) is administered once daily and provides a treprostinil Cₘₐₓ ranging from about 80%-125% of about 325 pg/mL to about 980 pg/mL.

In some embodiments, the dry powder composition comprises about 675 µg of the compound of Formula (II), and provides a treprostinil Cₘₐₓ ranging from about 80% to about 125% of about 717 (52.8) pg/mL.

In some embodiments, the dry powder composition comprises about 80 µg of the compound of Formula (II), and upon administration, provides a treprostinil AUC_{0-inf} ranging from about 375 pg*h/mL to about 1800 pg*h/mL, for example, 375 pg*h/mL, 400 pg*h/mL, 500 pg*h/mL, 600 pg*h/mL, about 700 pg*h/mL, about 800 pg*h/mL, about 900 pg*h/mL, about 1000 pg*h/mL, about 1100 pg*h/mL, about 1200 pg*h/mL, about 1300 pg*h/mL, about 1400 pg*h/mL, about 1500 pg*h/mL, about 1600 pg*h/mL, about 1700 pg*h/mL, or about 1800 pg*h/mL, including all values and ranges therein. In some embodiments, about 80 µg of the compound of Formula (II), is administered once daily and provides a treprostinil AUC_{0-inf} from about 80%-125% of a range of about 475 pg*h/mL to about 1430 pg*h/mL. In some embodiments, the dry powder composition comprises about 80 µg of the compound of Formula (II), and upon administration, provides a treprostinil AUC_{0-inf} from about 80%-125% of about 660 pg*h/mL to about 1240 pg*h/mL.

In some embodiments, the dry powder composition comprises about 112.5 µg of the compound of Formula (II), and provides a treprostinil AUC_{0-inf} ranging from about 80% to about 125% of about 1090 (91.8) pg*h/mL.

In some embodiments, the dry powder composition comprises about 160 µg of the compound of Formula (II), and upon administration, provides a treprostinil AUC_{0-inf} ranging from about 630 pg*h/mL to about 3000 pg*h/mL, for example, 630 pg*h/mL, about 700 pg*h/mL, about 800 pg*h/mL, about 900 pg*h/mL, about 1000 pg*h/mL, about 1100 pg*h/mL, about 1200 pg*h/mL, about 1300 pg*h/mL, about 1400 pg*h/mL, about 1500 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, or about 3000 pg*h/mL, including all values and ranges therein. In some embodiments, the dry powder composition comprises about 160 µg of the compound of Formula (II), and upon administration, provides a treprostinil AUC_{0-inf} from about 80%-125% of a range from about 785 pg*h/mL to about 2370 pg*h/mL. In some embodiments, the dry powder composition comprises about 160 µg of the compound of Formula (II), and upon administration, provides a treprostinil AUC_{0-inf} from about 80%-125% of a range from about 1100 pg*h/mL to about 2050 pg*h/mL.

In some embodiments, the dry powder composition comprises about 225 µg of a compound of Formula (II), and upon administration, provides an AUC_{0-inf} ranging from about 80% to about 125% of about 2130 (30.0) ng*h/mL. In some embodiments, the dry powder composition comprises about 225 µg of the compound of Formula (II), and provides a steady state treprostinil AUC₀₋₂₄ (CV%) ranging from about 80% to about 125% of about 1680 (28.7) ng*h/mL. In some embodiments, the dry powder composition comprises about 225 µg of the compound of Formula (II) or a stereoisomer thereof (or an equivalent dose of a pharmaceutically acceptable salt thereof, or a compound of Formula (I), a stereoisomer thereof, or pharmaceutically acceptable salt thereof), and provides a steady state treprostinil AUC₀₋₂₄ (CV%) ranging from about 80% to about 125% of about 1790 (39.6) ng*h/mL.

In some embodiments, the dry powder composition comprises about 450 µg of the compound of Formula (II), and upon administration, provides a treprostinil AUC_{0-inf} ranging from about 80% to about 125% of about 4040 (27.4) pg*h/mL.

In some embodiments, the dry powder composition comprises about 240 µg of the compound of Formula (II), and upon administration, provides a treprostinil AUC_{0-inf} ranging from about 880 pg*h/mL to about 4130 pg*h/mL, for example, about 800 pg*h/mL, about 900 pg*h/mL, about 950 pg*h/mL, about 1000 pg*h/mL, about 1050 pg*h/mL, about 1100 pg*h/mL, about 1150 pg*h/mL, about 1200 pg*h/mL, about 1250 pg*h/mL, about 1300 pg*h/mL, about 1350 pg*h/mL, about 1400 pg*h/mL, about 1450 pg*h/mL, about 1500 pg*h/mL, about 1550 pg*h/mL, about 1600 pg*h/mL, about 1650 pg*h/mL, about 1700 pg*h/mL, about 1750 pg*h/mL, about 1800 pg*h/mL, about 1850 pg*h/mL, about 1950 pg*h/mL, about 2000 pg*h/mL, about 2050 pg*h/mL, about 2100 pg*h/mL, about 2150 pg*h/mL, about 2200 pg*h/mL, about 2250 pg*h/mL, about 2300 pg*h/mL, about 2350 pg*h/mL, about 2400 pg*h/mL, about 2450 pg*h/mL, about 2500 pg*h/mL, about 2550 pg*h/mL, about 2600 pg*h/mL, about 2650 pg*h/mL, about 2700 pg*h/mL, about 2750 pg*h/mL, about 2800 pg*h/mL, about 2850 pg*h/mL, about 2950 pg*h/mL, about 3000 pg*h/mL, about 3050 pg*h/mL, about 3100 pg*h/mL, about 3150 pg*h/mL, about 3200 pg*h/mL, about 3250 pg*h/mL, about 3300 pg*h/mL, about 3350 pg*h/mL, about 3400 pg*h/mL, about 3450 pg*h/mL, about 3500 pg*h/mL, about 3550 pg*h/mL, about 3600 pg*h/mL, about 3650 pg*h/mL, about 3700 pg*h/mL, about 3750 pg*h/mL, about 3800 pg*h/mL, about 3850 pg*h/mL, about 3950 pg*h/mL, about 4000 pg*h/mL, about 4050 pg*h/mL, about 4100 pg*h/mL, about 4130 pg*h/mL, including all values and ranges therein. In some embodiments, the dry powder composition comprises about 240 µg of the compound of Formula (II), and upon administration, provides a treprostinil AUC_{0-inf} from about 80%-125% of a range from about 1100 pg*h/mL to about 3305 pg*h/mL. In some embodiments, the dry powder composition comprises about 240 µg of the compound of Formula (II), and upon administration, provides a treprostinil AUC_{0-inf} from about 80%-125% of a range from about 1540 pg*h/mL to about 2865 pg*h/mL.

In some embodiments, the dry powder composition comprises about 320 µg of the compound of Formula (II), and upon administration, provides a treprostinil AUC_{0-inf} ranging from about 1130 pg*h/mL to about 5310 pg*h/mL, for example, about 1130 pg*h/mL, about 1200 pg*h/mL, about 1300 pg*h/mL, about 1400 pg*h/mL, about 1450 pg*h/mL, about 1500 pg*h/mL, about 1550 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*h/mL, about 5200 pg*h/mL, about 5300 pg*h/mL, about 5300 pg*h/mL, or about 5310 pg*h/mL, including all values and ranges therein. In some embodiments, the dry powder composition comprises about 320 µg of the compound of Formula (II), and upon administration, provides a treprostinil AUC_{0-inf} from about 80%-125% of a range from about 1400 pg*h/mL to about 4250 pg*h/mL. In some embodiments, about 320 µg of the compound of Formula (II), or a stereoisomer thereof (or an equivalent dose of a pharmaceutically acceptable salt thereof, or a compound of Formula (I), a stereoisomer thereof, or pharmaceutically acceptable salt thereof), is administered once daily and provides a treprostinil AUC_{0-inf} from about 80%-125% of a range from about 1975 pg*h/mL to about 3680 pg*h/mL.

In some embodiments, the dry powder composition comprises about 400 µg of the compound of Formula (II), and upon administration, provides a treprostinil AUC_{0-inf} ranging from about 1380 pg*h/mL to about 6480 pg*h/mL, for example, about 1380 pg*h/mL, about 1400 pg*h/mL, about 1450 pg*h/mL, about 1500 pg*h/mL, about 1550 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*h/mL, about 5200 pg*h/mL, about 5300 pg*h/mL, about 5400 pg*h/mL, about 5500 pg*h/mL, about 5600 pg*h/mL, about 5700 pg*h/mL, about 5800 pg*h/mL, about 5900 pg*h/mL, about 6000 pg*h/mL, about 6100 pg*h/mL, about 6200 pg*h/mL, about 6300 pg*h/mL, about 6400 pg*h/mL, or about 6480 pg*h/mL, including all values and ranges therein. In some embodiments, the dry powder composition comprises about 400 µg of the compound of Formula (II), and upon administration, provides a treprostinil AUC_{0-inf} from about 80%-125% of a range from about 1725 pg*h/mL to about 5180 pg*h/mL. In some embodiments, the dry powder composition comprises about 400 µg of the compound of Formula (II), and upon administration, provides a treprostinil AUC_{0-inf} from about 80%-125% of a range from about 2415pg*h/mL to about 4490 pg*h/mL.

In some embodiments, the dry powder composition comprises about 480 µg of the compound of Formula (II), and upon administration, provides a treprostinil AUC_{0-inf} ranging from about 1630 pg*h/mL to about 7650 pg*h/mL, for example, about 1630 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*h/mL, about 5200 pg*h/mL, about 5300 pg*h/mL, about 5400 pg*h/mL, about 5500 pg*h/mL, about 5600 pg*h/mL, about 5700 pg*h/mL, about 5800 pg*h/mL, about 5900 pg*h/mL, about 6000 pg*h/mL, about 6100 pg*h/mL, about 6200 pg*h/mL, about 6300 pg*h/mL, about 6400 pg*h/mL, about 6500 pg*h/mL, about 6600 pg*h/mL, about 6700 pg*h/mL, about 6800 pg*h/mL, about 6900 pg*h/mL, about 7000 pg*h/mL, about 7100 pg*h/mL, about 7200 pg*h/mL, about 7300 pg*h/mL, about 7400 pg*h/mL, about 7500 pg*h/mL, or about 7650 pg*h/mL, including all values and ranges therein. In some embodiments, the dry powder composition comprises about 480 µg of the compound of Formula (II), and upon administration, provides a treprostinil AUC_{0-inf} from about 80%-125% of a range from about 2040 pg*h/mL to about 6120 pg*h/mL. In some embodiments, the dry powder composition comprises about 480 µg of the compound of Formula (II), and upon administration, provides a treprostinil AUC_{0-inf} from about 80%-125% of a range from about 2855 pg*h/mL to about 5310 pg*h/mL.

In some embodiments, the dry powder composition comprises about 640 µg of the compound of Formula (II), and upon administration, provides a treprostinil AUC_{0-inf} ranging from about 2130 pg*h/mL to about 10000 pg*h/mL, for example, about 2130 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*h/mL, about 5200 pg*h/mL, about 5300 pg*h/mL, about 5400 pg*h/mL, about 5500 pg*h/mL, about 5600 pg*h/mL, about 5700 pg*h/mL, about 5800 pg*h/mL, about 5900 pg*h/mL, about 6000 pg*h/mL, about 6100 pg*h/mL, about 6200 pg*h/mL, about 6300 pg*h/mL, about 6400 pg*h/mL, about 6500 pg*h/mL, about 6600 pg*h/mL, about 6700 pg*h/mL, about 6800 pg*h/mL, about 6900 pg*h/mL, about 7000 pg*h/mL, about 7100 pg*h/mL, about 7200 pg*h/mL, about 7300 pg*h/mL, about 7400 pg*h/mL, about 7500 pg*h/mL, about 7600 pg*h/mL, about 7700 pg*h/mL, about 7800 pg*h/mL, about 8000 pg*h/mL, about 8100 pg*h/mL, about 8200 pg*h/mL, about 8300 pg*h/mL, about 8400 pg*h/mL, about 8500 pg*h/mL, about 8600 pg*h/mL, about 8700 pg*h/mL, about 8800 pg*h/mL, about 8900 pg*h/mL, about 9000 pg*h/mL, about 9100 pg*h/mL, about 9200 pg*h/mL, about 9300 pg*h/mL, about 9350 pg*h/mL, about 9400 pg*h/mL, about 9450 pg*h/mL, about 9500 pg*h/mL, about 9600 pg*h/mL, about 9700 pg*h/mL, about 9800 pg*h/mL, about 9900 pg*h/mL, or about 10000 pg*h/mL, including all values and ranges therein. In some embodiments, the dry powder composition comprises about 640 µg of the compound of Formula (II), and upon administration, provides a treprostinil AUC_{0-inf} from about 80%-125% of a range from about 2650 pg*h/mL to about 8000 pg*h/mL. In some embodiments, the dry powder composition comprises about 640 µg of the compound of Formula (II), and upon administration, provides a treprostinil AUC_{0-inf} from about 80%-125% of a range from about 3730 to about 6935 pg*h/mL.

In some embodiments, the dry powder composition comprises about 675 µg of the compound of Formula (II) or a stereoisomer thereof (or an equivalent dose of a pharmaceutically acceptable salt thereof, or a compound of Formula (I), a stereoisomer thereof, or pharmaceutically acceptable salt thereof), and provides a treprostinil AUC₀₋₂₄ ranging from about 80% to about 125% of about 5480 (13.8) pg*h/mL. In a further embodiment, the compound is a compound of Formula (II).

In some embodiments, the dry powder composition comprises from about 80 µg to about 675 µg of the compound of Formula (II), and the dry power composition provides or following once daily administration the subject (e.g., patient) has a treprostinil plasma trough concentration ranging from about 3 pg/mL to about 150 mg/mL, for example about 4 pg/mL, about 4 pg/mL, about 5 pg/mL, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 100 pg/mL, about 105 pg/mL, about 110 pg/mL, about 115 pg/mL, about 120 pg/mL, about 125 pg/mL about 130 pg/mL, about 135 pg/mL, about 140 pg/mL, about 145 pg/mL, or about 150 pg/mL, including all values and ranges therein.

In some embodiments, the dry powder composition comprises about 80 µg of the compound of Formula (II), and the dry power composition provides or following once daily administration the subject (e.g., patient) has a treprostinil plasma trough concentration ranging from about 3 pg/mL to about 25 mg/mL, for example, about 3 pg/mL, about 4 pg/mL pg/mL, about 5 pg/mL, about 10 pg/mL, about 15 pg/mL, about 20 pg/mL, or about 25 pg/mL, including all values and ranges therein. In a further embodiment, the treprostinil plasma trough concentration ranges from about 6 pg/mL to about 18 mg/mL.

In some embodiments, the dry powder composition comprises about 112.5 µg of the compound of Formula (II), and the dry power composition provides or following once daily administration the subject (e.g., patient) has a treprostinil plasma trough concentration ranging from about 4 pg/mL to about 30 mg/mL, for example about 4 pg/mL, about 5 pg/mL, about 10 pg/mL, about 15 pg/mL, about 20 pg/mL, about 25 pg/mL, or about 30 pg/mL, including all values and ranges therein.

In some embodiments, the dry powder composition comprises about 160 µg of the compound of Formula (II), and the dry power composition provides or following once daily administration the subject (e.g., patient) has a treprostinil plasma trough concentration ranging from about 5 pg/mL to about 35 mg/mL, for example about 5 pg/mL, about 10 pg/mL, about 15 pg/mL, about 20 pg/mL, about 25 pg/mL, about 30 pg/mL, or about 35 pg/mL, including all values and ranges therein. In a further embodiment, the treprostinil plasma trough concentration ranges from about 10 pg/mL to about 30 mg/mL, or from 15 pg/mL to about 25 pg/mL.

In some embodiments, the dry powder composition comprises from about 225 µg of the compound of Formula (II), and the dry power composition provides or following once daily administration the subject (e.g., patient) has a treprostinil plasma trough concentration ranging from about 15 pg/mL to about 45 mg/mL, for example about 15 pg/mL, about 20 pg/mL, about 25 pg/mL, about 30 pg/mL, about 35 pg/mL, about 40 pg/mL, or about 45 pg/mL, including all values and ranges therein.

In some embodiments, the dry powder composition comprises from about 240 µg of the compound of Formula (II), and the dry power composition provides or following once daily administration the subject (e.g., patient) has a treprostinil plasma trough concentration ranging from about 7 pg/mL to about 50 mg/mL, for example about 7 pg/mL, about 10 pg/mL, about 15 pg/mL, about 20 pg/mL, about 25 pg/mL, about 30 pg/mL, about 35 pg/mL, about 40 pg/mL, about 45 pg/mL, or about 50 pg/mL, including all values and ranges therein. In some embodiments, the treprostinil plasma trough concentration ranges from about 15 pg/mL to about 50 mg/mL, or from 20 pg/mL to about 45 pg/mL.

In some embodiments, the dry powder composition comprises about 320 µg of the compound of Formula (II) and the dry power composition provides or following once daily administration the subject (e.g., patient) has a treprostinil plasma trough concentration ranging from about 9 pg/mL to about 65 mg/mL, for example about 9 pg/mL, about 10 pg/mL, about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, or about 65 pg/mL, including all values and ranges therein. In some embodiments, the treprostinil plasma trough concentration ranges from about 15 pg/mL to about 50 mg/mL, or from 20 pg/mL to about 45 pg/mL.

In some embodiments, the dry powder composition comprises about 400 µg of the compound of Formula (II), and the dry power composition provides or following once daily administration the subject (e.g., patient) has a treprostinil plasma trough concentration ranging from about 10 pg/mL to about 80 mg/mL, for example about 10 pg/mL, about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, or about 80 pg/mL including all values and ranges therein. In some embodiments, the treprostinil plasma trough concentration ranging from about 35 pg/mL to about 70 mg/mL, or from 40 pg/mL to about 65 pg/mL.

In some embodiments, the dry powder composition comprises about 480 µg of the compound of Formula (II), and the dry power composition provides or following once daily administration the subject (e.g., patient) has a treprostinil plasma trough concentration ranging from about 13 pg/mL to about 95 mg/mL, for example about 13 pg/mL, about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, including all values and ranges therein. In some embodiments, the treprostinil plasma trough concentration ranging from about 25 pg/mL to about 75 mg/mL, or from 30 pg/mL to about 70 pg/mL.

In some embodiments, the dry powder composition comprises about 640 µg of the compound of Formula (II), and the dry power composition provides or following once daily administration the subject (e.g., patient) has a treprostinil plasma trough concentration ranging from about 15 pg/mL to about 125 mg/mL, for example about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 105 pg/mL, about 110 pg/mL, about 115 pg/mL, about 120 pg/mL, or about 125 pg/mL, including all values and ranges therein. In some embodiments, the treprostinil plasma trough concentration ranging from about 35 pg/mL to about 100 mg/mL, or from 50 pg/mL to about 90 pg/mL.

In some embodiments, the dry powder composition comprises about 450 µg of the compound of Formula (II), and the dry power composition provides or following once daily administration the subject (e.g., patient) has a treprostinil plasma trough concentration ranging from about 30 pg/mL to about 75 mg/mL, for example about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, and about 75 pg/mL, including all values and ranges therein.

In some embodiments, the dry powder composition comprises from about 675 µg of the compound of Formula (II) and the dry power composition provides or following once daily administration the subject (e.g., patient) has a treprostinil plasma trough concentration ranging from about 50 pg/mL to about 100 mg/mL, for example about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, and about 100 pg/mL, including all values and ranges therein.

### Aerosolized compositions

The dry powder compositions described herein are in some embodiments, aerosolized via a DPI to provide an aerosolized composition. The aerosolized composition is administered to patient in need of treatment of PH. In another embodiment, the aerosolized composition is administered to patient in need of treatment of pulmonary fibrosis (e.g., PH-ILD where the ILD is pulmonary fibrosis). The aerosolized composition can be characterized by certain parameters known to those of skill in the art, such as mass median aerodynamic diameter (MMAD) and fine particle fraction (FPF).

Mass median aerodynamic diameter (MMAD) is the value of aerodynamic diameter for which 50% of the mass in a given aerosol is associated with particles smaller than the median aerodynamic diameter (MAD), and 50% of the mass is associated with particles larger than the MAD. MMAD can be determined by impactor measurements, e.g., the Andersen Cascade Impactor (ACT) or the Next Generation Impactor (NGI). In some embodiments, the aerosolized dry powder composition comprises particles with an MMAD of from about 1 µm to about 10 µm, from about 1 µm to about 7 µm, from about 1 µm to about 5 µm, or from about 1 µm to about 4 µm, or from about 1.5 µm to about 3.5 µm, or from about 2 µm to about 3 µm, as measured by NGI. In one embodiment, the dry powder composition exhibiting one of the MMAD profiles provided above comprises mannitol. In another embodiment, the dry powder composition exhibiting the MMAD profile provided above comprises trehalose.

"Fine particle fraction" or "FPF" refers to the fraction of an aerosol having a particle size less than 5 µm in diameter, as measured by cascade impaction. FPF is usually expressed as a percentage. FPF has been demonstrated to correlate to the fraction of the powder that is deposited in the lungs of the subject (e.g., patient). In some embodiments, the dry powder composition is in the form of an aerosol comprising particles with an FPF of at least 20%, at least 30%, at least 40%, at least 50%, from about 30% to about 60%, from about 35% to about 55%, or from about 40% to about 50%, as measured by the NGI. In one embodiment, the aerosolized dry powder composition comprises particles with an FPF of from about 40% to about 70%, from about 30% to about 60%, or from about 50% to about 60%, as measured by NGI. In one embodiment, the dry powder composition exhibiting one of the FPF profiles provided above comprises mannitol. In another embodiment, the dry powder composition exhibiting the FPF profile provided above comprises trehalose.

The dry powder compositions of the present disclosure may be produced from liquid compositions using lyophilization or spray-drying techniques. When lyophilization is used, the lyophilized composition may be milled to obtain the finely divided dry powder containing particles within the desired size range described above. When spray-drying is used, the process is carried out under conditions that result in a finely divided dry powder containing particles within the desired size range described above. Exemplary methods of preparing dry powder forms of pharmaceutical compositions are disclosed in WO 96/32149, WO 97/41833, WO 98/29096, and U.S. Patent Nos. 5,976,574, 5,985,248, and 6,001,336, the disclosure of each of which is incorporated herein by reference in their entireties. Exemplary spray drying methods are described in U.S. Application Publication No. 2020/0338005, and U.S. Patent Nos. 6,848,197 and 8,197,845, the disclosure of each of which is incorporated herein by reference in their entireties.

In some embodiments, the dry powder compositions of the present disclosure are prepared by the following process. A stock solution of a compound of Formula (I) or (II), a stereoisomer thereof, or a pharmaceutically acceptable salt thereof is prepared using an organic solvent, such as an alcohol (e.g., 1-propanol). Aqueous stock solutions of a sugar (e.g., mannitol or trehalose) and leucine are also prepared. Afterwards required amounts of the above stock solutions are added to a mixture of water and the organic solvent to form a spray drying feed solution. In the spray drying feed solution, the volume ratio of water to the organic solvent may be from about 3:2 to about 1:1.

Spray drying is initiated by starting the drying gas flow and heating up the drying gas by setting the desired inlet temperature at, for example, from about 120 °C to about 180 °C, or from about 135 °C to about 150 °C. After the spray dryer outlet temperature reaches a suitable temperature, for example, at from about 55 °C to about 65 °C, the liquid skid inlet is set to allow blank solvents to be atomized with the aid of nitrogen into the spray dryer, and the system is allowed to cool and stabilize. Product filter pulsing is initiated, and product filter purge flow is set, for example, to 10 to 20 scfh. After the system stabilizes, the liquid skid inlet is switched to the feed solution prepared above and the process is continued till the feed solution runs out. At the point when the feed solution runs out, the liquid skid inlet is switched back to blank solvents, which are allowed to spray for from about 5 to about 20 minutes. At this point, powder is collected at the bottom of the product filter. After spraying the blank solvent for from about 5 to about 20 minutes, the system is shut down by shutting down the liquid lines, atomization gas, drying gas heater, drying gas inlet and finally the exhaust.

The dry powder compositions of the present disclosure are delivered to the lungs of a subject (e.g., patient) via inhalation using a dry powder inhaler (DPI). In one embodiment, the dry powder inhaler is a single dose dry powder inhaler. A propellant-free device, a DPI delivers dry powder to the lungs of a subject (e.g., patient) using the subject (e.g., patient) inspiration. The unit dose of a dry powder composition used in a DPI device is often a dry powder blister disc of hard capsule. Exemplary DPI devices suitable for delivering the dry powder compositions of the present disclosure include the devices described in the following paragraphs, as well as the DPIs described in U.S. Patent Nos. 6,766,799, 7,278,425 and 8,496,002, the disclosure of each of which is herein incorporated by reference in their entireties.

The AIR^{®} inhaler (Alkermes) includes a small, breath-activated system that delivers porous powder from a capsule. The porous particles have an aerodynamic diameter of 1-5 µm. *See* International Patent Application Publication Nos. WO 99/66903 and WO 00/10541, the disclosure of each of which is incorporated herein by reference in their entireties.

Aerolizer^{™} (Novartis) is a single dose dry powder inhaler. In this device, dry powder medicament is stored in a capsule and released by piercing the capsule wall with TEFLON-coated steel pins. *See* U.S. Patent Nos. 6,488,027 and 3,991,761, the disclosure of each of which is incorporated herein by reference in their entireties.

Bang Olufsen provides a breath actuated inhaler using blister strips with up to sixty doses. The dose is made available only during the inhalation by a novel trigger mechanism. The device is equipped with a dose counter and can be disposed of after all doses have been used. *See* EP 1522325, the disclosure of which is incorporated herein by reference in its entirety.

Clickhaler^{®} (Innovata PLC) is a large reservoir breath-activated multidose device. *See* U.S. Pat. 5,437,270, the disclosure of which is incorporated herein by reference in its entirety.

DirectHaler^{™} (Direct-Haler A/S) is a single dose, pre-metered, pre-filled, disposable DPI device made from polypropylene. *See* U.S. Patent No. 5,797,392, the disclosure of which is incorporated herein by reference in its entirety.

Diskus^{™} (GlaxoSmithKline) is a disposable small DPI device that holds up to 60 doses contained in double foil blister strips to provide moisture protection. *See* GB2242134, the disclosure of which is incorporated herein by reference in its entirety.

Eclipse^{™} (Aventis) is a breath actuated re-usable capsule device capable of delivering up to 20 mg of a dry power composition. The powder is sucked from the capsule into a vortex chamber where a rotating ball assists in powder disaggregation as a subject (e.g., patient) inhales. See U.S. Pat. 6,230,707 and WO 9503846, the disclosure of each of which is incorporated herein by reference in their entireties.

Flexhaler^{®} is a plastic breath-activated dry powder inhaler and is amenable for use with the dry powder compositions provided herein.

FlowCaps^{®} (Hovione) is a capsule-based, re-fillable, re-usable passive dry-powder inhaler that holds up to 14 capsules. The inhaler itself is moisture-proof. *See* U.S. Pat. 5,673,686, the disclosure of which is incorporated herein by reference in its entirety.

Gyrohaler^{®} (Vectura) is a passive disposable DPI containing a strip of blisters. *See* GB2407042, the disclosure of which is incorporated herein by reference in its entirety.

The HandiHaler^{®} (Boehringer Ingelheim GmbH) is a single dose DPI device. It can deliver up to 30 mg of a dry powder composition in capsules. *See* International Patent Application Publication No. WO 04/024156, the disclosure of which is incorporated herein by reference in its entirety.

MicroDose DPI (Microdose Technologies) is a small electronic DPI device. It uses piezoelectric vibrator (ultrasonic frequencies) to deaggragate the drug powder in an aluminum blister (single or multiple dose). *See* U.S. Patent No. 6,026,809, the disclosure of which is incorporated herein by reference in its entirety.

Nektar Dry Powder Inhaler^{®} (Nektar) is a palm-sized and easy-to-use device. It provides convenient dosing from standard capsules and flow-rate-independent lung deposition.

Nektar Pulmonary Inhaler^{®} (Nektar) efficiently removes powders from the packaging, breaks up the particles and creates an aerosol cloud suitable for deep lung delivery. It enables the aerosolized particles to be transported from the device to the deep lung during a subject's (e.g., patient's) breath, reducing losses in the throat and upper airways. Compressed gas is used to aerosolize the powder. *See* AU4090599 and U.S. Patent No. 5,740,794, the disclosure of each of which is incorporated herein by reference in their entireties.

NEXT DPI^{™} is a device featuring multidose capabilities, moisture protection, and dose counting. The device can be used regardless of orientation (upside down) and doses only when proper aspiratory flow is reached. *See* EP 1196146, U.S. Patent No. 6,528,096, WO0178693, and WO0053158, the disclosure of each of which is incorporated herein by reference in their entireties.

Neohaler^{®} is a capsule-based plastic breath-activated dry powder inhaler.

Oriel^{™} DPI is an active DPI that utilizes a piezoelectric membrane and nonlinear vibrations to aerosolize powder formulations. *See* International Patent Application Publication No. WO 01/68169, the disclosure of which is incorporated herein by reference in its entirety.

The DPI in one embodiment, is a capsule based DPI. In a further embodiment, the capsule based DPI is manufactured by Plastiape. In even a further embodiment, the capsule based DPI is a RS01 monodose dry powder inhaler developed by Plastiape, which features a compact size and a simple and effective perforation system and is suited for both gelatin and HMPC capsules.

Pressair^{™} is a plastic breath-activated dry powder inhaler.

Pulvinal^{®} inhaler (Chiesi) is a breath-actuated multi-dose (100 doses) dry powder inhaler. The dry powder is stored in a reservoir which is transparent and clearly marked to indicate when the 100th dose has been delivered. *See* U.S. Patent No. 5,351,683, the disclosure of which is incorporated herein by reference in its entirety.

The Rotohaler^{®} (GlaxoSmithKline) is a single use device that utilizes capsules. *See* U.S. Patent Nos. 5,673,686 and 5,881,721, the disclosure of each of which is incorporated herein by reference in their entireties.

Rexam DPI (Rexam Pharma) is a single dose, reusable device designed for use with capsules. *See* U.S. Patent No. 5,651,359 and EP 0707862, the disclosure of each of which is incorporated herein by reference in their entireties.

S2 (Innovata PLC) is a re-useable or disposable single-dose DPI for the delivery of a dry powder composition in high concentrations. Its dispersion mechanism requires minimal effort to achieve excellent drug delivery to the subject's (e.g., patient's) lungs. S2 is easy to use and has a passive engine so no battery or power source is required. *See* AU3320101, the disclosure of which is incorporated herein by reference in its entirety.

SkyeHaler^{®} DPI (SkyePharma) is a multidose device containing up to 300 individual doses in a single-use, or replaceable cartridge. The device is powered by breath and requires no coordination between breathing and actuation. *See* U.S. Patent No. 6,182,655 and WO97/20589, the disclosure of each of which is incorporated herein by reference in their entireties.

Taifun^{®} DPI (LAB International) is a multiple-dose (up to 200) DPI device. It is breath actuated and flow rate independent. The device includes a unique moisture-balancing drug reservoir coupled with a volumetric dose metering system for consistent dosing. *See* U.S. Patent No. 6,132,394, the disclosure of which is incorporated herein by reference in its entirety.

The TurboHaler^{®} (AstraZeneca) is described in U.S. Patent No. 5,983,893, the disclosure of which is incorporated herein by reference in its entirety. This DPI device is an inspiratory flow-driven, multi-dose dry-powder inhaler with a multi-dose reservoir that provides up to 200 doses of a dry powder composition and a dose range from a few micrograms to 0.5 mg.

The Twisthaler^{®} (Schering-Plough) is a multiple dose device with a dose counting feature and is capable of 14-200 actuations. A dry powder composition is packaged in a cartridge that contains a desiccant. *See* U.S. Patent No. 5,829,434, the disclosure of which is incorporated herein by reference in its entirety.

Ultrahaler^{®} (Aventis) combines accurate dose metering and good dispersion. It is an easy-to-use, discrete, pocket-sized device with a numerical dose counter, dose taken indicator and a lock-out mechanism. The device is capable of delivering up to 20 mg of a dry powder composition. Ultrahaler^{®} is described in U.S. Patent No. 5,678,538 and WO2004026380, the disclosure of each of which is incorporated herein by reference in their entireties.

Xcelovair^{™} (Meridica/Pfizer) holds 60 pre-metered, hermetically sealed doses in the range of 5-20 mg. The device provides moisture protection under accelerated conditions of 40°C/75% RH. The dispersion system maximizes the fine particle fraction, delivering up to 50% fine particle mass.

In another aspect, a system is provided comprising (i) one of the dry powder compositions described herein and (ii) a dry powder inhaler (DPI) for administration of the dry powder composition. The DPI includes (a) a reservoir comprising the dry powder composition disclosed herein, and (b) a means for introducing the dry powder composition into the subject's lungs via inhalation. The reservoir in one embodiment, comprises the dry powder composition of the present invention in a capsule or in a blister pack. The material for the shell of a capsule can be gelatin, cellulose derivatives, starch, starch derivatives, chitosan, or synthetic plastics. The DPI may be a single dose or a multidose inhaler. In addition, the DPI may be pre-metered or device-metered. In one embodiment, the dry powder inhaler is a single dose dry powder inhaler.

The system, in one embodiment, is used for treating pulmonary hypertension (e.g., group 1 or group 3 PH), portopulmonary hypertension, or pulmonary fibrosis as described in further detail below. The system includes the dry powder composition disclosed herein, i.e., a dry powder composition comprising a compound of Formula (I) or (II), a stereoisomer thereof, or a pharmaceutically acceptable salt thereof, and a DPI. In one embodiment, the dry powder composition comprises a compound of Formula (I) or (II), or a pharmaceutically acceptable salt thereof. In another embodiment, the dry powder composition comprises a compound of Formula (I) or (II). The dry powder inhaler may be one described above, may be a single dose or a multidose inhaler, and/or may be pre-metered or device-metered. In one embodiment, the dry powder inhaler is a single dose dry powder inhaler.

The term "treating" includes: (1) preventing or delaying the appearance of clinical symptoms of the state, disorder or condition developing in the patient that may be afflicted with or predisposed to the state, disorder or condition but does not yet experience or display clinical or subclinical symptoms of the state, disorder or condition; (2) inhibiting the state, disorder or condition (e.g., arresting, reducing or delaying the development of the disease, or a relapse thereof in case of maintenance treatment, of at least one clinical or subclinical symptom thereof); and/or (3) relieving the condition (e.g., causing regression of the state, disorder or condition or at least one of its clinical or subclinical symptoms). In one embodiment, "treating" refers to inhibiting the state, disorder or condition (e.g., arresting, reducing or delaying the development of the disease, or a relapse thereof in case of maintenance treatment, of at least one clinical or subclinical symptom thereof). In another embodiment, "treating" refers to relieving the condition (for example, by causing regression of the state, disorder or condition or at least one of its clinical or subclinical symptoms). The benefit to a patient to be treated is either statistically significant as compared to the state or condition of the same patient before the treatment, or as compared to the state or condition of an untreated control patient, or the benefit is at least perceptible to the patient or to the physician.

"Effective amount" means an amount of a dry powder composition of the present disclosure that is sufficient to result in the desired therapeutic response. The "effective amount" is the amount of the compound of Formula (I) or (II) that is administered in a single dosing session.

In one aspect of the invention, a method for treating pulmonary hypertension (PH) in a patient in need thereof is provided. The method includes administering an effective amount of one of the dry powder compositions disclosed herein to the lungs of the patient via a dry powder inhaler (DPI), once daily during an administration period. The dry powder composition comprises a compound of Formula (I) or (II), or a pharmaceutically acceptable salt thereof. The administering comprises (i) aerosolizing the dry powder composition via a DPI to provide an aerosolized dry powder composition, and (ii) administering the aerosolized dry powder composition to the lungs of the patient via inhalation by the DPI.

The World Health Organization (WHO) has classified PH into five groups. Group 1 PH includes pulmonary arterial hypertension (PAH), idiopathic pulmonary arterial hypertension (IPAH), familial pulmonary arterial hypertension (FPAH), and pulmonary arterial hypertension associated with other diseases (APAH). For example, pulmonary arterial hypertension associated with collagen vascular disease (e.g., scleroderma), congenital shunts between the systemic and pulmonary circulation, portal hypertension and/or HIV infection are included in group 1 PH. Group 2 PH includes pulmonary hypertension associated with left heart disease, e.g., atrial or ventricular disease, or valvular disease (e.g., mitral stenosis). WHO group 3 pulmonary hypertension is characterized as pulmonary hypertension associated with lung diseases, e.g., chronic obstructive pulmonary disease (COPD), interstitial lung disease (ILD), and/or hypoxemia. Group 4 pulmonary hypertension is pulmonary hypertension due to chronic thrombotic and/or embolic disease. Group 4 PH is also referred to as chronic thromboembolic pulmonary hypertension. Group 4 PH patients experience blocked or narrowed blood vessels due to blood clots. Group 5 PH is the "miscellaneous" category, and includes PH caused by blood disorders (e.g., polycythemia vera, essential thrombocythemia), systemic disorders (e.g., sarcoidosis, vasculitis) and/or metabolic disorders (e.g., thyroid disease, glycogen storage disease).

The methods provided herein can be used to treat group 1, group 2, group 3, group 4 or group 5 PH patients, as characterized by the WHO.

In one embodiment of the methods, the pulmonary hypertension treated is chronic thromboembolic pulmonary hypertension.

In one preferred embodiment, the pulmonary hypertension is group 1 PH, as characterized by the WHO. In a further embodiment, the method provided herein is a method for treating treated is pulmonary arterial hypertension (PAH). In a further embodiment, the PAH is class I PAH, class II PAH, class III PAH, or class IV PAH, as characterized by the New York Heart Association (NYHA).

In one embodiment, the PAH is class I PAH, as characterized by the NYHA.

In another embodiment, the PAH is class II PAH, as characterized by the NYHA.

In yet another embodiment, the PAH is class III PAH, as characterized by the NYHA.

In still another embodiment, the PAH is class IV PAH, as characterized by the NYHA.

In one embodiment, the pulmonary hypertension (PH) is portopulmonary hypertension (PPH). PPH is defined by the coexistence of portal and pulmonary hypertension. The diagnosis of portopulmonary hypertension is based on hemodynamic criteria: (1) portal hypertension and/or liver disease (clinical diagnosis-ascites/varices/splenomegaly), (2) mean pulmonary artery pressure > 25 mmHg at rest, (3) pulmonary vascular resistance > 240 dynes s/cm⁵, (4) pulmonary artery occlusion pressure < 15mmHg or transpulmonary gradient > 12 mmHg. PPH is a serious complication of liver disease, and is present in 0.25 to 4% of patients suffering from cirrhosis. PPH is comorbid in an estimated 4-6% of those referred for a liver transplant.

In one preferred embodiment, the pulmonary hypertension is group 3 PH, as characterized by the WHO. In a further embodiment, the method provided herein is a method for treating PH associated with interstitial lung disease (PH-ILD).

In the methods for treating PH-ILD provided herein, the ILD may include one or more lung conditions. The one or more lung conditions comprise, in one embodiment, idiopathic pulmonary fibrosis (IPF), cryptogenic organizing pneumonia (COP), desquamative interstitial pneumonitis, nonspecific interstitial pneumonitis, hypersensitivity pneumonitis, acute interstitial pneumonitis, interstitial pneumonia (e.g., idiopathic interstitial pneumonia), connective tissue disease, sarcoidosis or asbestosis. In one embodiment, the ILD is connective tissue disease-associated interstitial lung disease (CTD-ILD). In another embodiment, the ILD is sarcoidosis. In yet another embodiment, the ILD is IPF. In even another embodiment, the ILD is an idiopathic interstitial peneumonia (IIP).

In one embodiment for treating PH-ILD provided herein, the ILD includes pulmonary fibrosis, e.g., idiopathic pulmonary fibrosis (IPF). Pulmonary fibrosis is a respiratory disease in which scars are formed in the lung tissues, leading to serious breathing problems. Scar formation, i.e., the accumulation of excess fibrous connective tissue, leads to thickening of the walls, and causes reduced oxygen supply in the blood. As a result, pulmonary fibrosis patients suffer from perpetual shortness of breath. In some patients the specific cause of the disease can be diagnosed, but in others the probable cause cannot be determined, a condition called IPF.

The length of the administration period in any given case may depend on the nature and severity of the PH being treated and how well a patient tolerates and responds to the therapy. The treatment methods provided herein are provided as a chronic therapy, and as such, a patient is on-therapy as long as the therapy is safe and effective. Accordingly, the administration period in one embodiment, continues until a patient dies. In another embodiment, the administration period is the length of time the treatment is effective.

In one embodiment, if a patient experiences an adverse reaction to the therapy, they are provided a decreased dose during the administration period. Similarly, a patient may be titrated to a higher dose should they show a lower dose be shown to be well tolerated. In one embodiment, the uptitration takes place only after the patient has shown to tolerate a lower dose for two or more days, e.g., two days, three days, four days, five days, six days or seven days.

In some embodiments, the administration period is about about 6 months, about 7 months, about 8 months, about 9 months, about 10 months, about 11 months, about 1 year, about 2 years, about 3 years, about 4 years, about 5 years, about 6 years, about 7 years, about 8 years, about 9 years, about 10 years, about 15 years, about 20 years or about 30 years.

In another embodiment, the administration period for the methods provided herein is at least about 6 months, at least about 7 months, at least about 8 months, at least about 9 months, at least about 10 months, at least about 11 months, at least about 1 year, at least about 2 years, at least about 3 years, at least about 4 years, at least about 5 years, at least about 6 years, at least about 7 years, at least about 8 years, at least about 9 years or at least about 10 years or at least about 20 years. The administration period, in another embodiment, is from about 30 days to about 2 years. In another embodiment, the administration period is from about 6 months to about 3 years, or from 6 months to about 4 years, or from about 6 months to about 5 years, or from about 6 months to about 6 years, or from about 6 months to about 7 years, or from about 6 months to about 8 years, or from about 1 year to about 10 years, or from about 2 years to about 10 years, or from about 6 months to about 20 years, or from about 5 years to about 20 years, or from about 10 years to about 30 years.

In one embodiment, the administration period is at least about 1 year.

In one embodiment, the administration period is at least about 5 years.

In one embodiment, the administration period is from about 1 year to about 15 years. In another embodiment, the administration period is from about 5 years to about 15 years. In yet another embodiment, the administration period is from about 10 years to about 20 years. In even another embodiment, the administration period is from about 1 year to about 20 years.

In one embodiment of the disclosed methods, a patient is administered the dry powder composition once daily in a single dosing session during an administration period. In another embodiment, the patient is administered the dry powder composition twice daily, i.e., in two separate dosing sessions. In one embodiment, the administration is with food. In one embodiment, each dosing session comprises 1 to 5 inhalations (puffs) from a DPI, for example 1 inhalation (1 puff), 2 inhalations (2 puffs), 3 inhalations (3 puffs), 4 inhalations (4 puffs) or 5 inhalations (5 puffs). As used herein, a "dosing session" refers to 1 to 5 inhalations (puffs) from a DPI as required to administer from about 80 µg to about 700 µg of the compound of Formula (I) or (II), a stereoisomer thereof, or a pharmaceutically acceptable salt thereof. The DPI, in one embodiment, is small and transportable by the patient. In one embodiment, the DPI is a single dose DPI.

In order to achieve a particular dose, in one embodiment, more than one DPI capsule comprising the composition can be employed. For example, in the case of a 640 µg dose, two 320 µg DPI capsules can be used. Each capsule can be administered via 1 or 2 inhalations, for example.

The effective amount of the compound of Formula (I) or (II), a stereoisomer thereof, or a pharmaceutically acceptable salt thereof, may include a fixed dose of a compound of Formula (I) or (II), a stereoisomer thereof, or a pharmaceutically acceptable salt thereof. The fixed dose, in one embodiment, is present in one or multiple DPI capsules. The fixed dose, in one embodiment, is a dose that is titrated (either up or down) from a prior dose. In another embodiment, the fixed dose is the same dose or substantially the same dose as a prior dose. The effective amount, in one embodiment, is the amount of the compound of Formula (I) or (II), a stereoisomer thereof, or a pharmaceutically acceptable salt thereof, administered during each dosing session. In some embodiments, the amount "administered" refers to the amount of the compound of Formula (I) or (II), a stereoisomer thereof, or a pharmaceutically acceptable salt thereof, in the capsule, or multiple capsules in the DPI, administered in a single dosing session. In some embodiments, the fixed dose ranges from about 80 µg to about 700 µg of a compound of Formula (I) or (II), a stereoisomer thereof, or a pharmaceutically acceptable salt thereof, e.g., about 80 µg, about 112.5 µg, about 160 µg, about 225 µg, about 240 µg, about 320 µg, about 400 µg, about 450 µg, about 480 µg, about 640 µg, or 675 µg of the compound of Formula (II), a stereoisomer thereof, or pharmaceutically acceptable salt thereof. For example, if the dry powder composition is administered once daily in a single dosing session, the effective amount can be considered to be the amount of the compound of Formula (I) or (II), a stereoisomer thereof, or a pharmaceutically acceptable salt thereof, in the capsule or multiple capsules that is administered during the single dosing session. For example, in one embodiment, one or more capsules may be formulated with the dry powder composition wherein the one or more capsules have a total dose of about 80 µg, about 112.5 µg, about 160 µg, about 225 µg, about 240 µg, about 320 µg, about 400 µg, about 450 µg, about 480 µg, about 640 µg, or 675 µg of a compound of Formula (I) or (II), a stereoisomer thereof, or a pharmaceutically acceptable salt thereof, and each of the aforementioned dosages may be an effective amount, and may also be referred to as the amount administered once daily in a single dosing session, during the administration period. As a further example, in one embodiment, the capsule comprises a dry powder composition comprising about 320 µg of a compound of Formula (II), a stereoisomer thereof, or a pharmaceutically acceptable salt thereof, and, for purposes of this disclosure, the amount administered is 640 µg, even if takes 2 or more puffs from two capsules to administer the 640 µg. Similarly, in this example, the amount administered is 640 µg even if a residual amount of compound of Formula (II), a stereoisomer thereof, or a pharmaceutically acceptable salt thereof remains in the DPI (e.g., if about 5%, 10%, 20%, 30%, 40%, or 50% remains in the DPI.)

The dose "administered" in a single dosing session also encompasses situations where the DPI is refiled or reloaded 1 or more times (e.g., by changing the capsules) in order to achieve the desired effective amount. In such situations, "administration" refers to the total dosage in the capsules which are administered in the dosing session. For example, to administer a dosage of 240 µg of a compound of Formula (II), a stereoisomer thereof, or a pharmaceutically acceptable salt thereof, one 80 µg capsule and one 160 µg capsule may be used. The DPI may be filed with a first 80 µg capsule, and after emptying the cartridge in 1 or more puffs, a 160 µg capsule may be loaded in the DPI and emptied in 1 or more puffs. Both capsules are used in the same dosing session, and therefore the dose administered is 240 µg.

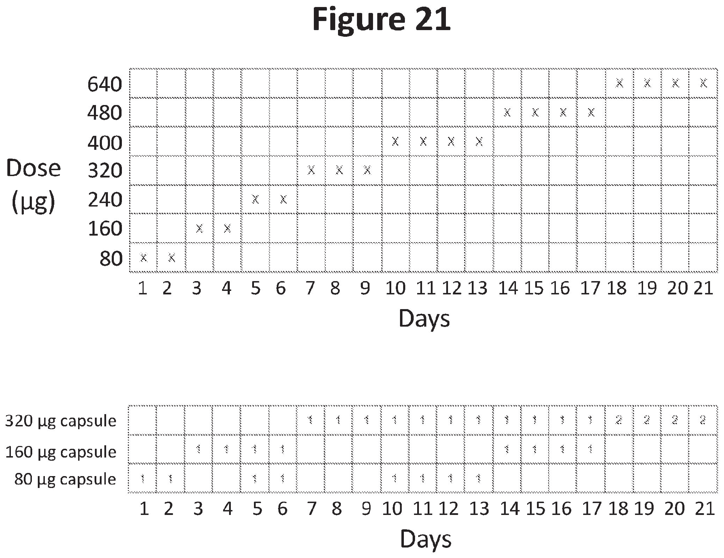

In another embodiment, the effective amount comprises an escalating dose during the administration period. In a further embodiment, the effective amount is based upon an upwards titration, based on the highest tolerated dose for the patient. In one embodiment, the patient is initially administered 80 µg. If this dose is well tolerated, the dose is uptitrated until reaching the patient's highest tolerable dose. During the titration period, the patient stays on the same dose for a minimum number of cumulative days, e.g., 2 days, 3 days or 4 days, prior to titrating to the next higher dose. See, e.g., Figure 21 for an embodiment of dose titration. If a dose is not tolerated, the dose may be decreased to the previous dose level.

During a titration period, each patient's dose can be uptitrated to the highest tolerated dose for that patient. As an example, a patient, in one embodiment, starts the method of the invention with a single 80 µg DPI capsule, once-daily. If this dose is well tolerated, the dose is uptitrated until reaching the patient's highest tolerable dose. During the Titration Period, patients stay on study drug for the minimum number of cumulative days (e.g., 2 days at 80 µg, 160 µg, or 240 µg, 3 days at 320 µg or 4 days at 400 µg or 480 µg) prior to starting the next higher dose. Study drug titration may occur slower than the above example, but not faster. Figure 21 provides an exemplary embodiment of dose titration for a patient in need of treatment. If a dose is not tolerated, the dose may be decreased to the previous dose level.

In some embodiments, the patient treated by the disclosed methods manifests one or more of the following therapeutic responses during the administration period as compared to prior to the administration period: (1) a reduction in the pulmonary vascular resistance index (PVRI), (2) a reduction in mean pulmonary artery pressure, (3) an increase in the hypoxemia score, (4) a decrease in the oxygenation index, (5) improved right heart function, and (6) improved exercise capacity (e.g., as measured by the six-minute walk test).

6MWT is a validated method for measuring exercise capacity and assessment of pulmonary function, and performed according to the American Thoracic Society (ATS) guidelines. See American Thoracic Society. ATS Statement: Guidelines for the six minute walk test. Am J Respir Crit Care Med. 2002;166(1):111-17, incorporated herein by reference in its entirety for all purposes. In one embodiment, the 6MWT is performed at approximately the same time on a day during the administration period as on a day prior to the administration period. In a further embodiment, the same equipment is used to perform the 6MWT. In still a further embodiment, the same person administers the 6MWT.

In one embodiment, the patient's distance walked in the 6MWT is increased during the administration period, as compared to prior to the administration period, by at least about 5 meters, at least about 10 meters, at least about 20 meters, at least about 30 meters, at least about 40 meters, or at least about 50 meters. In another embodiment, the patient's distance walked in the 6MWT is increased during the administration period, as compared to prior to the administration period, by from about 5 meters to about 60 meters, by from about 5 meters to about 50 meters, by from about 10 meters to about 50 meters, by from about 15 meters to about 50 meters, or by from about 20 meters to about 40 meters. In yet another embodiment, the patient's distance walked in the 6MWT is increased by at least about 30 meters, during the administration period, compared to prior to the administration period.

In one embodiment, the patient's distance walked in the 6MWT is increased during the administration period, as compared to prior to the administration period, by about 1%, by about 2%, by about 3%, by about 4%, by about 5%, by about 6%, by about 7%, by about 8%, by about 9%, by about 10%, by about 11%, by about 12%, by about 13%, by about 14%, by about 15%, by about 16%, by about 17%, by about 18%, by about 19%, by about 20%, by about 25%, by about 30%, by about 35%, by about 40%, by about 45%, by about 50%, by about 55%, by about 60%, by about 65%, by about 70%, by about 75%, by about 80%, by about 85%, or by about 90%. In another embodiment, the patient's distance walked in the 6MWT is increased during the administration period, as compared to prior to the administration period, by at least about 5%, by at least about 10%, by at least about 15%, by at least about 20%, by at least about 25%, by at least about 30%, by at least about 35%, by at least about 40%, by at least about 45%, or by at least about 50%. In another embodiment, the patient's distance walked in the 6MWT is increased during the administration period, as compared to prior to the administration period, by about 5% to about 50%, by about 5% to about 40%, by about 5% to about 30%, by about 5% to about 20%, by about 10% to about 50%, by about 15% to about 50%, by about 20% to about 50%, or by about 25% to about 50%.

In one embodiment for treating PH, treating comprises improving the quality of life of the patient during the administration period, compared to the quality of life of the patient prior to the administration period. The quality of life, in one embodiment, is measured by the Cambridge Pulmonary Hypertension Outcome Review (CAMPHOR) Questionnaire. McCabe et al. (2013). Chest. 2013;144(2):522-30, incorporated by reference herein in its entirety for all purposes. The CAMPHOR Questionnaire is a pulmonary hypertension specific measure of health-related quality of life (QOL) consisting of 3 sections that evaluate a total of 65 items (25 relating to symptoms, 15 relating to activities, and 25 relating to QOL). The CAMPHOR scoring is negatively weighted therefore, a higher score indicates worse QOL and greater functional limitation. Symptom and QOL items are both scored out of 25 and activity items have 3 possible responses (score 0-2), giving a score out of 30. Each CAMPHOR assessment takes an average of 10 minutes. In one embodiment for treating PH, treating comprises decreasing the patient's CAMPHOR Questionnaire score during the administration period, compared to the CAMPHOR Questionnaire score prior to the administration period. The decrease, in one embodiment, is by from 1 to about 10, from 1 to about 9, from 1 to 8, from 1 to 7, from 1 to 6, from 1 to 5, from 1 to 4, from 1 to 3 or from 1 to 2.

In one embodiment of a method for treating PH, the method comprises increasing the patient's saturation of peripheral capillary oxygenation (SpO₂) at rest assessed by pulse oximetry during the administration period, compared to the patient's SpO₂ at rest prior to the administration period.

Oxygen saturation is an indication of how much hemoglobin in the blood is bound to oxygen, and is typically provided as a percentage of oxyhemoglobin to the total hemoglobin. SpO₂ is an indication of oxygen saturation in the peripheral capillaries. Exemplary methods to measure SpO₂ include, but are not limited to, pulse oximetry using a pulse oximeter. In one embodiment of a method for treating PH provided herein, the method comprises increasing the patient's SpO₂ at rest during the administration period, as compared to prior to the administration period, by at least about 1%, at least about 2%, at least about 3%, at least about 4%, at least about 5%, at least about 6%, at least about 7%, at least about 8%, at least about 9%, at least about 10%, at least about 11%, at least about 12%, at least about 13%, at least about 14%, at least about 15%, at least about 16%, at least about 17%, at least about 18%, at least about 19%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or by at least about 90%. In another embodiment, the method for treating PH comprises increasing the patient's SpO₂ at rest during the administration period, as compared to prior to the administration period, by about 5% to about 50%, by about 5% to about 40%, by about 5% to about 30%, by about 5% to about 20%, by about 10% to about 50%, by about 15% to about 50%, by about 20% to about 50%, or by about 25% to about 50%.

In one embodiment, the method for treating PH provided herein comprises improving the lung function of the patient during the administration period, as compared to the lung function of the patient prior to the administration period. The improvement in lung function in one embodiment, is measured by spirometry.

Improving the lung function of the patient, in one embodiment, comprises increasing the patient's forced vital capacity (FVC), increasing the patient's percent predicted forced vital capacity (ppFVC), increasing the patient's forced expiratory volume in 1 second (FEV₁), increasing the patient's percent predicted forced expiratory volume in one second (ppFEV₁), increasing the patient's forced expiratory flow between 25% and 75% of FVC (FEF_{(25-75%)}), increasing the patient's total lung capacity (TLC), or increasing the patient's lung diffusion capacity for carbon monoxide (DLCO), during the administration period, as compared to the respective value prior to the administration period.

The assessment of lung function, e.g., via FVC, ppFVC, FEV₁ ppFEV₁, FEF(_{25-75%)}, TLC, or DLCO measurement, in one embodiment, comprises comparing the lung function in the patient prior to the administration period, e.g., immediately prior to treatment, to a time point during the administration period the administration period, or to an average of measurements taken during the administration period.

As provided herein, in one embodiment, the method for treating PH comprises improving the lung function in the patient during the administration period, as compared to the respective value prior to the administration period, wherein the lung function is measured by spirometry. Spirometry is a physiological test that measures how an individual inhales or exhales volumes of air. The primary signal measured in spirometry may be volume or flow. For the methods described herein, pulmonary function test (PFT) by spirometry (e.g., FEV₁, FVC, FEF_{(25-75%)}, and TLC) is performed per the American Thorasic Society (ATS) / European Respiratory Society (ERS) criteria, e.g., as set forth by Miller et al. (Miller et al., "Standardization of Spirometry," Eur. Respir. J. 26:319-38 (2005), incorporated by reference herein in its entirety for all purposes). DLCO can be measured using techniques described by Modi P, Cascella M, "Diffusing Capacity Of The Lungs For Carbon Monoxide," [Updated 2021 Mar 24]. In: StatPearls [Internet]. Treasure Island (FL): StatPearls Publishing; 2021 Jan- . Available from: www.ncbi.nlm.nih.gov/books/NBK556149/; Graham et al., "2017 ERS/ATS standards for single-breath carbon monoxide uptake in the lung," European Respiratory Journal 49:1600016 (2017); each of which is incorporated herein by reference in its entirety for all purposes.

In one embodiment, the spirometer is capable of accumulating volume for greater than or equal to 15 seconds, e.g., ≥ 20 seconds, ≥ 25 seconds, ≥ 30 seconds, ≥ 35 seconds. The spirometer in one embodiment can measure volumes of ≥ 8 L (BTPS) with an accuracy of at least ± 3% of reading or ± 0.050 L, whichever is greater, with flows between 0 and 14 L•s⁻¹. In one embodiment, the total resistance to airflow of the spirometer at 14 L•s⁻¹ is < 1.5 cmH₂O•L⁻¹•s⁻¹ (0.15 kPa? L⁻¹•s⁻¹). In one embodiment, the total resistance of the spirometer is measured with any tubing, valves, pre-filter, etc. included that may be inserted between the patient and the spirometer. With respect to devices that exhibit changes in resistance due to water vapor condensation, in one embodiment, spirometer accuracy requirements are met under BTPS (body temperature, ambient pressure, saturated with water vapor) conditions for up to eight successive FVC maneuvers performed in a 10-min period without inspiration from the instrument.

With respect to the forced expiratory maneuvers described herein, in one embodiment, the range and accuracy recommendations as set forth in Table 6 of Miller et al., are met (Miller et al., "Standardization of Spirometry," Eur. Respir. J. 26:319-38 (2005), incorporated by reference herein in its entirety for all purposes).

In one embodiment, improving lung function comprises improving the forced vital capacity (FVC) of the patient, i.e., the maximal volume of air exhaled with maximally forced effort from a maximal inspiration, during the administration period, as compared to the FVC prior to the administration period.. The FVC is expressed in liters at body temperature and ambient pressure saturated with water vapor (BTPS). In another embodiment, the improvement in lung function is an improvement in the percent predicted forced vital capacity (ppFVC).

"Forced vital capacity" (FVC) denotes the volume of gas which is exhaled during a forced expiration starting from a position of full inspiration and ending at complete expiration and is one measure of treatment efficacy. FVC may be expressed as a percentage of the predicted FVC (i.e., ppFVC) obtained from a normal population, based on the patient's age, height, gender, and sometimes weight and race. In one embodiment of a method for treating PH, improving the patient's lung function comprises increasing the patient's FVC or ppFVC during the administration period, compared to the patient's corresponding FVC or ppFVC prior to the administration period. The increase in FVC or ppFVC, in one embodiment, is an increase of at least about 5%, at least about 10%, at least about 15%, at least about 20%, at least about 25%, at least about 30%, at least about 35%, at least about 40%, at least about 45%, or at least about 50%. In another embodiment, the increase in FVC or ppFVC is an increase of from about 1% to about 20%, from about 1% to about 15%, from about 1% to about 10%, from about 1% to about 5%, from about 5% to about 50%, from about 5% to about 40%, from about 5% to about 30%, from about 5% to about 20%, from about 10% to about 50%, from about 15% to about 50%, from about 20% to about 50%, or from about 25% to about 50%. In one embodiment, increasing FVC or ppFVC is increasing pre-bronchodilator FVC or ppFVC. In another embodiment, increasing FVC or ppFVC is increasing post-bronchodilator FVC or ppFVC.

In one embodiment, the patient's ppFVC is 80% or less prior to the administration period. In a further embodiment, the patient's ppFVC is 70% or less prior to the administration period. In a further embodiment, the patient's ppFVC is 60% or less prior to the administration period. In a further embodiment, the patient's ppFVC is 50% or less prior to the administration period. In another embodiment, the patient's ppFVC is from 30% to 80%, from 40% to 70%, or from 50% to 60%, prior to the administration period.

FVC maneuvers can be performed according to the procedures known to those of ordinary skill in the art. Briefly, the three distinct phases to the FVC maneuver are (1) maximal inspiration; (2) a "blast" of exhalation and (3) continued complete exhalation to the end of test (EOT). The maneuver can be carried out via the closed circuit method or open circuit method. In either instance, the patient inhales rapidly and completely with a pause of less than 1 second at total lung capacity (TLC). The patient then exhales maximally until no more air can be expelled while maintaining an upright posture. The exhalation begins with a "blast" of air from the lungs and then is encouraged to fully exhale. Enthusiastic coaching of the patient continues for a minimum of three maneuvers.

FEV is the volume of gas exhaled in a specified time (typically 1 second, i.e., FEV₁) from the start of the forced vital capacity maneuver (Quanjer et al. (1993). Eur. Respir. J. 6, Suppl. 16, pp. 5-40, incorporated by reference herein in its entirety for all purposes). FEV₁ may also be expressed as a percentage of the predicted FEV₁ (i.e., ppFEV₁) obtained from a normal population, based on the patient's gender, height, and age, and sometimes race and weight.

In one embodiment, improving the lung function of the patient comprises increasing the patient's FEV₁ or ppFEV₁ during the administration period, compared to the patient's corresponding FEV₁ or ppFEV₁ prior to the administration period. The increase in FEV₁ or ppFEV₁, in one embodiment, is an increase of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 25%, about 30%, about 35%, about 40%, about 45%, about 50%, about 55%, about 60%, about 65%, about 70%, about 75%, about 80%, about 85%, or about 90%. In another embodiment, the increase in FEV₁ or ppFEV₁ is an increase of about 5%, about 10%, about 15%, about 20%, about 25%, about 30%, about 35%, about 40%, about 45%, or about 50%. In another embodiment, increasing the FEV₁ or ppFEV₁ comprises increasing by at least about 5%, at least about 10%, at least about 15%, at least about 20%, at least about 25%, at least about 30%, at least about 35%, at least about 40%, at least about 45%, or at least about 50%. In another embodiment, increasing FEV₁ or ppFEV₁ is increasing of about 5% to about 50%, about 5% to about 40%, about 5% to about 30%, about 5% to about 20%, about 10% to about 50%, about 15% to about 50%, about 20% to about 50%, or about 25% to about 50%.

In one embodiment, increasing FEV₁ or ppFEV₁ is increasing in pre-bronchodilator FEV₁ or ppFEV₁. In another embodiment, increasing FEV₁ or ppFEV₁ is increasing post-bronchodilator FEV₁ or ppFEV₁.

In one embodiment, the patient's ppFEV₁ is 80% or less prior to the administration period. In a further embodiment, the patient's ppFEV₁ is 70% or less prior to the administration period. In a further embodiment, the patient's ppFEV₁ is 60% or less prior to the administration period. In a further embodiment, the patient's ppFEV₁ is 50% or less prior to the administration period. In another embodiment, the patient's pp FEV₁ is from 30% to 80%, from 40% to 70%, or from 50% to 60%, prior to the administration period.

In another embodiment, improving the lung function of the patient comprises increasing the patient's FEV₁ during the administration period, compared to prior to the administration period, by from about 25 mL to about 500 mL, from about 25 mL to about 400 mL, from about 25 mL to about 300 mL, from about 25 mL to about 250 mL, from about 25 mL to about 200 mL, or from about 50 mL to about 200 mL, as compared to the patient's FEV₁ prior to the administration period. In one embodiment, increasing FEV₁ is increasing pre-bronchodilator FEV₁. In another embodiment, increasing FEV₁ is increasing post-bronchodilator FEV₁.

In one embodiment, improving the lung function of the patient comprises increasing the mean forced expiratory flow between 25% and 75% of FVC (FEF_{(25-75%)}) (also referred to as the maximum mid-expiratory flow) of the patient during the administration period, as compared to the patient's FEF_{(25-75%)} prior to the administration period. The FEF_{(25-75%)} measurement is dependent on the validity of the FVC measurement and the level of expiratory effort. The FEF_{(25-75%)} index is taken from the blow with the largest sum of FEV₁ and FVC.

In one embodiment, increasing the patient's FEF_{(25-75%)} during the administration period comprises increasing by at least about 1%, by at least about 5%, by at least about 10%, by at least about 15%, by at least about 20%, by at least about 25%, by at least about 30%, by at least about 35%, by at least about 40%, by at least about 45%, or by at least about 50%. In another embodiment, increasing the patient's FEF_{(25-75%)} during the administration period comprises increasing by about 5% to about 50%, by about 5% to about 40%, by about 5% to about 30%, by about 5% to about 20%, by about 10% to about 50%, by about 15% to about 50%, by about 20% to about 50%, or by about 25% to about 50%. In one embodiment, increasing FEF_{(25-75%)} is increasing pre-bronchodilator FEF_{(25-75%)}. In another embodiment, increasing FEF_{(25-75%)} is increasing post-bronchodilator FEF_{(25-75%)}.

Total lung capacity (TLC) is the sum of the vital capacity and residual volume that represents the total volume of air that can be contained in the lung. The total lung capacity (TLC) is divided into four volumes. The tidal volume (V_{T}) is the volume inhaled or exhaled in normal quiet breathing. The inspiratory reserve volume (IRV) is the maximum volume that can be inhaled following a normal quiet inhalation. The expiratory reserve volume (ERV) is the maximum volume that can be exhaled following a normal quiet exhalation. The residual volume (RV) is the volume remaining in the lungs following a maximal exhalation. The vital capacity (VC) is the maximum volume that can be exhaled following a maximal inhalation; VC=IRV+V_{T}+ERV. In one embodiment, improving the lung function of the patient comprises increasing the patient's total lung capacity (TLC) during the administration period, compared to the patient's TLC prior to the administration period. In one embodiment, increasing is by at least about 1%, at least about 2%, at least about 5%, at least about 10%, at least about 15%, at least about 20%, at least about 25%, at least about 30%, at least about 35%, at least about 40%, at least about 45%, or by at least about 50%. In another embodiment, increasing is by from about 1% to about 50%, by from about 5% to about 50%, by from about 5% to about 40%, by from about 5% to about 30%, by from about 5% to about 20%, by from about 10% to about 50%, by from about 15% to about 50%, by from about 20% to about 50%, or by from about 25% to about 50%.

Also known as the transfer factor, lung diffusion capacity for carbon monoxide (DLCO) is a measurement to assess the lungs' ability to transfer gas from inspired air to the bloodstream. Carbon monoxide (CO) has a high affinity for hemoglobin, and it follows the same pathway as that of oxygen to finally bind with hemoglobin. Inhaled CO is used for this test due to its high affinity for hemoglobin (200 to 250 times that of oxygen). As anemia can reduce DLCO, DLCO may be adjusted for hemoglobin values. DLCO may also need to be adjusted for several other factors, such as carboxyhemoglobin, FiO. See Modi P, Cascella M, "Diffusing Capacity Of The Lungs For Carbon Monoxide," [Updated 2021 Mar 24]. In: StatPearls [Internet]. Treasure Island (FL): StatPearls Publishing; 2021 Jan, incorporated herein by reference in its entirety for all purposes. In one embodiment, improving the lung function of the patient comprises increasing the patient's DLCO during the administration period, compared to the patient's DLCO prior to the administration period. In one embodiment, DLCO is adjusted for hemoglobin level, i.e., improving the lung function of the patient comprises increasing the patient's DLCO adjusted for hemoglobin during the administration period compared to the patient's DLCO adjusted for hemoglobin prior to the administration period. In another embodiment, improving the lung function of the patient comprises increasing the patient's DLCO percent (DLCO %) predicted during the administration period compared to the patient's DLCO % predicted prior to the administration period. Predicted normal DLCO values may be calculated according to the equation established by Crapo et al., Am Rev Respir Dis. 123(2):185-9 (1981), or according to the equation established by Miller et al., Am Rev Respir Dis. 127(3):270-7 (1983), each of which is incorporated by reference in its entirety for all purposes. In a further embodiment, the patient's DLCO % predicted is adjusted for hemoglobin.

In one embodiment, improving lung function comprises increasing the patient's DLCO or DLCO % predicted by at least about 1%, at least about 5%, at least about 10%, at least about 15%, at least about 20%, at least about 25%, at least about 30%, at least about 35%, at least about 40%, at least about 45%, or by at least about 50%. In another embodiment, improving lung function comprises increasing the patient's DLCO or DLCO % predicted by from about 5% to about 50%, by from about 5% to about 40%, by from about 5% to about 30%, by from about 5% to about 20%, by from about 10% to about 50%, by from about 15% to about 50%, by from about 20% to about 50%, or by from about 25% to about 50%. In a further embodiment, the patient's DLCO or DLCO % predicted is adjusted for hemoglobin.

In one embodiment, the patient's DLCO % predicted is 80% or less, 70% or less, 60% or less, or 50% or less, prior to the administration period. In a further embodiment, the patient's DLCO % predicted is adjusted for hemoglobin. In another embodiment, the patient's DLCO % predicted is from 30% to 80%, from 40% to 70%, or from 50% to 60%, prior to the administration period. In a further embodiment, the patient's DLCO % predicted is adjusted for hemoglobin.

In one embodiment of a method for treating PH provided herein, the method comprises increasing the length of time to clinical worsening, as compared to an untreated PH patient, or a PH patient not treated with a compound of Formula (I) or (II), wherein the clinical worsening is one selected from the group consisting of death, hospitalization due to a respiratory indication (e.g., dyspnea, and/or deterioration of lung function indicated by reductions in FVC, DLCO, and/or SpO₂), 10% or greater decline in percent predicted FVC (ppFVC) relative to the patient's ppFVC prior to the administration period on two consecutive occasions 4-14 weeks apart, lung transplantation, and 15% or greater decrease in distance walked in a 6-minute walk test (6MWT) relative to the patient's distance walked in a 6MWT prior to the administration period on two consecutive occasions at least 24 hours apart.

In one embodiment, the length of time to clinical worsening is increased by about 1 day, about 3 days, about 1 week, about 2 weeks, about 3 weeks, about 4 weeks, about 5 weeks, or about 6 weeks. In another embodiment, the length of time to clinical worsening is increased by at least about 1 day, at least about 3 days, at least about 1 week, at least about 2 weeks, at least about 3 weeks, at least about 4 weeks, at least about 5 weeks, or at least about 6 weeks. In another embodiment, the length of time to clinical worsening is increased about 20 days to about 100 days, about 30 days to about 100 days, about 20 days to about 75 days, about 20 days to about 50 days, or about 20 days to about 40 days. In another embodiment, the length of time to clinical worsening is increased at least 1 month, e.g., about 1 month to about 6 months, about 1 month to about 4 months, or about 1 month to about 3 months.

In one embodiment, a method for treating PH provided herein comprises increasing the patient's lung lobar volume and/or airway volume assessed by computerized tomography (CT) during the administration period, compared to the patient's lung lobar volume and/or airway volume prior to the administration period. CT may be performed via chest CT scan during a breathing cycle to generate CT images at functional residual capacity (FRC) and/or total lung capacity (TLC). In one embodiment, the lung lobar volume is the volume of the lung lobar structure of the patient's respiratory system at TLC or FRC, and the airway volume is the volume of the airway structure of the patient's respiratory system at TLC or FRC.

In one embodiment, increasing the patient's lung lobar volume and/or airway volume comprises increasing by at least about 1%, at least about 5%, at least about 10%, at least about 15%, at least about 20%, at least about 25%, at least about 30%, at least about 35%, at least about 40%, at least about 45%, or by at least about 50%. In another embodiment, the patient's lung lobar volume and/or airway volume is increased by from about 5% to about 50%, by from about 5% to about 40%, by from about 5% to about 30%, by from about 5% to about 20%, by from about 10% to about 50%, by from about 15% to about 50%, by from about 20% to about 50%, or by from about 25% to about 50%.

### Additional Embodiments

Embodiment 1. A dry powder composition comprising:
(a) from about 0.1 wt% to about 5 wt% of a compound of Formula (I): 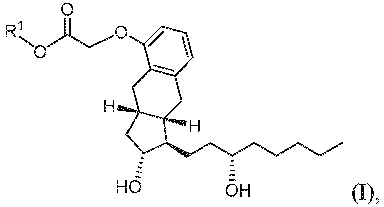 or an enantiomer, diastereomer, or a pharmaceutically acceptable salt thereof, wherein R¹ is tetradecyl, pentadecyl, hexadecyl, heptadecyl, or octadecyl,
(b) from about 10 wt% to about 50 wt% of leucine, and
the balance being (c) a sugar selected from the group consisting of trehalose and mannitol, wherein the entirety of (a), (b), and (c) is 100 wt%.

Embodiment 2. The dry powder composition of Embodiment 1, wherein (a) is a compound of Formula (I) or a pharmaceutically acceptable salt thereof.

Embodiment 3. The dry powder composition of Embodiment 1 or 2, wherein (a) is a compound of Formula (I).

Embodiment 4. The dry powder composition of any one of Embodiments 1-3, wherein R¹ is tetradecyl.

Embodiment 5. The dry powder composition of Embodiment 4, wherein R¹ is linear tetradecyl.

Embodiment 6. The dry powder composition of any one of Embodiments 1-3, wherein R¹ is pentadecyl.

Embodiment 7. The dry powder composition of Embodiment 6, wherein R¹ is linear pentadecyl.

Embodiment 8. The dry powder composition of any one of Embodiments 1-3, wherein R¹ is heptadecyl.

Embodiment 9. The dry powder composition of Embodiment 8, wherein R¹ is linear heptadecyl.

Embodiment 10. The dry powder composition of any one of Embodiments 1-3, wherein R¹ is octadecyl.

Embodiment 11. The dry powder composition of Embodiment 10, wherein R¹ is linear octadecyl.

Embodiment 12. The dry powder composition of any one of Embodiments 1-3, wherein R¹ is hexadecyl.

Embodiment 13. The dry powder composition of Embodiment 12, wherein R¹ is linear hexadecyl.

Embodiment 14. The dry powder composition of any one of Embodiments 1-13, wherein the compound of Formula (I), or an enantiomer, diastereomer, or a pharmaceutically acceptable salt thereof is present at from about 0.1 wt% to about 4.5 wt% of the total weight of the dry powder composition.

Embodiment 15. The dry powder composition of Embodiment 14, wherein the compound of Formula (I), or a pharmaceutically acceptable salt thereof is present at from about 0.1 wt% to about 4.5 wt% of the total weight of the dry powder composition.

Embodiment 16. The dry powder composition of Embodiment 14 or 15, wherein the compound of Formula (I) is present at from about 0.1 wt% to about 4.5 wt% of the total weight of the dry powder composition.

Embodiment 17. The dry powder composition of any one of Embodiments 1-13, wherein the compound of Formula (I), or an enantiomer, diastereomer, or a pharmaceutically acceptable salt thereof is present at from about 0.1 wt% to about 4 wt% of the total weight of the dry powder composition.

Embodiment 18. The dry powder composition of Embodiment 17, wherein the compound of Formula (I), or a pharmaceutically acceptable salt thereof is present at from about 0.1 wt% to about 4 wt% of the total weight of the dry powder composition.

Embodiment 19. The dry powder composition of Embodiment 17 or 18, wherein the compound of Formula (I) is present at from about 0.1 wt% to about 4 wt% of the total weight of the dry powder composition.

Embodiment 20. The dry powder composition of any one of Embodiments 1-13, wherein the compound of Formula (I), or an enantiomer, diastereomer, or a pharmaceutically acceptable salt thereof is present at from about 0.1 wt% to about 3.5 wt% of the total weight of the dry powder composition.

Embodiment 21. The dry powder composition of Embodiment 20, wherein the compound of Formula (I), or a pharmaceutically acceptable salt thereof is present at from about 0.1 wt% to about 3.5 wt% of the total weight of the dry powder composition.

Embodiment 22. The dry powder composition of Embodiment 20 or 21, wherein the compound of Formula (I) is present at from about 0.1 wt% to about 3.5 wt% of the total weight of the dry powder composition.

Embodiment 23. The dry powder composition of any one of Embodiments 1-13, wherein the compound of Formula (I), or an enantiomer, diastereomer, or a pharmaceutically acceptable salt thereof is present at from about 0.1 wt% to about 3 wt% of the total weight of the dry powder composition.

Embodiment 24. The dry powder composition of Embodiment 23, wherein the compound of Formula (I), or a pharmaceutically acceptable salt thereof is present at from about 0.1 wt% to about 3 wt% of the total weight of the dry powder composition.

Embodiment 25. The dry powder composition of Embodiment 23 or 24, wherein the compound of Formula (I) is present at from about 0.1 wt% to about 3 wt% of the total weight of the dry powder composition.

Embodiment 26. The dry powder composition of any one of Embodiments 1-13, wherein the compound of Formula (I), or an enantiomer, diastereomer, or a pharmaceutically acceptable salt thereof is present at from about 0.5 wt% to about 3.5 wt%, or from about 0.8 wt% to about 3.3 wt%, of the total weight of the dry powder composition.

Embodiment 27. The dry powder composition of Embodiment 26, wherein the compound of Formula (I), or a pharmaceutically acceptable salt thereof is present at from about 0.5 wt% to about 3.5 wt%, or from about 0.8 wt% to about 3.3 wt%, of the total weight of the dry powder composition.

Embodiment 28. The dry powder composition of Embodiment 26 or 27, wherein the compound of Formula (I) is present at from about 0.5 wt% to about 3.5 wt%, or from about 0.8 wt% to about 3.3 wt%, of the total weight of the dry powder composition.

Embodiment 29. The dry powder composition of any one of Embodiments 1-13, wherein the compound of Formula (I), or an enantiomer, diastereomer, or a pharmaceutically acceptable salt thereof is present at from about 1 wt% to about 2 wt% of the total weight of the dry powder composition.

Embodiment 30. The dry powder composition of Embodiment 29, wherein the compound of Formula (I), or a pharmaceutically acceptable salt thereof is present at from about 1 wt% to about 2 wt% of the total weight of the dry powder composition.

Embodiment 31. The dry powder composition of Embodiment 29 or 30, wherein the compound of Formula (I) is present at from about 1 wt% to about 2 wt% of the total weight of the dry powder composition.

Embodiment 32. The dry powder composition of any one of Embodiments 1-13, wherein the compound of Formula (I), or an enantiomer, diastereomer, or a pharmaceutically acceptable salt thereof is present at from about 1.2 wt% to about 1.8 wt% of the total weight of the dry powder composition.

Embodiment 33. The dry powder composition of Embodiment 32, wherein the compound of Formula (I), or a pharmaceutically acceptable salt thereof is present at from about 1.2% wt to about 1.8 wt% of the total weight of the dry powder composition.

Embodiment 34. The dry powder composition of Embodiment 32 or 33, wherein the compound of Formula (I) is present at from about 1.2 wt% to about 1.8 wt% of the total weight of the dry powder composition.

Embodiment 35. The dry powder composition of any one of Embodiments 1-13, wherein the compound of Formula (I), or an enantiomer, diastereomer, or a pharmaceutically acceptable salt thereof is present at from about 1 wt% to about 1.5 wt% of the total weight of the dry powder composition.

Embodiment 36. The dry powder composition of Embodiment 35, wherein the compound of Formula (I), or a pharmaceutically acceptable salt thereof is present at from about 1 wt% to about 1.5 wt% of the total weight of the dry powder composition.

Embodiment 37. The dry powder composition of Embodiment 35 or 36, wherein the compound of Formula (I) is present at from about 1 wt% to about 1.5 wt% of the total weight of the dry powder composition.

Embodiment 38. The dry powder composition of any one of Embodiments 1-13, wherein the compound of Formula (I), or an enantiomer, diastereomer, or a pharmaceutically acceptable salt thereof is present at from about 1.4 wt% to about 1.6 wt% of the total weight of the dry powder composition.

Embodiment 39. The dry powder composition of Embodiment 38, wherein the compound of Formula (I), or a pharmaceutically acceptable salt thereof is present at from about 1.4 wt% to about 1.6 wt% of the total weight of the dry powder composition.

Embodiment 40. The dry powder composition of Embodiment 38 or 39, wherein the compound of Formula (I) is present at from about 1.4 wt% to about 1.6 wt% of the total weight of the dry powder composition.

Embodiment 41. The dry powder composition of any one of Embodiments 1-13, wherein the compound of Formula (I), or an enantiomer, diastereomer, or a pharmaceutically acceptable salt thereof is present at about 1 wt% of the total weight of the dry powder composition.

Embodiment 42. The dry powder composition of Embodiment 41, wherein the compound of Formula (I), or a pharmaceutically acceptable salt thereof is present at about 1 wt% of the total weight of the dry powder composition.

Embodiment 43. The dry powder composition of Embodiment 41 or 42, wherein the compound of Formula (I) is present at about 1 wt% of the total weight of the dry powder composition.

Embodiment 44. The dry powder composition of any one of Embodiments 1-13, wherein the compound of Formula (I), or an enantiomer, diastereomer, or a pharmaceutically acceptable salt thereof is present at about 1.5 wt% of the total weight of the dry powder composition.

Embodiment 45. The dry powder composition of Embodiment 44, wherein the compound of Formula (I), or a pharmaceutically acceptable salt thereof is present at about 1.5 wt% of the total weight of the dry powder composition.

Embodiment 46. The dry powder composition of Embodiment 44 or 45, wherein the compound of Formula (I) is present at about 1.5 wt% of the total weight of the dry powder composition.

Embodiment 47. The dry powder composition of any one of Embodiments 1-13, wherein the compound of Formula (I), or an enantiomer, diastereomer, or a pharmaceutically acceptable salt thereof is present at from about 0.5 wt% to about 1.5 wt% of the total weight of the dry powder composition.

Embodiment 48. The dry powder composition of Embodiment 47, wherein the compound of Formula (I), or a pharmaceutically acceptable salt thereof is present at from about 0.5 wt% to about 1.5 wt% of the total weight of the dry powder composition.

Embodiment 49. The dry powder composition of Embodiment 47 or 48, wherein the compound of Formula (I) is present at from about 0.5 wt% to about 1.5 wt% of the total weight of the dry powder composition.

Embodiment 50. The dry powder composition of any one of Embodiments 1-13, wherein the compound of Formula (I), or an enantiomer, diastereomer, or a pharmaceutically acceptable salt thereof is present at from about 0.7 wt% to about 1.3 wt% of the total weight of the dry powder composition.

Embodiment 51. The dry powder composition of Embodiment 50, wherein the compound of Formula (I), or a pharmaceutically acceptable salt thereof is present at from about 0.7 wt% to about 1.3 wt% of the total weight of the dry powder composition.

Embodiment 52. The dry powder composition of Embodiment 50 or 51, wherein the compound of Formula (I) is present at from about 0.7 wt% to about 1.3 wt% of the total weight of the dry powder composition.

Embodiment 53. The dry powder composition of any one of Embodiments 1-13, wherein the compound of Formula (I), or an enantiomer, diastereomer, or a pharmaceutically acceptable salt thereof is present at from about 0.8 wt% to about 1.2 wt% of the total weight of the dry powder composition.

Embodiment 54. The dry powder composition of Embodiment 53, wherein the compound of Formula (I), or a pharmaceutically acceptable salt thereof is present at from about 0.8 wt% to about 1.2 wt% of the total weight of the dry powder composition.

Embodiment 55. The dry powder composition of Embodiment 53 or 54, wherein the compound of Formula (I) is present at from about 0.8 wt% to about 1.2 wt% of the total weight of the dry powder composition.

Embodiment 56. The dry powder composition of any one of Embodiments 1-13, wherein the compound of Formula (I), or an enantiomer, diastereomer, or a pharmaceutically acceptable salt thereof is present at from about 0.9 wt% to about 1.1 wt% of the total weight of the dry powder composition.

Embodiment 57. The dry powder composition of claim 56, wherein the compound of Formula (I), or a pharmaceutically acceptable salt thereof is present at from about 0.9 wt% to about 1.1 wt% of the total weight of the dry powder composition.

Embodiment 58. The dry powder composition of Embodiment 56 or 57, wherein the compound of Formula (I) is present at from about 0.9 wt% to about 1.1 wt% of the total weight of the dry powder composition.

Embodiment 59. The dry powder composition of any one of Embodiments 1-13, wherein the compound of Formula (I), or an enantiomer, diastereomer, or a pharmaceutically acceptable salt thereof is present at from about 1.5 wt% to about 3.5 wt% of the total weight of the dry powder composition.

Embodiment 60. The dry powder composition of claim 59, wherein the compound of Formula (I), or a pharmaceutically acceptable salt thereof is present at from about 1.5 wt% to about 3.5 wt% of the total weight of the dry powder composition.

Embodiment 61. The dry powder composition of claim 59 or 60, wherein the compound of Formula (I) is present at from about 1.5 wt% to about 3.5 wt% of the total weight of the dry powder composition.

Embodiment 62. The dry powder composition of any one of Embodiments 1-13, wherein the compound of Formula (I), or an enantiomer, diastereomer, or a pharmaceutically acceptable salt thereof is present at from about 2.5 wt% to about 3.5 wt% of the total weight of the dry powder composition.

Embodiment 63. The dry powder composition of Embodiment 62, wherein the compound of Formula (I), or a pharmaceutically acceptable salt thereof is present at from about 2.5 wt% to about 3.5 wt% of the total weight of the dry powder composition.

Embodiment 64. The dry powder composition of Embodiment 62 or 63, wherein the compound of Formula (I) is present at from about 2.5 wt% to about 3.5 wt% of the total weight of the dry powder composition.

Embodiment 65. The dry powder composition of any one of Embodiments 1-13, wherein the compound of Formula (I), or an enantiomer, diastereomer, or a pharmaceutically acceptable salt thereof is present at from about 2.7 wt% to about 3.3 wt% of the total weight of the dry powder composition.

Embodiment 66. The dry powder composition of claim 65, wherein the compound of Formula (I), or a pharmaceutically acceptable salt thereof is present at from about 2.7 wt% to about 3.3 wt% of the total weight of the dry powder composition.

Embodiment 67. The dry powder composition of Embodiment 65 or 66, wherein the compound of Formula (I) is present at from about 2.7 wt% to about 3.3 wt% of the total weight of the dry powder composition.

Embodiment 68. The dry powder composition of any one of Embodiments 1-13, wherein the compound of Formula (I), or an enantiomer, diastereomer, or a pharmaceutically acceptable salt thereof is present at from about 2.8 wt% to about 3.2 wt% of the total weight of the dry powder composition.

Embodiment 69. The dry powder composition of Embodiment 68, wherein the compound of Formula (I), or a pharmaceutically acceptable salt thereof is present at from about 2.8 wt% to about 3.2 wt% of the total weight of the dry powder composition.

Embodiment 70. The dry powder composition of Embodiment 68 or 69, wherein the compound of Formula (I) is present at from about 2.8 wt% to about 3.2 wt% of the total weight of the dry powder composition.

Embodiment 71. The dry powder composition of any one of Embodiments 1-13, wherein the compound of Formula (I), or an enantiomer, diastereomer, or a pharmaceutically acceptable salt thereof is present at from about 2.9 wt% to about 3.1 wt% of the total weight of the dry powder composition.

Embodiment 72. The dry powder composition of Embodiment 71, wherein the compound of Formula (I), or a pharmaceutically acceptable salt thereof is present at from about 2.9 wt% to about 3.1 wt% of the total weight of the dry powder composition.

Embodiment 73. The dry powder composition of Embodiment 71 or 72, wherein the compound of Formula (I) is present at from about 2.9 wt% to about 3.1 wt% of the total weight of the dry powder composition.

Embodiment 74. The dry powder composition of any one of Embodiments 1-13, wherein the compound of Formula (I), or an enantiomer, diastereomer, or a pharmaceutically acceptable salt thereof is present at about 3 wt% of the total weight of the dry powder composition.

Embodiment 75. The dry powder composition of Embodiment 74, wherein the compound of Formula (I), or a pharmaceutically acceptable salt thereof is present at about 3 wt% of the total weight of the dry powder composition.

Embodiment 76. The dry powder composition of Embodiment 74 or 75, wherein the compound of Formula (I) is present at about 3 wt% of the total weight of the dry powder composition.

Embodiment 77. The dry powder composition of any one of Embodiments 1-76, wherein the leucine is present at from about 12 wt% to about 42 wt% of the total weight of the dry powder composition.

Embodiment 78. The dry powder composition of Embodiment 77, wherein the leucine is present at from about 15 wt% to about 40 wt% of the total weight of the dry powder composition.

Embodiment 79. The dry powder composition of Embodiment 78, wherein the leucine is present at from about 18 wt% to about 33 wt% of the total weight of the dry powder composition.

Embodiment 80. The dry powder composition of Embodiment 79, wherein the leucine is present at from about 20 wt% to about 33 wt% of the total weight of the dry powder composition.

Embodiment 81. The dry powder composition of Embodiment 80, wherein the leucine is present at from about 25 wt% to about 33 wt% of the total weight of the dry powder composition.

Embodiment 82. The dry powder composition of Embodiment 81, wherein the leucine is present at from about 27 wt% to about 33 wt% of the total weight of the dry powder composition.

Embodiment 83. The dry powder composition of Embodiment 82, wherein the leucine is present at from about 27 wt% to about 31 wt% of the total weight of the dry powder composition.

Embodiment 84. The dry powder composition of Embodiment 83, wherein the leucine is present at from about 27 wt% to about 30 wt% of the total weight of the dry powder composition.

Embodiment 85. The dry powder composition of Embodiment 84, wherein the leucine is present at from about 28 wt% to about 30 wt% of the total weight of the dry powder composition.

Embodiment 86. The dry powder composition of Embodiment 80, wherein the leucine is present at about 20 wt% of the total weight of the dry powder composition.

Embodiment 87. The dry powder composition of Embodiment 80, wherein the leucine is present at about 30 wt% of the total weight of the dry powder composition.

Embodiment 88. The dry powder composition of any one of Embodiments 1-87, wherein the sugar is trehalose.

Embodiment 89. The dry powder composition of any one of Embodiments 1-87, wherein the sugar is mannitol.

Embodiment 90. The dry powder composition of any one of Embodiments 1-13, which comprises (a) about 1.5 wt% of the compound of Formula (I), or an enantiomer, diastereomer, or a pharmaceutically acceptable salt thereof, (b) about 29.3 wt% of the leucine, and the balance being (c) mannitol.

Embodiment 91. The dry powder composition of Embodiment 90, which comprises (a) about 1.5 wt% of the compound of Formula (I), or a pharmaceutically acceptable salt thereof, (b) about 29.3 wt% of the leucine, and the balance being (c) mannitol.

Embodiment 92. The dry powder composition of Embodiment 90 or 91, which comprises (a) about 1.5 wt% of the compound of Formula (I), (b) about 29.3 wt% of the leucine, and the balance being (c) mannitol.

Embodiment 93. The dry powder composition of any one of Embodiments 1-13, which comprises (a) about 1 wt% of the compound of Formula (I), or an enantiomer, diastereomer, or a pharmaceutically acceptable salt thereof, (b) about 29.3 wt% of the leucine, and the balance being (c) mannitol.

Embodiment 94. The dry powder composition of Embodiment 93, which comprises (a) about 1 wt% of the compound of Formula (I), or a pharmaceutically acceptable salt thereof, (b) about 29.3 wt% of the leucine, and the balance being (c) mannitol.

Embodiment 95. The dry powder composition of Embodiment 93 or 94, which comprises (a) about 1 wt% of the compound of Formula (I), (b) about 29.3 wt% of the leucine, and the balance being (c) mannitol.

### EXAMPLES

The present invention is further illustrated by reference to the following Examples. However, it should be noted that these Examples, like the embodiments described above, are illustrative and are not to be construed as restricting the scope of the invention in any way.

The following examples relate to two different treprostinil palmitil inhalation powder (TPIP) formulations (TPIP-A and TPIP-B). The compositions of TPIP-A and TPIP-B expressed in weight ratios, targeted weight percentages calculated based on the weight ratios, and actual weight percentages of the components from a typical batch of each formulation are summarized in Tables D and E, respectively.

| **Table D.** Composition of TPIP-A in weight ratio, targeted weight percentages, and actual weight percentages of components from a typical batch. | | | | | |
|---|---|---|---|---|---|
| **Composition Treprostinil Palmitil/DSPE-PEG2000/Man/Leu Wt ratio** | **Composition Wt%** | | | | |
| | **Treprostinil Palmitil** | **DSPE-PEG2000** | **Mannitol** | **Leucine** | **Total** |
| 1.5/0.75/70/30 | Targeted | | | | |
| | 1.47 | 0.73 | 68.46 | 29.34 | 100 |
| | Actual* | | | | |
| | 1.50 | 0.75 | 68.45 | 29.30 | 100 |
| * The actual wt% values shown are typical wt% values for the components in TPIP-A. Batches of TPIP-A with wt% for each component independently varying at or within ±5% of the typical wt% value as shown were observed to have equivalent properties and performance. | | | | | |

| **Table E.** Composition of TPIP-B in weight ratio, targeted weight percentages, and actual weight percentages of components from a typical batch. | | | | | |
|---|---|---|---|---|---|
| **Composition Treprostinil Palmitil/DSPE-PEG2000/Man/Leu Wt ratio** | **Composition Wt%** | | | | |
| | **Treprostinil Palmitil** | **DSPE-PEG2000** | **Mannitol** | **Leucine** | **Total** |
| 1.5/0/70/30 | Targeted | | | | |
| | 1.48 | 0 | 68.96 | 29.56 | 100 |
| | Actual* | | | | |
| | 1.50 | 0 | 69.20 | 29.30 | 100 |
| * The actual wt% values shown are typical wt% values for the components in TPIP-B. Batches of TPIP-B with wt% for each component independently varying at or within ±5% of the typical wt% value as shown were observed to have equivalent properties and performance. | | | | | |

### Example 1: Manufacture, characterization, and encapsulation of inhalable treprostinil palmitil dry powder formulation

This example describes the manufacture by spray drying and encapsulation of TPIP-B. This example also describes the characterization of TPIP-B in parallel with TPIP-A for water content, residual solvents, particle morphology using scanning electron microscopy (SEM), particle size distribution, and thermal properties.

### 1. Spray drying manufacture of TPIP-B

Spray dried TPIP-B was manufactured using a BLD-200 spray dryer with a 200 kg/hr drying gas flow rate capacity. Specifically, a spray solution was prepared according to the composition shown in Table 1.

| **Table 1.** Composition of the spray solution | | |
|---|---|---|
| **Component** | **Weight Tolerance^{a} (%)** | **Spray Solution Composition (%)** |
| Treprostinil Palmitil | ± 0.5 | 0.030 |
| Mannitol | ± 0.5 | 1.384 |
| L-Leucine | ± 0.5 | 0.586 |
| Water | ± 1.5 | 49.0 |
| N-Propanol | ± 1.5 | 49.0 |
| Total | | 100.0 |
| ^{a .}Tolerance based on mass of solution component added, not spray solution composition. | | |

The composition of the final spray dried TPIP-B is shown in Table 2.

| **Table 2.** Composition of the final spray dried TPIP-B | |
|---|---|
| **Component** | **SDP Composition (mg/g)** |
| Treprostinil Palmitil | 15 |
| Mannitol | 692 |
| L-Leucine | 293 |

The process for the manufacture of the spray dried TPIP-B is summarized in Table 3.

| **Table 3.** Summary of the process for the manufacture of the spray dried TPIP-B | | | |
|---|---|---|---|
| **Process step** | **Process step description** | | |
| 1. Solvent addition #1 | Add 100% N-propanol to solution preparation tank #1. | | |
| 2. Active pharmaceutical ingredient (API) addition | Add the API (Treprostinil Palmitil) to the solution preparation tank #1, using agitation. Mix the solution after the API has been added. | | |
| 3. Solvent addition #2 | Add water to solution preparation tank #1. | | |
| 4. Excipient addition #1 | Add mannitol to the solution preparation tank #1, using agitation. Mix the solution after the excipient has been added. | | |
| 5. Excipient addition #2 | Add L-leucine to the solution preparation tank #1, using agitation. Mix the solution after the excipient has been added. | | |
| 6. Spray drying | Use room temperature 50/50 purified water/N-propanol for warm-up and shutdown of the spray dryer. Spray dry at the following operating conditions. | | |
| | | Target | Target range |
| | Process gas inlet temperature | 150 °C | 120 - 180 °C |
| | Process gas outlet temperature | 60 °C | 55 - 65 °C |
| | Process gas flow rate | 2720 g/min | 2420 - 3020 g/min |
| | Liquid feed flow rate | 110 g/min | 100 - 120 g/min |
| | Atomization pressure | 35 psig | 32 - 38 psig |

### 2. Analytical characterization and stability study of TPIP-B

TPIP-B, as well as TPIP-A, was manufactured, packaged in high-density polyethylene bottles enclosed in low-density polyethylene bags with desiccant and then sealed in foil bags, and stored at 2-8 °C. Initial analytical characterization as well as the stability study was performed afterwards. The initial analytical characterization included water content, residual solvents, particle morphology using SEM, particle size distribution, and thermal properties. The methodologies for the above-mentioned analytical characterization were described in U.S. Application No. 16/860,428, the disclosure of which is incorporated herein by reference in its entirety. The physical stability of the two spray dried powder formulations was assessed at 25 °C/60 % RH and 40 °C/75 % RH storage conditions for 1, 3, and 6 months and based on the changes from the initial time point in thermal properties, water content, particle size distribution, particle morphology using SEM.

Table 4 is a summary of the results of the initial characterization of TPIP-B and TPIP-A, indicating that TPIP-B and TPIP-A had similar characteristics measured.

| **Table 4.** Summary of the results of the initial characterization | | |
|---|---|---|
| | **TPIP-B** | **TPIP-A** |
| Water Content (Wt.%) | 0.24 ± 0.00 | 0.25 ± 0.01 |
| Residual Solvents n-propanol (ppm) n=2 | 1050, 920 | 1040, 980 |
| Morphology by SEM | Collapsed spheres Rough surfaces | Collapsed spheres Rough surfaces |

| Particle Size Distribution | | |
|---|---|---|
| D(v 0.1), µm | 0.3 ± 0.0 | 0.3 ± 0.0 |
| D(v 0.5), µm | 1.9 ± 0.0 | 1.8 ± 0.0 |
| D(v 0.9), µm | 4.2 ± 0.0 | 3.9 ± 0.0 |
| Thermal Properties by modulated differential scanning calorimetry (mDSC) | 164 | 164 |
| Tm (°C) | | |

Tables 5A, 5B, and 5C show the results of the stability study at 1, 3, and 6 months, respectively. The results indicate that TPIP-B and TPIP-A had similar stability profiles.

| **Table 5A.** Stability study results at 1 month | | | | |
|---|---|---|---|---|
| | **TPIP-B** | | **TPIP-A** | |
| | **25 °C/60 % RH** | **40 °C/75 % RH** | **25 °C/60 % RH** | **40 °C/75 % RH** |
| Water Content (Wt. %) | 0.2 ± 0.0 | 0.1 ± 0.0 | 0.1 ± 0.0 | 0.2 ± 0.0 |
| Morphology by SEM | Collapsed spheres Rough surfaces | Collapsed spheres Filaments | Collapsed spheres Rough surfaces | Collapsed spheres Filaments |

| Particle Size Distribution | | | | |
|---|---|---|---|---|
| D(v 0.1), µm | 0.3 ± 0.0 | 0.3 ± 0.0 | 0.3 ± 0.0 | 0.3 ± 0.0 |
| D(v 0.5), µm | 1.9 ± 0.0 | 2.0 ± 0.1 | 1.8 ± 0.0 | 1.9 ± 0.0 |
| D(v 0.9), µm | 4.3 ± 0.0 | 4.4 ± 0.1 | 4.0 ± 0.0 | 4.2 ± 0.0 |
| Thermal Properties by modulated differential scanning calorimetry (mDSC) Tm (°C) | 164 | 165 | 165 | 164 |

| **Table 5B.** Stability study results at 3 months | | | | |
|---|---|---|---|---|
| | **TPIP-B** | | **TPIP-A** | |
| | **25 °C/60 % RH** | **40 °C/75 % RH** | **25 °C/60 % RH** | **40 °C/75 % RH** |
| Water Content (Wt. %) | 0.2 ± 0.0 | 0.2 ± 0.0 | 0.2 ± 0.0 | 0.2 ± 0.0 |
| Morphology by SEM | Collapsed spheres Rough surfaces | Collapsed spheres Filaments | Collapsed spheres Rough surfaces | Collapsed spheres Filaments |

| Particle Size Distribution | | | | |
|---|---|---|---|---|
| D(v 0.1), µm | 0.3 ± 0.0 | 0.3 ± 0.0 | 0.3 ± 0.0 | 0.3 ± 0.0 |
| D(v 0.5), µm | 1.9 ± 0.0 | 2.0 ± 0.1 | 1.8 ± 0.0 | 1.8 ± 0.0 |
| D(v 0.9), µm | 4.3 ± 0.0 | 4.4 ± 0.1 | 4.1 ± 0.0 | 4.1 ± 0.0 |
| Thermal Properties by modulated differential scanning calorimetry (mDSC) | 165 | 165 | 164 | 164 |
| Tm (°C) | | | | |

| **Table 5C.** Stability study results at 6 months | | | | |
|---|---|---|---|---|
| | **TPIP-B** | | **TPIP-A** | |
| | **25 °C/60 % RH** | **40 °C/75 % RH** | **25 °C/60 % RH** | **40 °C/75 % RH** |
| Water Content (Wt.%) | 0.1 ± 0.0 | 0.1 ± 0.0 | 0.1 ± 0.0 | 0.1 ± 0.0 |
| Morphology by SEM | Collapsed spheres | Collapsed spheres Protrusions | Collapsed spheres | Collapsed spheres Protrusions |

| Particle Size Distribution | | | | |
|---|---|---|---|---|
| D(v 0.1), µm | 0.3 ± 0.0 | 0.3 ± 0.0 | 0.3 ± 0.0 | 0.3 ± 0.0 |
| D(v 0.5), µm | 1.9 ± 0.1 | 1.9 ± 0.0 | 1.8 ± 0.0 | 1.9 ± 0.0 |
| D(v 0.9), µm | 4.3 ± 0.1 | 4.3 ± 0.0 | 4.1 ± 0.0 | 4.1 ± 0.0 |
| Thermal Properties by modulated differential scanning calorimetry (mDSC) | 164 | 164 | 164 | 164 |
| Tm (°C) | | | | |

### 3. Powder encapsulation

Approximately 7.5 mg of spray dried TPIP-B was loaded into a size # 3 hydroxypropyl methylcellulose (HPMC) DPI grade capsule by using an Xcelodose 600S. Three sets of capsules were prepared, packaged in high-density polyethylene bottles enclosed in low-density polyethylene bags with desiccant and then sealed in foil bags, and stored at 2-8 °C. The fine particle doses (FPDs) and MMAD by NGI of the dry powder formulation from the stored capsules were then determined. The FPD and MMAD results are shown in Table 6. Additionally, the amount of treprostinil palmitil per capsule was determined to be 114.3 mcg.

| **Table 6.** FPD and MMAD data of encapsulated TPIP-B | | |
|---|---|---|
| | **FPD (µg)** | **MMAD (µm)** |
| Set 1 | 60.9 | 2.7 |
| Set 2 | 62.0 | 2.5 |
| Set 3 | 60.6 | 2.8 |
| Mean | 61.2 | 2.6 |

### Example 2: Pharmacokinetic evaluation of TPIP-B and TPIP-A in Sprague-Dawley rats

### MATERIALS AND METHODS

### A. Species

Male Sprague-Dawley rats that weighed between 300 to 350 g were used for these PK studies. The exact weight of the rats was recorded on the day of the experiment.

### B. Identification and randomization of the test system

1. The animals arrived on site at least 3 days prior to the planned experiment.
2. The animals were identified upon arrival as per CCAC guidelines.
3. All animal care and vivarium maintenance were recorded, with documents kept at the test facility.
4. The animals were randomly assigned before the experiment by the study director, who kept records of each animal's ID number.

### C. Drug administrations and dose selection

170 mg of TPIP-B or TPIP-A were loaded into the Vilnius Aerosol Generator (VAG), which was connected to a 12-port rodent nose-only inhalation system (CH Technologies, Westwood, NJ, USA) at the bottom of the tower. Airflow through the nose-only chamber was set at 7 L/min. The material from the VAG was delivered at output voltage of 1.0 Volt and the aerosol was turned off when all the material had been aerosolized, which took approximately 40 minutes. The actual duration of aerosolization was recorded for each exposure. A glass fiber filter was placed on one of the exposure ports and connected to a vacuum source at 0.5 L/min vacuum flow for a period of 5 minutes (started at 5 min after the start of the aerosolization and ended at 10 min). A Mercer-style cascade impactor was placed on one of the exposure ports and connected to a vacuum source at 0.5 L/min vacuum flow for a period of 5 minutes. Following administration of the test article (i.e., TPIP-B or TPIP-A), animals were euthanized for the collection of various biological samples (bronchoalveolar lavage fluids, lungs, spleen, liver, kidneys, heart, stomach and plasma) depending on the time point (Tables 7 and 8). The tower, nose-only restraining tubes and all connecting tubing were cleaned in between experiments with an aqueous solution of 0.5% sodium dodecyl sulfate (SDS), tap water and distilled water. The powder in the cup of the VAG was removed and all parts of the VAG system was clean with blown air.

### D. Samples analysis

Filters collected from the nose-only inhalation tower and Mercer-style cascade impactor collected powder were analyzed. The concentrations of treprostinil palmitil (TP) and Treprostinil (TRE) in the lungs, liver, heart, kidneys, spleen, stomach, BALC and BALF and plasma were analyzed by LC-MS/MS. Values of TP and TRE reported as below the level of quantitation (BLQ) were each assigned a value of zero.

### E. Study design and experimental procedures

### 1. Study design

Thirty-six (36) rats were exposed to TPIP-A and thirty-six (36) rats exposed to TPIP-B. Rats were acclimated to the nose-cone chamber by placing them in the chamber once a day for 3 consecutive days with increasing duration each time (starting with 5 minutes, increasing to 15 minutes, and ending with 20 minutes). On the day of dosing, a first cohort of nine rats was placed inside the nose-cone restraint chambers which are connected to a 12-port nose-only inhalation chamber. The test article was delivered by VAG with an airflow of 7 L/min and the actual dose duration was recorded. A glass fiber filter was placed on one of the exposure ports and connected to a vacuum source at 0.5 L/min vacuum flow for a period of 5 minutes (started at 5 min after the start of the aerosolization and ended at 10 min). A Mercer-style cascade impactor was placed on one of the exposure ports and connected to a vacuum source at 0.5 L/min vacuum flow for a period of 5 minutes. After sampling, the impactor was disassembled, and the aerosol was collected on each stage with 4 mL (4 times 1 mL) of 75 % IPA. The collection with the Mercer cascade impactor was conducted on cohorts 2 and 4. This experiment has been conducted twice, with each cohort containing nine rats. On the next day, cohorts 3 and 4 were exposed to the test article. At the end of the compound exposure, blood and tissue samples were obtained according to the schedule outlined in Table 7. The IPD necropsy time was recorded. For each time point, the rats undergoing the terminal time point were anesthetized with 2% isoflurane inhaled with pure oxygen. Rats were weighed. Blood samples of approximately 3.0 mL was obtained by heart puncture. The K₂-EDTA tubes were centrifuged at 3,000 rpm, 4 °C for 10 minutes. Approximately 0.5 mL of the plasma was aliquoted into three 1 mL tubes and labeled with the study number, animal identification, dose group and time point. The plasma samples were snap-frozen and stored frozen (-80 °C) before drug concentration analysis. The animal was exsanguinated by cutting the abdominal aorta. For the collection of BAL fluid in cohorts 3 and 4, the trachea was isolated and a 14G InSyte catheter inserted towards the lungs, just above the thoracic inlet making sure to keep it positioned above the carina. A syringe containing 2 mL of sterile PBS was flushed into the lungs. The thorax was massaged gently 4 times by applying inward pressure to the rib cage after which the BAL fluid was withdrawn back into the syringe. The lavage was repeated with another 2 mL of sterile PBS and transferred to the same Eppendorf tube. The BALF liquid was centrifuged, the supernatant was removed and stored at -80 °C. The very last drop of BALF (to remove as much as possible) was discarded. The cell pellet was saved, snap-frozen and stored at -80 °C. Lungs, spleen, kidneys, heart, and a liver lobe were collected and cleaned to remove excess tissue and stomachs were cut open and emptied of solid contents. All organs were weighed, placed in 5.0 mL Eppendorf tubes, snap-frozen and stored at -80 °C for subsequent analysis of lung drug concentration.

| **Table 7.** Timepoint distribution between cohorts. | | | | |
|---|---|---|---|---|
| **Plasma & Lung collection** | **Cohort 1** | **Cohort 2** | **Cohort 3** | **Cohort 4** |
| IPD (0.5 h) | 3 | 3 | 3 | 3 |
| 3 h | 0 | 3 | 0 | 3 |
| 6 h | 3 | 0 | 3 | 0 |
| 12 h | 3 | 0 | 3 | 0 |
| 24 h | 0 | 3 | 0 | 3 |
| Total | 9 | 9 | 9 | 9 |

### F. Delivered drug dose calculations based upon filter data

The total and pulmonary delivered dose were calculated from the equation described by Alexander DJ et al. in Association of Inhalation Toxicologists (AIT) Working Party Recommendation for Standard Delivered Dose Calculation and Expression in Non-Clinical Aerosol Inhalation Toxicology Studies with Pharmaceuticals. Inhal. Tox. 20: p1179-1189, 2008 that are derived from the concentration of TP in the nose-only inhalation tower (filter results), the respiratory minute volume, duration of exposure, deposition fraction and body weight: $Dose \left(\frac{μ g}{kg}\right) = \frac{\left[C \left(\frac{μ g}{L}\right)x RMV \left(\frac{L}{min}\right)x D \left(min.\right) x DF\right]}{BW \left(kg\right)}$ where,
C = Concentration (µg/L) in air inhaled
RMV = Respiratory minute volume (L/minute), where the RMV is calculated from the formula: RMV (L/min) = 0.608 x BW (kg) ^{0.852}.
D = Duration of exposure (minutes)
DF = Deposition Fraction, assumed as being 100% for calculation of Total Delivered Dose and 10% for calculation of the Pulmonary Dose
BW = Body Weight (kg)

### G. Dose of TP as input for PK Solver

TP Absolute Dose (ng) = TP Exposed Dose (µg/kg) x BW (kg) x 1000 ng/µg, where BW = Average body weight of the rats in the experiment. This TP dose was used as input for PK analysis with the PK Solver (Zhang Y, Huo M, Zhou J and Xie S. PKSolver: An add-in program for pharmacokinetic and pharmacodynamic data analysis in Microsoft Excel. Comp. Methods Prog. Biomed. 99:p306-314, 2010).

### H. Lung TPeq concentration calculations

$Lung TPeq \left(ng/g\right) = TP + TRE \left(614.95/390.52\right) ,$ where: Molecular Weight TRE = 390.52 g/mol and Molecular Weight TP = 614.95 g/mol

### I. Methods

1. Male Sprague-Dawley rats weighing between 300 - 350 g at the start of dosing arrived at the facility site at least three days before the day of dosing. Animals were housed by two during the experiment.
2. Rats were acclimated to the nose-cone chamber by placing them in the chamber once a day for 3 consecutive days with increasing duration each time (starting with 5 minutes, increasing to 15 minutes, and ending with 30 minutes at the end of the acclimation period).
3. Nine (9) rats were introduced into the nose-cone only chamber before dosing starts. 170 mg of test article was loaded into the VAG, and delivered until no more powder out of the chamber. VAG setting of 1.0 volt was used with an airflow of 7 L/min. The exact duration of drug exposure was measured.
4. A filter was connected to one of the nose-only inhalation ports and sampling was done starting at 5 minutes after the start of dosing and continued for 5 min. Vacuum airflow for the filter sampling was 0.5 L/min. A Mercer-style cascade impactor was placed on one of the exposure ports and connected to a vacuum source at 0.5 L/min vacuum flow for a period of 5 minutes. The Mercer cascade impactor is a seven-stage aerosol sampler. During operation, aerosol is drawn through a series of successively smaller jet openings and impacted on collection surfaces (impaction plates). After particles pass through each jet, they must make a right angle turn to follow the air stream. Larger particles cannot make this turn and impact on the collection surface. Each lower stage of the impactor is designed to provide successively higher jet velocities so that the average size of particles collected is progressively smaller. A filter follows the final stage to collect very small particles that have successfully bypassed all of the collection plates. Prior to sampling each stage of the impactor was coated with glycerol to facilitate recovery of the particles. After sampling, the impactor was disassembled, and the aerosol will be collected on each stage with 2 mL 75 % IPA and placed into 4 mL vials. In the event that the 75% IPA solutions is not clear, or that there was visible material remaining on the stage, the rinsing process was repeated with an additional 2 mL of 75% IPA; the washing procedure may have been repeated up to three times. The collection with the Mercer cascade impactor was done on the first cohort only.
5. After exposure to the test article, blood and other biological samples were collected at the correct time point according to Table 8. The IPD collection of blood and lungs was 0.5 h after the exposure to test article has finished. The dry powder left in the chamber after the delivery was weighed.
6. The exposure procedure described in steps 3 and 4 were repeated with the second, third, and fourth cohorts of animals. For the cohort 3 and 4, BAL fluid was collected prior the collection of the lung. Each cohort contained 9 animals. Cohorts 1-2 and 3-4 have different exposure dates.
7. For rats undergoing the terminal time point, they were anesthetized with 2 % isoflurane inhaled with pure oxygen and blood samples of approximately 3.0 mL was obtained by heart puncture. The K2-EDTA tube was centrifuged at 3,000 rpm, 4°C for 10 minutes.
8. The plasma was aliquoted into a 1 mL tube (3 tubes for terminal time point) and labeled with the study number, animal identification, dose group and time point. The plasma samples were snap-frozen and stored frozen (at approximately -80°C) for drug concentration analysis.
9. Lungs were removed from the thorax, cleaned to remove excess tissue, weighed, snap-frozen and stored at -80 °C for subsequent analysis of lung drug concentration. All other tissues were treated in a similar manner.
10. For the collection of BAL fluid, the trachea was isolated and a 14G InSyte catheter inserted towards the lungs, just above the thoracic inlet making sure to keep it positioned above the carina. A syringe containing 2 mL of sterile PBS was flushed into the lungs. The thorax was massaged gently 4 times by applying inward pressure to the rib cage after which the BAL fluid was withdrawn back into the syringe. The BAL fluid was placed in a 5 mL Eppendorf Tube and kept at 2-4°C on ice before centrifugation. The lavage was repeated with another 2 mL of sterile PBS and transferred to the same Eppendorf tube. The BALF liquid was centrifuged at 400 g for 10 min at 4°C. The supernatant was removed and stored at -80 °C. The very last drop of BALF (to remove as much as possible) was discarded. The cell pellet was saved, snap-frozen and stored at -80 °C.

| **Table 8.** Summary Table of Dosing Plan and Necropsy Schedule | | | |
|---|---|---|---|
| **Time (h)** | **Cohort** | **# of Rats** | **Tissue Harvest** |
| IPD(0.5 h) | 1 | 3 | Lung and Plasma |
| IPD(0.5 h) | 2 | 3 | Lung and Plasma |
| 3 h | | 3 | Lung and Plasma |
| 6 h | 2 1 | 3 | Lung and Plasma |
| 12 h | 1 | 3 | Lung and Plasma |
| 24 h | 2 | 3 | Lung, Plasma, Liver, Heart, Kidney, Spleen, and Stomach |
| IPD(0.5 h) | 3 | 3 | BAL, Plasma, Lung |
| IPD(0.5 h) | 4 | 3 | BAL, Plasma, Lung |
| 3 h | 4 | 3 | BAL, Plasma, Lung |
| 6 h | 3 | 3 | BAL, Plasma, Lung |
| 12 h | 3 | 3 | BAL, Plasma, Lung |
| 24 h | 4 | 3 | BAL, Plasma, Lung, Liver, Heart, Kidney, Spleen, and Stomach |

### RESULTS

### A. Pharmacokinetics Modeling Definitions

| **Abbreviation** | **Unit** | **Description** |
|---|---|---|
| lambda_z | 1/h | Terminal elimination rate constant |
| T_{1/2} | h | Half-Life |
| Tₘₐₓ | h | Time of maximal concentration |
| Cₘₐₓ | ng/g or ng/mL | Maximal concentration in lung or plasma |
| AUC ₀₋ₜ | ng/g*h or ng/mL*h | Area under the concentration curve between time zero and the last time point |
| AUC _{0-inf_obs} | ng/g*h or ng/mL*h | Area under the concentration curve extrapolated to infinity |

### B. Drug dose calculations

| **Table 9.** Summary table of delivered drug dose calculations based upon filter data | | | | | | | | | |
|---|---|---|---|---|---|---|---|---|---|
| **Dose** | **Cohort** | **Aerosol Conc. (µg/L)** | **RMV (L/min)** | **D (min)** | **BW (kg)** | **DF** | **TPeq Dose-Total (µg/kg)** | **DF** | **TPeq Dose-Pulmonary (µg/kg)** |
| **TPIP-B** | 1 | 6.67 | 0.230 | 40.4 | 0.320 | 1 | 193.98 | 0.1 | 19.40 |
| | 2 | 1.66 | 0.239 | 41.5 | 0.334 | 1 | 49.16 | 0.1 | 4.92 |
| **170 mg @ 1.0 V** | 3 | 1.86 | 0.239 | 42.5 | 0.334 | 1 | 56.54 | 0.1 | 5.65 |
| | 4 | 3.47 | 0.269 | 42.1 | 0.385 | 1 | 102.17 | 0.1 | 10.22 |
| | **Average** | **3.41** | **0.244** | **41.6** | **0.343** | **1** | **100.46** | **0.1** | **10.05** |
| | **SD** | **2.31** | **0.017** | **0.9** | **0.028** | **1** | **66.61** | **0.1** | **6.66** |
| **TPIP-A** | 1 | 1.50 | 0.236 | 41.6 | 0.329 | 1 | 44.74 | 0.1 | 4.47 |
| | 2 | 2.34 | 0.239 | 41.1 | 0.334 | 1 | 68.72 | 0.1 | 6.87 |
| **170 mg @ 1.0 V** | 3 | 3.35 | 0.236 | 41.2 | 0.329 | 1 | 99.00 | 0.1 | 9.90 |
| | 4 | 4.49 | 0.240 | 40.4 | 0.335 | 1 | 129.56 | 0.1 | 12.96 |
| | **Average** | **2.92** | **0.238** | **41.1** | **0.332** | **1** | **85.51** | **0.1** | **8.55** |
| | **SD** | **1.29** | **0.002** | **0.5** | **0.003** | **1** | **36.82** | **0.1** | **3.69** |
| **Abbreviations**. RMV: Respiratory minute volume; D: Duration of exposure; DF: Deposition fraction assumed as being 100% for calculation of total delivered dose and 10% for calculation of the pulmonary dose; BW: Body weight. | | | | | | | | | |
| Dose (µg/kg) = C (µg/L) x RMV (L/min) x D (min) x DF ÷ BW | | | | | | | | | |

### C. Lung concentrations of TP, TRE and TPeq

| **Table 10.** Concentration of TP, TRE and TPeq in the lungs after inhaled TPIP-B or TPIP-A. | | | | | | | |
|---|---|---|---|---|---|---|---|
| | | | **Timepoint (h)** | | | | |
| | | | **0.5** | **3** | **6** | **12** | **24** |
| **TPIP-B** | **Cohort 1-2 (100.46 µg/Kg)** | TP (ng/g) | 2244.83 | 1863.86 | 1049.64 | 479.72 | 124.14 |
| | | ± SEM | 227.79 | 191.54 | 148.57 | 26.35 | 17.06 |
| | | N | 6 | 3 | 3 | 3 | 3 |
| | | TRE (ng/g) | 332.49 | 211.90 | 194.10 | 107.68 | 22.00 |
| | | ± SEM | 16.21 | 31.32 | 38.32 | 1.98 | 2.60 |
| | | N | 6 | 3 | 3 | 3 | 3 |
| | | TPeq (ng/g) | 2768.40 | 2197.54 | 1355.29 | 649.28 | 158.78 |
| | | ± SEM | 249.15 | 240.85 | 200.88 | 28.29 | 18.92 |
| | | N | 6 | 3 | 3 | 3 | 3 |
| | **Cohort 3-4 (100.46 µg/Kg)** | TP (ng/g) | 964.83 | 1066.00 | 657.56 | 278.94 | 55.64 |
| | | ± SEM | 57.29 | 219.14 | 81.36 | 10.12 | 7.83 |
| | | N | 6 | 3 | 3 | 3 | 3 |
| | | TRE (ng/g) | 160.07 | 141.30 | 95.34 | 55.36 | 12.14 |
| | | ± SEM | 12.50 | 12.61 | 17.11 | 5.42 | 1.44 |
| | | N | 6 | 3 | 3 | 3 | 3 |
| | | TPeq (ng/g) | 1216.89 | 1288.50 | 807.69 | 366.12 | 74.76 |
| | | ± SEM | 63.60 | 218.68 | 98.64 | 11.10 | 9.97 |
| | | N | 6 | 3 | 3 | 3 | 3 |
| **TPIP-A** | **Cohort 1-2 (85.51 µg/kg)** | TP (ng/g) | 2027.62 | 1715.34 | 980.10 | 375.76 | 74.38 |
| | | ± SEM | 208.94 | 297.19 | 87.20 | 74.10 | 15.64 |
| | | N | 6 | 3 | 3 | 3 | 3 |
| | | TRE (ng/g) | 149.89 | 98.90 | 71.26 | 24.42 | 5.98 |
| | | ± SEM | 16.71 | 3.53 | 10.50 | 3.36 | 1.05 |
| | | N | 6 | 3 | 3 | 3 | 3 |
| | | TPeq (ng/g) | 2263.65 | 1871.08 | 1092.31 | 414.21 | 83.80 |
| | | ± SEM | 229.75 | 299.75 | 95.96 | 79.39 | 17.27 |
| | | N | 6 | 3 | 3 | 3 | 3 |
| | **Cohort 3-4 (85.51 µg/kg)** | TP (ng/g) | 977.97 | 773.22 | 708.02 | 301.60 | 67.72 |
| | | ± SEM | 50.42 | 23.55 | 126.86 | 15.68 | 11.20 |
| | | N | 6 | 3 | 3 | 3 | 3 |
| | | TRE (ng/g) | 67.30 | 68.58 | 41.68 | 19.22 | 7.56 |
| | | ± SEM | 10.54 | 1.93 | 8.43 | 2.01 | 0.63 |
| | | n | 6 | 3 | 3 | 3 | 3 |
| | | TPeq (ng/g) | 1083.95 | 881.21 | 773.65 | 331.87 | 79.62 |
| | | ± SEM | 54.72 | 26.03 | 138.62 | 14.32 | 11.69 |
| | | n | 6 | 3 | 3 | 3 | 3 |
| See also Figures 1, 2 and 3. | | | | | | | |

| **Table 11.** Pharmacokinetic parameters of lung TP, TRE and TPeq after inhaled TPIP-B or TPIP-A. | | | | | | | | |
|---|---|---|---|---|---|---|---|---|
| **TP** | **Dose** | **lambda_ z** | **T_{1/2}** | **Tₘₐₓ** | **Cₘₐₓ** | **AUC_{0- 24h}** | **AUC_{0 -inf_obs}** | |
| **Table 11.** Pharmacokinetic parameters of lung TP, TRE and TPeq after inhaled TPIP-B or TPIP-A. | | | | | | | | |
| | | **µg/kg** | **1/h** | **h** | **h** | **µg/g** | **µg/g *h** | **µg/g *h** |
| **TPIP -B** | Cohort 1-2 Cohort 3-4 | 100.4 6 | 0.126 0.140 | 5.52 4.97 | 0.5 3 | 2.25 1.07 | 18.28 10.18 | 19.27 10.58 |
| **TPIP -A** | Cohort 1-2 Cohort 3-4 | 85.51 | 0.145 0.116 | 4.79 5.97 | 0.5 0.5 | 2.03 0.98 | 15.10 9.90 | 16.51 10.48 |
| | | | | | | | | |

| **TRE** | | **Dose** | **lambda_ z** | **T_{1/2}** | **Tₘₐₓ** | **Cₘₐₓ** | **AUC_{0- 24h}** | **AUC_{0 -inf_obs}** |
|---|---|---|---|---|---|---|---|---|
| | | **µg/kg** | **1/h** | **h** | **h** | **µg/g** | **µg/g *h** | **µg/g *h** |
| **TPIP -B** | Cohort 1-2 Cohort 3-4 | 100.4 6 | 0.112 0.111 | 6.19 6.22 | 0.5 0.5 | 0.33 0.16 | 3.06 1.63 | 3.25 1.74 |
| **TPIP -A** | Cohort 1-2 Cohort 3-4 | 85.51 | 0.138 0.102 | 5.03 6.78 | 0.5 3 | 0.15 0.07 | 1.07 0.70 | 1.12 0.77 |
| | | | | | | | | |

| **TPeq** | | **Dose** | **lambda_ z** | **T_{1/2}** | **Tₘₐₓ** | **Cₘₐₓ** | **AUC_{0- 24h}** | **AUC_{0 -inf_obs}** |
|---|---|---|---|---|---|---|---|---|
| | | **µg/kg** | **1/h** | **h** | **h** | **µg/g** | **µg/g *h** | **µg/g *h** |
| **TPIP -B** | Cohort 1-2 | 100.4 6 | 0.123 | 5.64 | 0.5 | 2.77 | 23.09 | 24.38 |
| | Cohort 3-4 | | 0.134 | 5.16 | 3 | 1.29 | 12.75 | 13.30 |
| **TPIP -A** | Cohort 1-2 | 85.51 | 0.144 | 4.81 | 0.5 | 2.26 | 17.69 | 18.27 |
| | Cohort 3-4 | | 0.114 | 6.08 | 0.5 | 1.08 | 10.10 | 11.69 |
| **Abbreviations.** Lambda z: terminal elimination rate constant; T_{1/2}: half-life; Tₘₐₓ: time of maximal concentration; Cₘₐₓ: maximal concentration; AUC_{0-24 h}: area under the concentration curve between time zero and 24-hours; AUC_{0-inf_obs}: area under the concentration curve extrapolated to infinity. | | | | | | | | |

### D. Plasma concentrations of TP, TRE and TPeq

| **Table 12.** Concentration of TRE and in plasma after inhaled TPIP-B or TPIP-A. | | | | | | | |
|---|---|---|---|---|---|---|---|
| | | | **Timepoint** | | | | |
| | | | **0.5** | **3** | **6** | **12** | **24** |
| **TPIP-B** | **Cohort 1-2 (100.46 µg/Kg)** | TRE (ng/mL) | 0.67 | 0.38 | 0.64 | 0.07 | 0.00 |
| | | ± SEM | 0.13 | 0.06 | 0.32 | 0.01 | 0.00 |
| | | n | 6 | 3 | 3 | 3 | 3 |
| | **Cohort 3-4 (100.46 µg/Kg)** | TRE (ng/mL) | 0.70 | 0.45 | 0.25 | 0.12 | 0.00 |
| | | ± SEM | 0.10 | 0.05 | 0.01 | 0.02 | 0.00 |
| | | n | 6 | 3 | 3 | 3 | 3 |
| **TPIP-A** | **Cohort 1-2 (85.51 µg/kg)** | TRE (ng/mL) | 0.56 | 0.56 | 0.40 | 0.06 | 0.00 |
| | | ± SEM | 0.08 | 0.25 | 0.20 | 0.01 | 0.00 |
| | | n | 6 | 3 | 3 | 3 | 3 |
| | **Cohort 3-4 (85.51 µg/kg)** | TRE (ng/mL) | 0.73 | 0.39 | 0.32 | 0.11 | 0.00 |
| | | ± SEM | 0.11 | 0.04 | 0.02 | 0.01 | 0.00 |
| | | n | 6 | 3 | 3 | 3 | 3 |
| See also Figure 4. | | | | | | | |

| **Table 13.** Pharmacokinetic parameters of plasma TRE after inhaled TPIP-B or TPIP-A. | | | | | | | | |
|---|---|---|---|---|---|---|---|---|
| **TRE** | | **Dose** | **lambda_z** | **T_{1/2}** | **Tₘₐₓ** | **Cₘₐₓ** | **AUC₀₋₂₄ₕ** | **AUC_{0- inf_obs}** |
| | | **µg/kg** | **1/h** | **h** | **h** | **ng/mL** | **ng/mL *h** | **ng/mL *h** |
| **TPIP-B** | Cohort 1-2 | 100.46 | 0.180 | 3.85 | 0.5 | 0.665 | 5.12 | 5.53 |
| | Cohort 3-4 | | 0.156 | 4.45 | 0.5 | 0.702 | 3.77 | 4.51 |
| **TPIP-A** | Cohort 1-2 | 85.51 | 0.209 | 3.32 | 0.5 | 0.560 | 4.36 | 4.63 |
| | Cohort 3-4 | | 0.156 | 4.43 | 0.5 | 0.725 | 3.95 | 4.65 |
| **Abbreviations.** Lambda z: terminal elimination rate constant; T_{1/2}: half-life; Tₘₐₓ: time of maximal concentration; Cₘₐₓ: maximal concentration; AUC_{0-24 h}: area under the concentration curve between time zero and 24-hours; AUC_{0-inf_obs}: area under the concentration curve extrapolated to infinity. | | | | | | | | |

### E. Bronchoalveolar lavage cells concentrations of TP, TRE and TPeq

| **Table 14.** Concentration of TP, TRE and TPeq in BALC after inhaled TPIP-B or TPIP-A. | | | | | | | |
|---|---|---|---|---|---|---|---|
| | | | **Timepoint (h)** | | | | |
| | | | **0.5** | **3** | **6** | **12** | **24** |
| **TPIP-B** | **Cohort 3-4 (100.46 µg/Kg)** | TP (ng/mL) | 4463.07 | 2961.49 | 2458.65 | 1168.25 | 741.04 |
| | | ± SEM | 653.94 | 656.86 | 334.53 | 162.60 | 72.36 |
| | | n | 6 | 3 | 3 | 3 | 3 |
| | | TRE (ng/mL) | 122.17 | 102.28 | 85.50 | 52.26 | 8.10 |
| | | ± SEM | 21.99 | 1.40 | 5.59 | 0.84 | 0.92 |
| | | n | 6 | 3 | 3 | 3 | 3 |
| | | TPeq (ng/mL) | 4655.44 | 3122.54 | 2593.29 | 1250.55 | 753.79 |
| | | ± SEM | 654.98 | 658.90 | 334.26 | 162.45 | 71.51 |
| | | n | 6 | 3 | 3 | 3 | 3 |
| **TPIP-A** | **Cohort 3-4 (85.51 µg/kg)** | TP (ng/mL) | 5168.96 | 4994.22 | 3753.86 | 1457.84 | 340.49 |
| | | ± SEM | 546.56 | 752.54 | 782.25 | 388.71 | 54.54 |
| | | n | 6 | 3 | 3 | 3 | 3 |
| | | TRE (ng/mL) | 98.19 | 206.01 | 46.62 | 27.06 | 7.42 |
| | | ± SEM | 15.39 | 35.70 | 6.50 | 3.68 | 1.09 |
| | | n | 6 | 3 | 3 | 3 | 3 |
| | | TPeq (ng/mL) | 654.98 | 3122.54 | 2593.29 | 1250.55 | 753.79 |
| | | ± SEM | 6 | 658.90 | 334.26 | 162.45 | 71.51 |
| | | n | 5168.96 | 3 | 3 | 3 | 3 |
| See also Figures 5, 6 and 7. | | | | | | | |

| **Table 15.** Pharmacokinetic parameters of BALC TP, TRE and TPeq after inhaled TPIP-B or TPIP-A | | | | | | | | |
|---|---|---|---|---|---|---|---|---|
| **TP** | | **Dose** | **lambda z** | **T_{1/2}** | **T_{ma x}** | **Cₘₐₓ** | **AUC₀₋₂₄ₕ** | **AUC_{0-inf- _obs}** |
| | | **µg/kg** | **1/h** | **h** | **h** | **µg/m L** | **µg/mL *h** | **µg/mL *h** |
| **TPIP-B** | Cohort 3-4 | 100.4 6 | 0.075 | 9.23 | 0.5 | 4.46 | 40.86 | 50.76 |
| **TPIP-A** | Cohort 3-4 | 85.51 | 0.122 | 5.67 | 0.5 | 5.17 | 53.54 | 56.32 |
| | | | | | | | | |

| **TRE** | | **Dose** | **lambda z** | **T_{1/2}** | **T_{ma x}** | **Cₘₐₓ** | **AUC₀₋₂₄ₕ** | **AUC_{0-inf- _obs}** |
|---|---|---|---|---|---|---|---|---|
| | | **µg/kg** | **1/h** | **h** | **h** | **µg/m L** | **µg/mL *h** | **µg/mL *h** |
| **TPIP-B** | Cohort 3-4 | 100.4 6 | 0.116 | 5.98 | 0.5 | 0.12 | 1.37 | 1.44 |
| **TPIP-A** | Cohort 3-4 | 85.51 | 0.140 | 4.97 | 3 | 0.20 | 1.21 | 1.26 |
| | | | | | | | | |

| **TPeq** | | **Dose** | **lambda z** | **T_{1/2}** | **T_{ma x}** | **Cₘₐₓ** | **AUC₀₋₂₄ₕ** | **AUC_{0- inf_obs}** |
|---|---|---|---|---|---|---|---|---|
| | | **µg/kg** | **1/h** | **h** | **h** | **µg/m L** | **µg/mL *h** | **µg/mL *h** |
| **TPIP-B** | Cohort 3-4 | 100.4 6 | 0.076 | 9.12 | 0.5 | 4.66 | 43.02 | 52.94 |
| **TPIP-A** | Cohort 3-4 | 85.51 | 0.123 | 5.65 | 0.5 | 5.32 | 55.45 | 58.32 |
| **Abbreviations.** Lambda z: terminal elimination rate constant; T_{1/2}: half-life; Tₘₐₓ: time of maximal concentration; Cₘₐₓ: maximal concentration; AUC_{0-24 h}: area under the concentration curve between time zero and 24-hours; AUC_{0-inf_obs}: area under the concentration curve extrapolated to infinity. | | | | | | | | |

### F. Bronchoalveolar lavage fluid concentrations of TP, TRE and TPeq

| **Table 16.** Concentration of TP, TRE and TPeq in BALF after inhaled TPIP-A or TPIP-B | | | | | | | |
|---|---|---|---|---|---|---|---|
| | | | **Timepoint (h)** | | | | |
| | | | **0.5** | **3** | **6** | **12** | **24** |
| **TPIP-B** | **Cohort 3-4 (100.46 µg/Kg)** | TP (ng/mL) | 530.28 | 377.31 | 329.90 | 127.38 | 25.13 |
| | | ± SEM | 52.49 | 89.46 | 15.63 | 10.18 | 4.21 |
| | | n | 6 | 3 | 3 | 3 | 3 |
| | | TRE (ng/mL) | 10.00 | 7.98 | 5.17 | 2.01 | 1.34 |
| | | ± SEM | 2.93 | 2.70 | 0.15 | 0.34 | 0.20 |
| | | n | 6 | 3 | 3 | 3 | 3 |
| | | TPeq (ng/mL) | 546.03 | 389.46 | 338.05 | 130.55 | 27.23 |
| | | ± SEM | 54.78 | 88.27 | 15.85 | 10.02 | 4.51 |
| | | n | 6 | 3 | 3 | 3 | 3 |
| **TPIP-A** | **Cohort 3-4 (85.51 µg/kg)** | TP (ng/mL) | 688.93 | 373.89 | 146.67 | 77.93 | 24.23 |
| | | ± SEM | 46.43 | 13.02 | 11.96 | 15.88 | 3.63 |
| | | n | 6 | 3 | 3 | 3 | 3 |
| | | TRE (ng/mL) | 9.87 | 10.78 | 3.79 | 1.88 | 0.47 |
| | | ± SEM | 1.91 | 3.23 | 0.42 | 0.32 | 0.03 |
| | | n | 6 | 3 | 3 | 3 | 3 |
| | | TPeq (ng/mL) | 704.47 | 390.87 | 152.64 | 80.90 | 24.97 |
| | | ± SEM | 48.10 | 14.34 | 12.61 | 16.31 | 3.68 |
| | | n | 6 | 3 | 3 | 3 | 3 |
| See also Figures 8, 9 and 10. | | | | | | | |

| **Table 17.** Pharmacokinetic parameters of BALF TP, TRE and TPeq after inhaled TPIP-A or TPIP-B | | | | | | | | |
|---|---|---|---|---|---|---|---|---|
| **TP** | **Dose µg/kg** | **lambda_ z 1/h** | **T_{1/2} h** | **T_{ma x} h** | **Cₘₐₓ µg/m L** | **AUC₀₋₂₄ₕ µg/mL *h** | **AUC_{0-inf_obs} µg/mL *h** | |
| **Table 17.** Pharmacokinetic parameters of BALF TP, TRE and TPeq after inhaled TPIP-A or TPIP-B | | | | | | | | |
| **TPIP -B** | Cohort 3-4 | 100.4 6 | 0.131 | 5.29 | 0.5 | 0.53 | 4.61 | 4.81 |
| **TPIP -A** | Cohort 3-4 | 85.51 | 0.136 | 5.11 | 0.5 | 0.69 | 3.57 | 3.75 |
| | | | | | | | | |

| **TRE** | | **TRE µg/kg** | **lambda_ z 1/h** | **T_{1/2} h** | **T_{ma x} h** | **Cₘₐₓ µg/m L** | **AUC₀₋₂₄ₕ µg/mL *h** | **AUC_{0-inf_obs} µg/mL *h** |
|---|---|---|---|---|---|---|---|---|
| **TPIP -B** | Cohort 3-4 | 100.4 6 | 0.089 | 7.80 | 0.5 | 0.01 | 0.09 | 0.10 |
| **TPIP -A** | Cohort 3-4 | 85.51 | 0.138 | 5.02 | 3 | 0.01 | 0.08 | 0.08 |
| | | | | | | | | |

| **TPeq** | | **Dose µg/kg** | **lambda_ z 1/h** | **T_{1/2} h** | **T_{ma x} h** | **Cₘₐₓ µg/m L** | **AUC₀₋₂₄ₕ µg/mL *h** | **AUC_{0-inf_obs} µg/mL *h** |
|---|---|---|---|---|---|---|---|---|
| **TPIP -B** | Cohort 3-4 | 100.4 6 | 0.129 | 5.38 | 0.5 | 0.55 | 4.75 | 4.96 |
| **TPIP -A** | Cohort 3-4 | 85.51 | 0.136 | 5.10 | 0.5 | 0.70 | 3.70 | 3.88 |
| **Abbreviations.** Lambda z: terminal elimination rate constant; T_{1/2}: half-life; Tₘₐₓ: time of maximal concentration; Cₘₐₓ: maximal concentration; AUC_{0-24 h}: area under the concentration curve between time zero and 24-hours; AUC_{0-inf_obs}: area under the concentration curve extrapolated to infinity. | | | | | | | | |

### G. Other tissues concentrations of TP, TRE and TPeq

| **Table 18.** Concentration of TP, TRE and TPeq in other tissues 24h after inhaled TPIP-A or TPIP-B. | | | | | | | |
|---|---|---|---|---|---|---|---|
| | | | **Timepoint (h)** | | | | |
| | | | Heart | Kidney | Spleen | Liver | Stomach |
| **TPIP-B** | **Cohort 2 (100.46 µg/Kg)** | TP (ng/g) ± SEM n | 0 | 0 | 0 | 0 | 0 |
| | | | 0 | 0 | 0 | 0 | 0 |
| | | | 3 | 3 | 3 | 3 | 3 |
| | | TRE (ng/g) ± SEM n | 0 | 0.26 | 0 | 0.75 | 1.09 |
| | | | 0 | 0.04 | 0 | 0.31 | 0.93 |
| | | | 3 | 3 | 3 | 3 | 3 |
| | | TPeq (ng/g) ± SEM n | 0 | 0.41 | 0 | 1.18 | 1.71 |
| | | | 0 | 0.07 | 0 | 0.50 | 1.46 |
| | | | 3 | 3 | 3 | 3 | 3 |
| | **Cohort 4 (100.46 µg/Kg)** | TP (ng/g) ± SEM n | 0 | 0 | 0 | 0 | 0 |
| | | | 0 | 0 | 0 | 0 | 0 |
| | | | 3 | 3 | 3 | 3 | 3 |
| | | TRE (ng/g) ± SEM n | 0 | 0.34 | 0 | 1.28 | 0 |
| | | | 0 | 0.06 | 0 | 0.54 | 0 |
| | | | 3 | 3 | 3 | 3 | 3 |
| | | TPeq (ng/g) ± SEM n | 0 | 0.54 | 0 | 1.28 | 0 |
| | | | 0 | 0.09 | 0 | 0.86 | 0 |
| | | | 3 | 3 | 3 | 3 | 3 |
| **TPIP-A** | **Cohort 2 (85.51 µg/kg)** | TP (ng/g) ± SEM n | 0 | 0 | 0 | 0.04 | 0 |
| | | | 0 | 0 | 0 | 0.04 | 0 |
| | | | 3 | 3 | 3 | 3 | 3 |
| | | TRE (ng/g) ± SEM n | 0 | 0.17 | 0 | 0.75 | 0 |
| | | | 0 | 0.02 | 0 | 0.17 | 0 |
| | | | 3 | 3 | 3 | 3 | 3 |
| | | TPeq (ng/g) ± SEM n | 0 | 0.27 | 0 | 1.22 | 0 |
| | | | 0 | 0.03 | 0 | 0.31 | 0 |
| | | | 3 | 3 | 3 | 3 | 3 |
| | **Cohort 4 (85.51 µg/kg)** | TP (ng/g) ± SEM n | 0 | 0 | 0 | 0.58 | 0 |
| | | | 0 | 0 | 0 | 0.24 | 0 |
| | | | 3 | 3 | 3 | 3 | 3 |
| | | TRE (ng/g) ± SEM n | 0 | 0.29 | 0 | 1.14 | 1.24 |
| | | | 0 | 0.07 | 0 | 0.32 | 1.15 |
| | | | 3 | 3 | 3 | 3 | 3 |
| | | TPeq (ng/g) ± SEM n | 0 | 0.46 | 0 | 2.38 | 21.95 |
| | | | 0 | 0.10 | 0 | 0.61 | 1.82 |
| | | | 3 | 3 | 3 | 3 | 3 |

In this study, the plasma, tissues and BAL (fluid and cells) pharmacokinetics of 2 different formulations, TPIP-A and TPIP-B, were evaluated. Exposure of TPIP-A and TPIP-B was well-tolerated at each dose and did not result in any mortality. The total delivered inhaled doses for TPIP-B and TPIP-A were 100.5 and 85.5 µg/kg body weight, respectively (Table 9). The corresponding lung TPeq concentrations at Cₘₐₓ (0.5 h) of cohorts 1-2 exposed to TPIP-B and TPIP-A averaged 2768 and 2264 ng/g lung tissue, respectively (Table 10). Levels of lung TPeq in cohorts 3-4 exposed to TPIP-B and TPIP-A were lower, 1217 and 1084 ng/g respectively, than their comparative cohorts 1-2, since BAL extraction was performed on cohorts 3-4 (Table10).

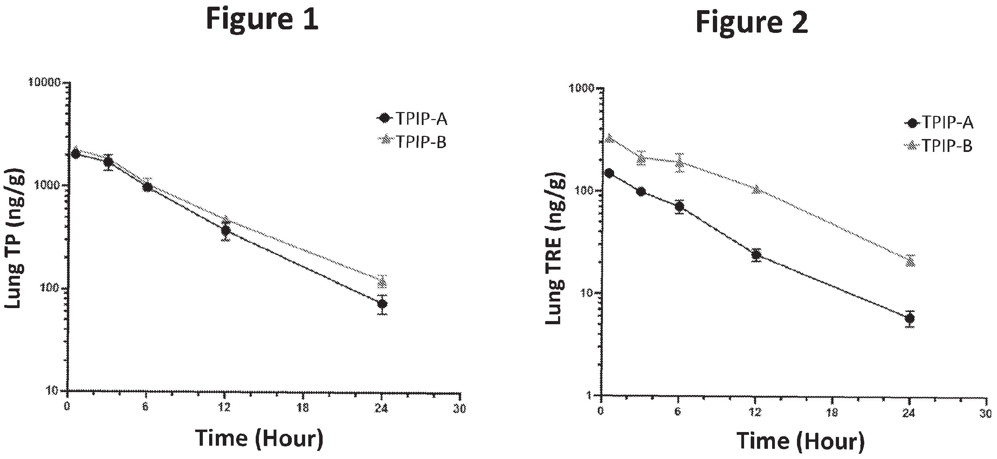

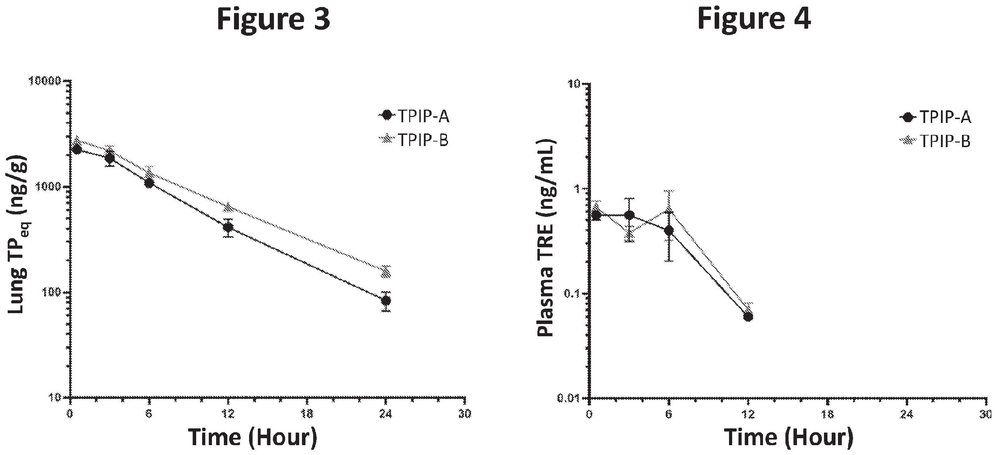

Over a 24-hour period, the highest concentrations of TP, TRE and TPeq in the lungs (Cmax) occurred at 0.5 h after exposure with TPIP-B and TPIP-A for cohorts 1-2 (Table 11). Furthermore, there was a mono-exponential decline in lung drug concentrations over this 24-hour period (Table 9 and Figures 1-3). The profile of TPeq in the lungs for cohorts 3-4, with TPIP-B, is slightly different since the Cmax happened at 3 h post exposure and where TRE Cmax appeared also at 3h post exposure of TPIP-A (Table 11). This difference could be explained by the BAL which were carried out on these rats. In general, TPIP-B and TPIP-A have the same pharmacokinetic profile.

Plasma concentration of TRE after inhaled TPIP-A and TPIP-B, was highest at 0.5 hours after exposure and decreased mono-exponentially over twenty-four hours (Table 12). The concentration of TP in the plasma was very low at 0.5 hours (Table 13).

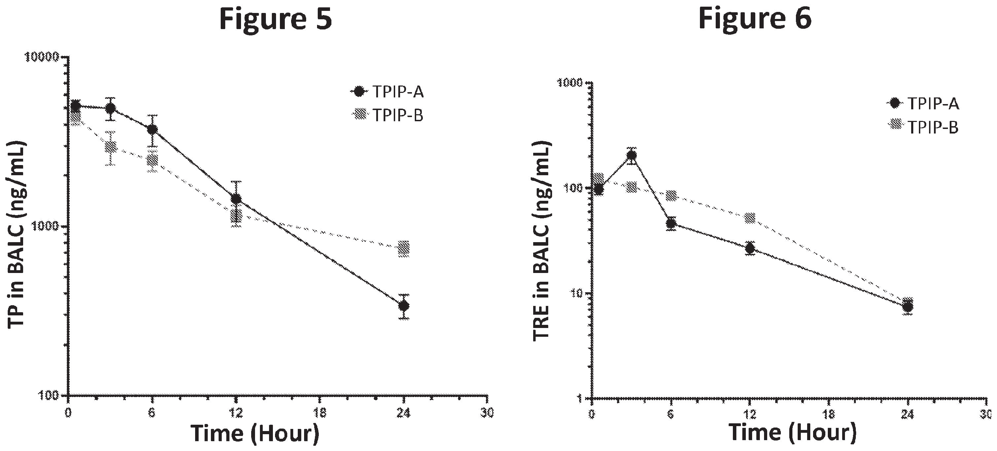

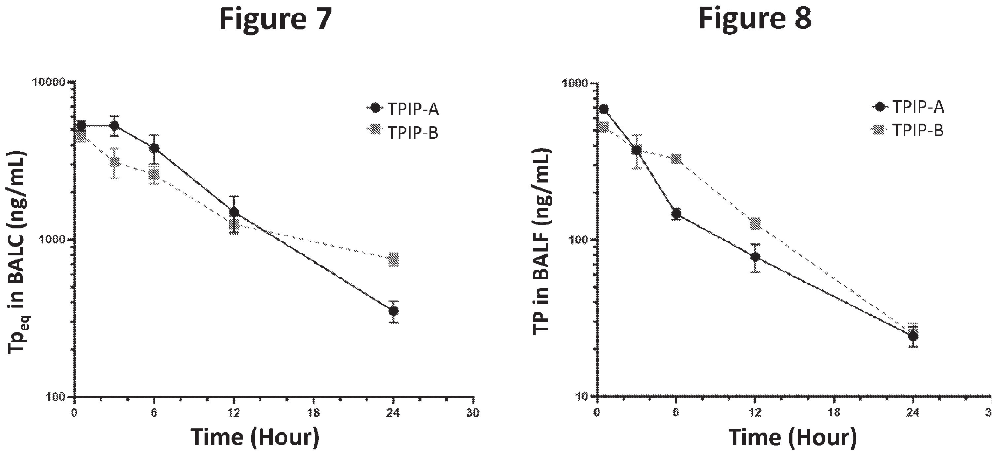

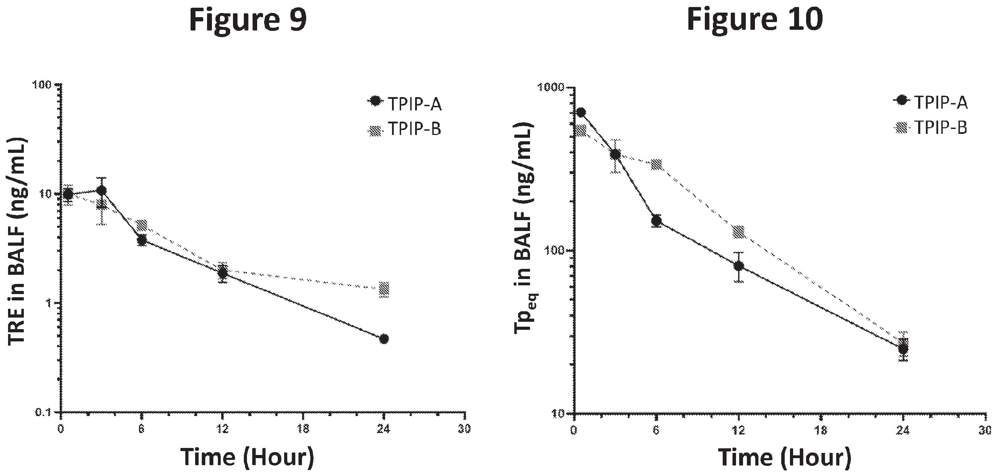

Pharmacokinetic profile of TPIP-A and TPIP-B was also evaluated by bronchoalveolar lavage (BAL). TP, TRE and TPeq concentrations were analyzed in the cells and in the liquid collected from the BAL after removal of cells. Highest concentrations were found in the cells and fluid at 0.5 hours for both formulations except for cohort 3-4 exposed to TPIP, where the TRE Cmax was observed at 3 hours post-dose (Tables 15 and 17 and Figures 5-10).

In summary, the PK profiles of inhaled TPIP-A and TPIP-B demonstrated similar profiles of drug with the highest concentrations of TPeq in the lungs and TRE in the plasma observed by 30 minutes and a mono-exponential decline in the drug levels over twenty-four hours. Some exceptions have been observed for cohorts 1-2 and 3-4 exposed to TPIP-B. Concentration of TRE in the plasma was slightly increased at 6 hours for cohort 1-2 and TPeq in the lungs was slightly increased at 3 hours for cohort 3-4.

### Example 3: Efficacy of different doses of TPIP-B in hypoxia-challenged telemetered rats

### MATERIALS AND METHODS

### A. Species

Male Sprague-Dawley rats that weighed between 300 to 500 g at the time of implantation with a dual-pressure telemetry implant device (TRM-54-PP) were used at the start of dosing in the study. The exact weight of the rats was recorded on the day of the experiment.

### B. Identification and randomization of the test system

1. The animals arrived on site at least 3 days prior to the planned experiment.
2. The animals were identified upon arrival as per CCAC guidelines.
3. All animal care and vivarium maintenance were recorded, with documents kept at the test facility.
4. The animals were randomly assigned before the experiment by the study director, who kept records of each animal's ID number.

### C. Drug administrations and dose selection

TPIP-B was administered using a Vilnius Aerosol Generator (VAG). The VAG was connected to a 12-port rodent nose-only inhalation system (CH Technologies, Westwood, NJ, USA) at the bottom of the tower. Airflow, connected to the bottom and exited from the top of the nose-only inhalation chamber, was introduced into the VAG at a flow rate of 7 L/min. TPIP-B was placed in the VAG chamber in amounts of 25 mg, 50 mg, 90 mg and 170 mg for the aerosolization of the material at VAG voltages of 0.125, 0.25, 0.5 and 1.0 Volt (V), respectively. The aerosol was turned off when all the material had been aerosolized and no drug was visibly seen exiting from the VAG chamber or present in the outlet port of the nose-only inhalation. The time for complete aerosolization of the material was measured. The nose-only inhalation tower, tubing and other materials used in the dry powder process were cleaned by sequentially running an aqueous solution of 0.5% sodium dodecyl sulfate (SDS), tap water, and distilled water. After use, the remaining powder inside the aerosol generator was removed using blown air in a fume hood equipped with a HEPA filter. After thorough cleaning of the tower and VAG, the next experiment was performed.

### D. Samples analysis

Filters collected from the nose-only inhalation tower were used for analysis of C16TR by high performance liquid chromatography (HPLC) and a Charged Aerosol Detector (CAD). Lungs and plasma samples were also analyzed for the concentrations of C16TR and TRE in the lungs and plasma using LC-MS/MS. Values of C16TR and TRE reported as below the level of quantitation (BLQ) were each assigned a value of zero.

### E. Acquisition system

A networked personal computer running Microsoft Windows Office 2016 was used for data acquisition. Data, for systemic arterial blood pressure (SAP) and RVPP were acquired with Powerlab acquisition system (ADinstruments) at a frequency of 500Hz/sec and the software used was Labchart. All records were saved on the server for further analysis. Data was recorded every minute and the results were represented during the normoxia-hypoxia-normoxia periods. To avoid false interpretation of artifactual data generated by animal movements or positioning of the probe against the ventricular wall, 3 to 4 consecutive, typical pulses in both RVPP and SAP were manually selected. The normal right ventricular pressure has a waveform that is almost square and has no spike. Good signals were obtained within the last minute of the 10-minute duration of each of the 3 steps (normoxia-hypoxia-normoxia). Each of these values were re-transcribed in an excel file that lists the data for individual rats at each time point before (baseline data) and at different times after exposure to the drug.

### F. Study design and experimental procedures

### 1. Study design

Seven (7) telemetered implanted male Sprague-Dawley rats were used in total for these studies. Three (3) telemetered rats were used for the efficacy evaluations and seven (7) PK rats dedicated to PK determinations, for each dose. In each experiment, a filter was connected to the 1 remaining port of the nose-only inhalation chamber to sample the inhaled drug content. The hypoxic challenges for the telemetered rats and the blood draws and tissue collections for the PK rats are shown in Tables 19 and 20. In PK rats, blood draw samples were collected from the jugular vein and at the terminal time point, blood was collected by cardiac puncture and the lungs were harvested, cleaned free from surrounding tissues and weighed. Plasma and lungs were stored at -80°C and filters at 4°C. All telemetered rats were habituated to the hypoxia exposure chamber and the rats (both telemetered rats and PK rats) dedicated to inhalation studies were habituated to the nose only inhalation tower once a day for 3 consecutive days with increasing duration each time (beginning with 5 minutes and ending with 20 minutes at the end of the acclimation period).

| **Table 19.** Hypoxic challenge in telemetered rats exposed to TPIP-B | | |
|---|---|---|
| | Day (-1) | Day (0) |
| Dosing | | Inhalation |
| BSL | HxCh (X3) | |
| 1h | | HxCh |
| 6h | | HxCh |
| 12h | | HxCh |
| 24h | | HxCh |
| HxCh: hypoxic challenge with telemetry recording of RVPP and SAP was made on 3 separate occasions before exposure to TPIP-B on day -1 and at times of 1, 6, 12, and 24 h after TPIP-B exposure on day 0. | | |

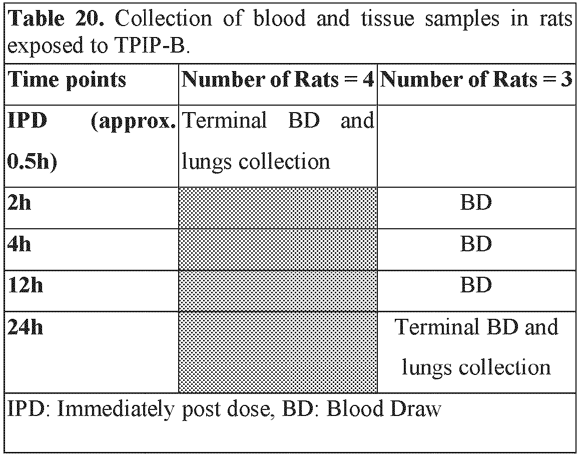

### 2. Normoxia/hypoxia challenges in telemetered rats

Each rat, single housed in an 8x16x8 inch cage, was placed on top of a telemetry receiver (smartpad). A custom-made lid was placed on top of the cage that contained a port to provide air inflow, another exhaust port to evacuate the air and an oxygen probe (Vernier, Beaverton, OR, USA) to continuously measure the oxygen concentration inside the cage. A separate mix box was prefilled with hypoxic (10% O₂/90% N₂) gas mixture that was obtained by combining 100% N₂ and ambient air so that the oxygen levels stabilized at 10% O₂. The hypoxic gas mixture was delivered at a flow rate of approximately 35 L/min to 4 individual chambers that housed the telemetered rats. With the rats exposed to room air breathing, the cardiovascular data was collected for a 10-min period. This was followed by switching a 3-way stopcock and directing the hypoxic gas from the mix box to the cage containing the rats. The hypoxic air then flowed through the inflow hole to replace the normoxic air in the rat cage. Equilibration took approximately 2 min for the rat to be fully exposed to the 10% O₂/90% N₂ gas mixture. Cardiovascular parameters were continually recorded during the 10-min exposure to the hypoxic gas. At the end of this 10-min hypoxic challenge, the inflow hypoxic air from the mix box was turned off and the sealed lid was opened to return the rats back to breathing normoxic gas. Cardiovascular parameters were continuously recorded for the 10-min recovery period on normoxia that followed the exposure to hypoxia. After collection of the data for the normoxia/hypoxia/normoxia exposures, the rats were returned to the vivarium. All rats were given food and water ad libitum after the drug and hypoxia exposures.

### 3. Inhalation of TPIP-B

The 3 telemetered and 7 PK rats were exposed to inhaled TPIP-B at voltages of 0.125, 0.25, 0.5 and 1.0 V, using a nose-cone chamber connected to a 12-port nose-only inhalation chamber (CH Technologies). Airflow was circulated through the nose-only chamber using an inflow of air at flow rate of 7 L/min. A glass fiber filter was connected to one of the exposure ports for the duration of the studies. The airflow sampling was performed with a vacuum source established at 0.5 L/min for 5 minutes, began at 5 minutes after the beginning of the aerosolization and end at 10 min. The circulation of air through the nose-only inhalation tower entered at the bottom and exited through a port at the top of the tower.

### G. Methods

1. Seven (7) male Sprague-Dawley rats already implanted with a dual-pressure telemetry implants were used in total for these studies. For these experiments, 3 telemetered rats were used at 0.125, 0.25, 0.5 V, and 1 V. Additionally, a cohort of 7 rats was used for PK determinations in each study. A filter was connected to the one remaining port in each study.
2. Twenty-four hours prior to exposing the telemetered rats to the test articles, they were exposed to the normoxia /hypoxia /normoxia challenge with cardiopulmonary responses of RVPP and SAP continuously measured during this procedure. This procedure was repeated on 3 separate occasions, performed at times of 1, 6 and 12 hours in a single day, and the average response to these 3 determinations was used to represent the baseline, pre-drug response to hypoxia.
3. After the baseline hypoxia response had been obtained, exposure to the test articles was performed. The rats were exposed to TPIP-B until no powder remain in the VAG cup. The cardiovascular responses to the normoxial hypoxia/ return to normoxia challenge were made as scheduled in Table 21. Blood and lungs samples were withdrawn from the rats dedicated to PK at the times indicated in Table 20.
4. Filters were analyzed.
5. For the blood draws, 0.5 mL of blood was obtained from the jugular vein of conscious rats and deposited in a 0.5 mL K₂-EDTA tube. The K₂-EDTA tube was centrifuged at 900 g at 4°C for 10 minutes.
6. Plasma was aliquoted into a 1 mL tube, snap-frozen and stored at approximately -80°C before analysis.
7. Rats undergoing the terminal time point were anesthetized with 2 % isoflurane inhaled with pure oxygen and blood samples of approximately 3.0 mL obtained by heart puncture. The K₂-EDTA tubes were centrifuged at 900 x g at 4 °C for 10 minutes.
8. The plasma was separated into three 1 mL tubes and stored at approximately - 80°C before drug concentration analysis.
9. Right and left lungs were collected, weighed and stored snap-frozen at -80°C for subsequent analysis of lung drug concentration.

### RESULTS

### A. Inhaled TPIP-B

| **Table 21.** RVPP response to hypoxic challenge in rats exposed to TPIP-B at 0.125 volt (6 µg/kg). | | | | | | |
|---|---|---|---|---|---|---|
| Day | Hr | Normoxia | Hypoxia | Δ Due to hypoxia | Return to Normoxia | N |
| -1 (Baseline) | 1 | 31.5 ± 3.32 | 47.8 ± 6.31 | 15.52 ± 3.00 | 32.6 ± 4.25 | 3 |
| | 6 | 33.1 ± 2.88 | 47.8 ± 6.83 | 14.47 ± 4.02 | 33.6 ± 2.50 | 3 |
| | 12 | 32.0 ± 5.01 | 50.1 ± 8.55 | 18.12 ± 4.18 | 32.1 ± 3.62 | 3 |
| Average (Baseline) | | 32.5 ± 3.74 | 48.9 ± 7.23 | 16.04 ± 3.73 | 32.4 ± 3.46 | 9 |
| 0 (Post drug) | 1 | 32.5 ± 2.15 | 37.5 ± 3.24 | 5.70 ± 1.46 | 32.4 ± 2.10 | 3 |
| | 6 | 34.3 ± 4.66 | 39.1 ± 6.06 | 4.99* ± 1.41 | 32.1 ± 2.61 | 3 |
| | 12 | 33.1 ± 7.05 | 41.3 ± 9.14 | 7.92* ± 2.13 | 32.4 ± 5.09 | 3 |
| | 24 | 33.1 ± 5.07 | 35.3 ± 1.12 | 6.95 ± 0.92 | 32.5 ± 4.46 | 2 |
| Values represent the RVPP and ΔRVPP due to hypoxia (mean ± SEM) from studies in 3 telemetered rats in units of mmHg. N represents the number of rats. | | | | | | |
| ΔRVPP = Hypoxia values - Normoxia values. | | | | | | |
| * P < 0.05 compared to average baseline on Day -1 using a paired t test with repeated measures. | | | | | | |
| See also, Figure 11. | | | | | | |

| **Table 22.** RVPP response to hypoxic challenge in rats exposed to inhaled TPIP-B at 0.25 volt (23 µg/kg). | | | | | | |
|---|---|---|---|---|---|---|
| Day | Hr | Normoxia | Hypoxia | Δ Due to hypoxia | Return to Normoxia | N |
| -1 (Baseline) | 1 | 30.8 ± 3.56 | 40.91 ± 4.67 | 10.53 ± 1.34 | 29.1 ± 1.77 | 3 |
| | 6 | 26.8 ± 1.28 | 34.01 ± 1.88 | 7.62 ± 0.62 | 26.2 ± 2.18 | 3 |
| | 12 | 31.6 ± 0.07 | 43.09 ± 1.63 | 11.83 ± 1.55 | 32.8 ± 0.28 | 2 |
| Average (Baseline) | | 29.4 ± 1.64 | 39.33 ± 2.73 | 9.99 ± 1.17 | 29.4 ± 1.41 | 8 |
| 0 (Post drug) | 1 | 28.2 ± 1.73 | 31.76 ± 2.08 | 3.24* ± 0.36 | 27.1 ± 1.43 | 3 |
| | 6 | 30.3 ± 2.38 | 34.39 ± 3.39 | 3.67 ± 1.02 | 30.6 ± 2.75 | 2 |
| | 12 | 26.1 ± 5.66 | 30.82 ± 8.02 | 3.91 ± 2.36 | 24.7 ± 6.24 | 2 |
| | 24 | 31.6 ± 0.47 | 37.34 ± 4.34 | 5.88 ± 3.87 | 30.8 ± 0.90 | 2 |
| Values represent the RVPP and ΔRVPP due to hypoxia (mean ± SEM) from studies in 3 telemetered rats in units of mmHg. N represents the number of rats. | | | | | | |
| ΔRVPP = Hypoxia values - Normoxia values. | | | | | | |
| * P < 0.05 compared to average baseline on Day -1 using a paired t test with repeated measures. | | | | | | |
| See also, Figure 12. | | | | | | |

| **Table 23.** RVPP response to hypoxic challenge in rats exposed to inhaled TPIP-B at 0.5 volt (57 µg/kg). See also Figure 13 | | | | | | |
|---|---|---|---|---|---|---|
| Day | Hr | Normoxia | Hypoxia | Δ Due to hypoxia | Return to Normoxia | N |
| -1 (Baseline) | 1 | 30.4 ± 0.79 | 39.07 ± 0.38 | 8.44 ± 0.83 | 32.6 ± 1.25 | 3 |
| | | 30.7 ± 1.16 | 39.76 ± 0.54 | 8.99 ± 0.66 | 30.1 ± 0.32 | 3 |
| | 12 | 30.8 ± 0.74 | 39.99 ± 0.07 | 2.01 ± 0.70 | 30.6 ± 0.95 | 3 |
| Average (Baseline) | | 30.0 ± 0.90 | 39.61 ± 0.33 | 8.81 ± 0.73 | 30.1 ± 0.84 | 3 |
| 0 (Post drug) | 1 | 31.3 ± 1.57 | 37.43 ± 1.68 | 5.80 ± 0.11 | 31.3 ± 1.24 | 2 |
| | 5 | 30.2 ± 1.14 | 35.24 ± 0.88 | 4.91 ± 1.56 | 30.7 ± 0.69 | 3 |
| | 12 | 31.9 ± 2.37 | 34.72 ± 2.56 | 3.03* ± 0.24 | 32.3 ± 6.70 | 3 |
| | 24 | 30.0 ± 3.42 | 33.13 ± 2.73 | 2.54 ± 0.68 | 32.9 ± 1.03 | 2 |
| Values represent the RVPP and ΔRVPP due to hypoxia (mean ± SEM) from studies in 3 telemetered rats in units of mmHg. N represents the number of rats. | | | | | | |
| ΔRVPP = Hypoxia values - Normoxia values. | | | | | | |
| * P < 0.05 compared to average baseline on Day -1 using a paired t test with repeated measures. | | | | | | |
| See also, Figure 13. | | | | | | |

| **Table 24.** RVPP response to hypoxic challenge in rats exposed to inhaled TPIP-B at 1 volt (138 µg/kg). | | | | | | |
|---|---|---|---|---|---|---|
| Day | Hr | Normoxia | Hypoxia | Δ Due to hypoxia | Return to Normoxia | N |
| | 1 | 35.4 ± 2.24 | 46.24 ± 4.37 | 11.20 ± 2.14 | 38.1 ± 5.84 | 3 |
| -1 (Baseline) | 6 | 34.5 ± 1.88 | 49.75 ± 5.46 | 15.50 ± 3.76 | 31.5 ± 0.16 | 3 |
| | 12 | 33.6 ± 3.36 | 45.40 ± 5.23 | 12.24± 1.97 | 34.7 ± 4.31 | 3 |
| Average (Baseline) | | 34.5 ± 2.49 | 47.13 ± 5.02 | 12.98± 2.62 | 34.8 ± 3.44 | 9 |
| | 1 | 35.3 ± 4.36 | 42.88 ± 6.83 | 7.74* ± 2.53 | 33.4 ± 1.83 | 3 |
| 0 (Post drug) | 6 | 35.8 ± 6.30 | 39.52 ± 9.79 | 3.93* ± 3.71 | 32.3 ± 4.38 | 3 |
| | 12 | 34.8 ± 2.66 | 38.39 ± 5.22 | 4.31 ± 3.11 | 31.4 ± 2.48 | 3 |
| | 24 | 32.0 ± 1.78 | 38.43 ± 3.92 | 5.84* ± 2.21 | 31.4 ± 3.40 | 3 |
| Values represent the RVPP and ΔRVPP due to hypoxia (mean ± SEM) from studies in 3 telemetered rats in units of mmHg. N represents the number of rats. | | | | | | |
| ΔRVPP = Hypoxia values - Normoxia values. | | | | | | |
| * P < 0.05 compared to average baseline on Day -1 using a paired t test with repeated measures. | | | | | | |
| See also Figure 14. | | | | | | |

| **Table 25.** Concentration of TRE in plasma after inhaled TPIP-B. | | | | | | |
|---|---|---|---|---|---|---|
| | | **Timepoint (h)** | | | | |
| | | **0.5** | **2** | **4** | **12** | **24** |
| TPIP-B @ 1.0 Volt | TRE (ng/mL) | 0.71 | 0.60 | 0.46 | 0.15 | 0.06 |
| | ± SEM | 0.09 | 0.03 | 0.03 | 0.01 | 0.01 |
| | n | 4 | 3 | 3 | 3 | 3 |
| TPIP-B @ 0.5 Volt | TRE (ng/mL) | 0.44 | 0.39 | 0.27 | 0.09 | 0.05 |
| | ± SEM | 0.05 | 0.09 | 0.07 | 0.01 | 0.01 |
| | n | 4 | 3 | 3 | 3 | 3 |
| TPIP-B @ 0.25 Volt | TRE (ng/mL) | 0.20 | 0.15 | 0.10 | 0.04 | 0.02 |
| | ± SEM | 0.04 | 0.01 | 0.01 | 0.01 | 0.00 |
| | n | 4 | 3 | 3 | 3 | 3 |
| TPIP-B @ 0.125 Volt | TRE (ng/mL) | 0.10 | 0.12 | 0.10 | 0.03 | 0.01 |
| | ± SEM | 0.01 | 0.01 | 0.04 | 0.01 | 0.01 |
| | n | 4 | 3 | 3 | 3 | 3 |
| See also Figure 15. | | | | | | |

| **Table 26.** Concentration of C 16TR, TRE, and C16TReq in lungs after inhaled TPIP-B. | | | | | | |
|---|---|---|---|---|---|---|
| Study | Value | Timepoint (h) | | | | |
| | | 0.5 | 2 | 4 | 12 | 24 |
| TPIP-B @ 1.0 Volt | C16TR (ng/g) | 1500.51 | | | | 85.60 |
| | ± SEM | 51.54 | | | | 8.54 |
| | N | 4 | | | | 3 |
| | TRE (ng/g) | 184.95 | | | | 10.46 |
| | ± SEM | 9.05 | | | | 0.68 |
| | N | 4 | | | | 3 |
| | C16TReq (ng/g) | 1791.75 | | | | 102.07 |
| | ± SEM | 63.87 | | | | 9.33 |
| | N | 4 | | | | 3 |
| TPIP-B @ 0.5 Volt | C16TR (ng/g) | 1140.68 | | | | 42.92 |
| | ± SEM | 184.65 | | | | 11.12 |
| | N | 4 | | | | 3 |
| | TRE (ng/g) | 91.65 | | | | 5.42 |
| | ± SEM | 12.44 | | | | 1.27 |
| | N | 4 | | | | 3 |
| | C16TReq (ng/g) | 1285.00 | | | | 51.45 |
| | ± SEM | 202.37 | | | | 12.91 |
| | N | 4 | | | | 3 |
| TPIP-B @ 0.25 Volt | C16TR (ng/g) | 377.90 | | | | 17.88 |
| | ± SEM | 16.96 | | | | 1.34 |
| | N | 4 | | | | 3 |
| | TRE (ng/g) | 37.68 | | | | 1.26 |
| | ± SEM | 2.77 | | | | 0.09 |
| | N | 4 | | | | 3 |
| | C16TReq (ng/g) | 437.23 | | | | 19.86 |
| | ± SEM | 17.87 | | | | 1.29 |
| | N | 4 | | | | 3 |
| TPIP-B @ 0.125 Volt | C16TR (ng/g) | 250.13 | | | | 6.68 |
| | ± SEM | 26.26 | | | | 0.33 |
| | N | 4 | | | | 3 |
| | TRE (ng/g) | 16.75 | | | | 0.21 |
| | ± SEM | 2.62 | | | | 0.10 |
| | N | 4 | | | | 3 |
| | C16TReq (ng/g) | 276.50 | | | | 7.01 |
| | ± SEM | 27.52 | | | | 0.27 |
| | N | 4 | | | | 3 |
| See also Figures 16, 17 and 18. | | | | | | |

| **Table 27.** Pharmacokinetic parameters of plasma TRE after inhaled TPIP-B | | | | | | |
|---|---|---|---|---|---|---|
| | lambda_z | T 1/2 | Tmax | Cmax | AUCO-24h | AUC0-inf_obs |
| | 1/h | h | h | ng/ml | ng/ml*h | ng/ml*h |
| TPIP-B @ 1.0 Volt | 0.109 | 6.337 | 0.500 | 0.705 | 5.873 | 6.391 |
| TPIP-B @ 0.5 Volt | 0.096 | 7.239 | 0.500 | 0.438 | 3.676 | 4.198 |
| TPIP-B @ 0.25 Volt | 0.102 | 6.800 | 0.500 | 0.198 | 1.488 | 1.651 |
| TPIP-B @ 0.125 Volt | 0.098 | 7.089 | 2.000 | 0.115 | 1.127 | 1.274 |

| **Table 28.** Pharmacokinetic parameters of plasma Cl6TReq after inhaled TPIP-B | | | | | | |
|---|---|---|---|---|---|---|
| | lambda_z | T 1/2 | Tmax | Cmax | AUCO-24h | AUC0-inf_obs |
| | 1/h | h | h | ng/ml | ng/ml*h | ng/ml*h |
| TPIP-B @ 1.0 Volt | 0.111 | 6.238 | 0.500 | 1.205 | 9.342 | 10.146 |
| TPIP-B @ 0.5 Volt | 0.099 | 7.010 | 0.500 | 0.799 | 5.898 | 6.695 |
| TPIP-B @ 0.25 Volt | 0.107 | 6.460 | 0.500 | 0.401 | 2.432 | 2.677 |
| TPIP-B @ 0.125 Volt | 0.106 | 6.568 | 0.500 | 0.272 | 1.888 | 2.102 |

In this study, the efficacy of different doses of the DSPE-PEG free TPIP (TPIP-B) was evaluated. Experiments were performed in rats that were prepared with telemetry probes implanted in the right ventricle and descending aorta to measure the increase in RVPP and change in SAP that was induced by exposure to acute hypoxia. Exposure of TPIP-B was well tolerated and did not result in any mortality.

All doses of TPIP-B inhibited the ΔRVPP response to hypoxia over 24 hours. At the highest dose of 138 µg/kg, statistically significant (p < 0.05) inhibition was observed over 24 hours, except at 12 hours, with an effect of 40% to 70% inhibition. A slightly lower dose of TPIP-B of 57 µg/kg had an increasing activity over time and reached a maximum effect (71% inhibition) at 24 hours. The lowest doses of 23 and 6 µg/kg showed similar drug effect with a maximum activity at 1 hour (approximately 65% inhibition), and decreasing to 57% and 40% respectively at 24 hours.

There was a dose-dependent increase in the treprostinil palmitil equivalent (C16TReq) concentration in the lungs and TRE concentration in the plasma with increasing doses of TPIP-B. The concentration of C16TReq in the lungs was high at 0.5 hours and declined by 94-97% over 24 hours with each dose of TPIP-B. The plasma TRE concentration was highest at 0.5 hours with all doses of TPIP-B with a mono-exponential decline over 12 hours and declined by 89-92% over 24 hours.

In summary, efficacy study in hypoxia-challenged telemetered rats demonstrate that at the highest dose of TPIP-B of 138 µg/kg, there was a statistically significant inhibition of the increase in RVPP induced by the hypoxia challenge over 24 hours. Lower doses of TPIP-B were less effective, but had activity over 24 hours, although not significant at all time points.

### Example 4: Assessment of TPIP-B on Cough and Ventilation in Guinea Pigs

In this example, TPIP-B was evaluated for impact on cough, change in ventilation and change in Penh, in conscious male guinea pigs. Penh is a dimensionless index of altered breathing pattern typically seen during bronchoconstriction (See Chong BTY et al.

(1998). Measurement of bronchoconstriction using whole-body plethysmograph: comparison of freely moving versus restrained guinea pigs. J. Pharmacol. Toxicol. Methods 39, 163-168 and Lomask M (2006). Further exploration of the Penh parameter. Exp. and Toxicol. Pathol. 57,13-20).

### A. Methods

1. Experiments were performed in male Hartley guinea pigs (230-430 g). After a 3-day period of acclimation to the experimental surroundings, the guinea pigs were placed in a whole body plethysmograph for the measurement of ventilation (tidal volume, respiratory rate and minute volume), Penh and cough using established techniques. Cough was measured from plethysmograph recordings showing a large inspiration followed by a large expiration and confirmed by manual observations, video recordings and cough sounds. The ventilation, Penh and cough data were measured during a 15 min baseline period before the exposure to the dry powder aerosol.
2. Dosing of the test articles for this study was achieved by aerosolizing a specific amount of dry powder using the Vilnius Aerosol Generator (VAG) (CH Technologies, Westwood, NJ) at a specific voltage output and Microdust range, followed by a 120 min observation, after the aerosolized compounds were administered. Approximately 110 mg of TPIP-B placebo was aerosolized at a setting of 1 volt with 2500 mg/m³ Microdust range until the powder was completely consumed (Table 29). TPIP-B was then dosed under similar conditions using approximately 110 mg or 200 mg. To reduce the exposure time, 200 mg doses were also administered using an output of 0.3 volt with 25 g/m³ Microdust range. To standardize the duration of exposure to the test articles, additional experiments were performed in which an excess of TPIP-B, ranging from approximately 200 mg to 450 mg, was aerosolized for 15 min at an increasing VAG output of 0.15 volt, 0.3 volt and 0.5 volt with the Microdust range of 25 g/m³. Finally, to compare TPIP-A to TPIP-B, approximately 250 mg to 400 mg TPIP-A was delivered for 15 min, at settings of 0.15 volt and 0.5 volt with Microdust range of 25 g/m³ (Table 29).
3. The air for the aerosol delivery for all of the experiments was supplied by an air compressor set at a total inflow of humidified air (30% RH) of 5.5 L/min; 4.5 L/min to disperse the aerosol, combined with 1 L/min of humidified air, to facilitate aerosol delivery to the plethysmograph and minimize problems with static adhesion. Ventilation, Penh and cough were measured before, during and after exposure to the test articles. A vacuum draw of 8 L/min was established at the bottom of the plethysmograph such that the air and aerosols entered the top and exited the bottom of the system. A separate vacuum source of 0.5 L/min was also connected to a glass fiber filter assembly that was attached to a port in the plethysmograph to sample the aerosol concentration in the TPIP-B placebo (containing 70 wt% mannitol and 30 wt% leucine), TPIP-B and TPIP-A aerosols. With the exception of the TPIP-B placebo, the filter samples for TPIP-B and TPIP-A were analyzed for the TP (C16TR) analyte content using HPLC and CAD to determine the TP aerosol concentration. The filter sampling was maintained for the full duration of the study; i.e. 135 min, but the filter exposure time or drug delivery time duration (full duration time drug was delivered until depleted at the beginning of the study adjusted to 15 min drug delivery time later on in additional studies) was used to calculate the TP aerosol concentration in the plethysmograph.
4. The inhaled total TP delivered drug dose at the nose in guinea pigs was calculated using the following equation when deposition factor (DF) is 100%: $\begin{matrix}TP Dose \left(\frac{μ g}{kg}\right) \\ = \frac{\left[respiratory minute volume \left(\frac{L}{min}\right)x TP aerosol concentration \left(\frac{μ g}{L}\right) x Dose time \left(min\right) x DF\right]}{Body Weight \left(kg\right)}\end{matrix}$
5. At the end of the study, the guinea pigs were euthanized and blood (plasma) and lung samples were collected to measure the TP (C16TR) and TRE concentrations using LC-MS/MS in these samples.

### RESULTS

Exposures to TPIP-B placebo, TPIP-B, and TPIP-A were well tolerated and did not result in any mortality. In the first series of experiments in which the test article was aerosolized until all the material had disappeared, aerosolization of 100-115 mg TPIP-B placebo for 32 to 45 min produced no cough in all 4 guinea pigs studied. Aerosolization of 89-105 mg TPIP-B for 23-32 min (average inhaled total delivered dose = 5.7 µg/kg body weight) did not produce cough in the 2 guinea pigs studied and increasing the amount of drug aerosolized to 184-201 mg TPIP-B study (average inhaled total delivered dose = 69.1 µg/kg body weight, exposure time ranging from 62 to 74 min) produced cough in 1 out of the 3 guinea pigs. However, aerosolization of 197 mg TPIP-B (average inhaled total delivered dose = 69.2 µg/kg body weight) for 19 min did not produce cough in the 1 guinea pig studied.

In the second series of experiments in which an excess of test article was aerosolized for a fixed time of 15 min, aerosolization of 102-111 mg TPIP-B (average inhaled total delivered dose = 17.7 µg/kg body weight) produced cough in 1 out of 5 guinea pigs and increasing the amount of drug aerosolized to 115-139 mg TPIP-B study (average inhaled total delivered dose = 43.2 µg/kg body weight) did not produce cough in the 2 guinea pigs studied. However, further increasing the amount of drug aerosolized to 211-457 mg TPIP-B study (average inhaled total delivered dose = 153.2 µg/kg body weight) produced cough in 3 out of 4 guinea pigs (Table 29).

In summary, the results from this study demonstrate that cough was seen at a threshold inhaled dose of 17.7 µg/kg for TPIP-B. For comparison, 90-98 mg of TPIP-A was aerosolized for 15 min (average inhaled total delivered dose = 8.3 µg/kg body weight) and it did not produce cough in the 2 guinea pigs studied and increasing the amount of drug aerosolized to 322 mg TPIP-A study (average inhaled total delivered dose = 185.4 µg/kg body weight) did not produce cough in the 1 guinea pig studied either. However, based on the results of a previous study, cough was observed at a threshold inhaled dose of 12.8 µg/kg for TPIP.

The administration of TPIP-B produced a 1- to 2- fold increase in Penh compared to values produced by exposure to TPIP-B placebo. From previous experiences with bronchoconstrictor agents such as capsaicin or citric acid having values typically observed in the range of 1,000% and higher during challenge, the Penh parameter values suggested that TPIP-B did not likely cause bronchoconstriction and there were no consistent changes in ventilation at the inhaled doses for TPIP-B.

The lung TPeq concentration increased as a function of the inhaled drug dose (Table 29).

| **Table 29.** Summarized Data for Cough, Inhaled Dose, TPeq Concentration in the Lungs, TRE Concentration in the Plasma of Guinea Pigs and Penh Values Exposed to TPIP-B Vehicle, TPIP-B or TPIP-A. | | | | | | | | | |
|---|---|---|---|---|---|---|---|---|---|
| Sample | VAG Setting (volt) (Dry Powder aerosolize d, mg) | Dry powder delivery time (hh:mm:ss ) | # of GP tested | # of GP coug hed | Avg. Coug h (# ± SEM) | Avg. Inhaled dose (µg/kg ± SEM) | Avg. Lung TPeq (ng/g ± SEM) t | Avg. Plasma TRE (ng/mL ± SEM)^{†} | Avg. Penh Values (%) |
| TPIP-B placeb o | 1.0^{a} (100-115 mg) | 00:31:52 - 00:45:00 | 4 | 0 | 0 | - | - | - | 105 |
| TPIP-B | 1.0 ^{a} (89-105 mg) | 00:22:50 - 00:31:58 | 2 | 0 | 0 | 5.7 ± 2.3 | 317.1 ± 2.2 | 0.07 ± 0.03 | 172 |
| | 1.0^{a} (184-201 mg) | 01:02:06 - 01:14:14 | 3 | 1 | 8.3 ± 8.3 | 69.1 ± 20.4 | 473.8 ± 32.4 | 0.1 ± 0.01 | 108 |
| | 0.3 ^{b} (197 mg) | 00:19:06 | 1 | 0 | 0 | 69.2 | 391.4 | 0.08 | 183 |
| | 0.15^{b} 102-111 mg) | 00:15:00 | 5 | 1 | 3.6 ± 3.6 | 17.7 ± 5.0 | 283.8 ± 46.2 | 0.05 ± 0.01 | 105 |
| | 0.3 ^{b} (115-139 mg) | 00:15:00 | 2 | 0 | 0 | 43.2 ± 1.6 | 413.1 ± 183.4 | 0.05 ± 0.02 | 166 |
| | 0.5 ^{b} (211-457 mg) | 00:15:00 | 4 | 3 | 26.8 ± 13.4 | 153.2 ± 35.6 | 1705.1 ± 419.3 | 0.2 ± 0.07 | 100 |
| TPIP-A | 0.15^{b} (90-98 mg) | 00:15:00 | 2 | 0 | 0 | 8.3 ± 3.7 | 225.1 ± 111.8 | BLQ | 77 |
| | 0.5 ^{b} (322 mg) | 00:15:00 | 1 | 0 | 0 | 185.4 | 2121.1 | 0.3 | 2 |
| a: Microdust range: 2500 mg/m³; b: Microdust range: 25000 mg/m³. | | | | | | | | | |
| † After exposure to drug, TPIP-B samples were obtained at approximately 150 min at 1 volt (110 mg)/2500 mg/m³; 195 min at 1 volt (200 mg)/2500 mg/m³; 140 min at 0.3 volt (200 mg)/25 g/m³; 135 min at 0.15, 0.3, and 0.5 volt (15 min exposure)/25 g/m³; and for TPIP-A samples at 135 min at 0.15 and 0.5 volt (15 min exposure)/25 g/m³. | | | | | | | | | |
| BLQ = Below limit of quantitation (LOQ = 0.04 ng/mL) | | | | | | | | | |

This study investigated the effect of TPIP-B on cough and ventilation in guinea pigs which is a species that exhibits cough after exposure to inhaled TRE given by nebulization. The results from this study demonstrate that cough occurred with TPIP-B and was seen at a threshold delivered dose of 17.7 µg TP/kg body weight (equivalent to 11.2 µg TRE/kg body weight), which is about 9-fold higher than the threshold dose of 1.2 µg TRE/kg body weight that causes cough in guinea pigs. The TPIP-B cough threshold is similar to the TPIP-A cough threshold at 12.8 µg TP/kg body weight (equivalent to 8.1 µg TRE/kg body weight).

The TRE dose is derived from the equation:$TRE \left(equivalent\right) dose = TP dose \times 390.52 / 614.94 ,$ (where 614.94 and 390.52 are the molecular weights of TP and TRE, respectively).

After exposure to TPIP-B at the cough threshold inhaled dose, the first bout of coughing occurred at 34 minutes, which was later than the timing of cough with nebulized TRE that occurred within the first 10 min of exposure. The cough response was representative of that observed with exposure to treprostinil and occurred in distinct bouts of coughing (as was seen with the TPIP-A study) rather than as individual coughs.

In summary, cough occurred with TPIP-B at delivered dose 17.7 µg TP/kg body weight (equivalent to 11.2 µg TRE/kg body weight), which was 9-fold higher than the delivered dose of nebulized TRE that causes cough in guinea pigs. There was no significant change in the cough and ventilation responses between TPIP-B and TPIP-A.

### Example 5: Assessment of the safety, tolerability, and PK profile of single and multiple daily dosing of TPIP-B in healthy adults

### Design

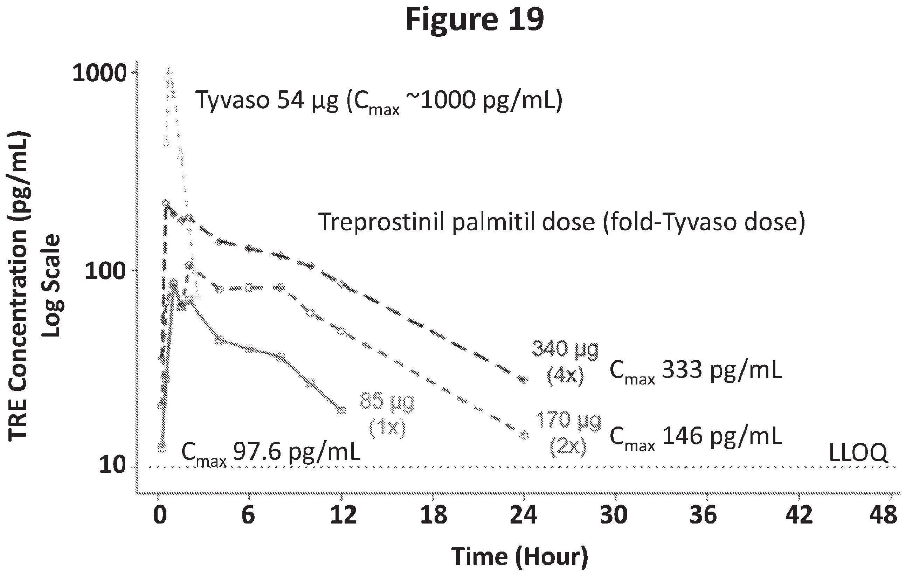

To assess the and PK profile of TPIP-B in healthy adults, TPIP-B was formulated as a dry powder composition and was administered via inhalation in single or multiple dose trials as shown in Figure 19. The following single-doses were tested: 112.5 µg; 225 µg; 450 µg; and 675 µg. The multiple-dose group was structured as follows: 225 µg; and an up-titration in which 112.5 µg was administered on days 1-4, and then on day 5 the dose was increased to 225 µg.

All doses were administered using 112.5-µg, single-actuation capsules. Blood samples for PK assessments in the single-dose groups were collected within 15 minutes prior to dosing and at 0.25, 0.5, 1, 1.5, 2, 4, 6, 8, 10, 12, 24 (day 2), 36 (day 2), 48 (day 3), and 72 (day 4) hours after administration of TPIP-A or placebo. PK assessments in the multiple-dose groups were performed within 30 minutes prior to dosing and at 0.25, 0.5, 1, 1.5, 2, 4, 6, 8, 10, and 12 hours after dosing on day 1, predose only on days 2, 3, 4, 5, and 6, and predose on day 7 and 0.25, 0.5, 1, 1.5, 2, 4, 6, 8, 10, 12, 24 (day 8), 48 (day 9), and 72 (day 10) hours after dosing.

### Results

Treprostinil PK was linear (i.e., CL/F, Vd/F, and t_{1/2} are dose independent), and systemic exposure was linearly related to the dose with low to moderate interindividual variability. No accumulation at steady state was observed. A rapid Cₘₐₓ and long t_{1/2} (7-12 hours) was observed in both single or multiple daily dosing. The PK profile for the single-dose group and multiple dose-group is provided in Table 30A (single-dose group) and 30B (multiple-dose group). Cₘₐₓ, AUC, and t_{1/2} may range from 80-125% of the values provided in Tables 30A and 30B.

| **Table 30A.** TPIP-B single-dose groups (N=26) | | | | | |
|---|---|---|---|---|---|
| **PK parameter, mean (CV%)** | **Day** | **112.5 µg (n=6)** | **225 µg (n=6)** | **450 µg (n=6)** | **675 µg (n=6)** |
| Cₘₐₓ, pg/mL | 1 | 78.4 (72.9) | 287.0 (46.6) | 387.0 (38.6) | 717.0 (52.8) |
| AUC, pg·h/mL^{a} | 1 | 1090.0 (19.8) | 2130.0 (30.0)^{b} | 4040.0 (27.4) | 5480.0 (13.8) |
| t_{1/2}, h | 1 | 11.6 (19.4) | 8.7 (10.2)^{b} | 9.4 (22.6) | 9.8 (10.0) |
| CL/F, L/h | 1 | 106 (18.9) | 112 (24.7)^{b} | 119 (28.5) | 124 (10.6) |
| Vd/F, L | 1 | 1740 (20.0) | 1430 (32.7) | 1590(35.0) | 1760 (16.2) |

| **Table 30B.** TPIP-B multiple-dose groups (N=16) | | | |
|---|---|---|---|
| **PK parameter, mean (CV%)** | **Day** | **225 µg QD (n=6)** | **112.5 µg QD + 225 µg QD (n=6)** |
| Cₘₐₓ, pg/mL | 1 | 293.0 (73.9) | 96.0 (51.9) |
| | 7 | 193.0 (32.9)^{b} | 228.0 (46.4) |
| AUC, pg·h/mL^{c} | 1 | 1560.0 (22.0) | 837.0 (30.6) |
| | 7 | 1680.0 (28.7)^{b} | 1790.0 (39.6) |
| t_{1/2}, h | 1 | 11.7 (19.1) | 9.7 (41.8)^{b} |
| | 7 | 8.8 (14.6)^{b} | 6.8 (22.4) |
| CL/F, L/h | 1 | 114 (28.5) | 96.1 (22.4)^{b} |
| | 7 | - | - |
| Vd/F, L | 1 | 1880 (26.9) | 1280 (28.5)^{b} |
| | 7 | 1810 (29.3)^{b} | 1390 (51.6) |

For Tables 30A and 30B: AUC, area under the plasma concentration vs time curve; CL/F, apparent total drug clearance following oral administration; CV, coefficient of variation; Cₘₐₓ, maximum observed plasma concentration; PK, pharmacokinetic; QD, once daily; t_{1/2}, terminal phase half-life; TPIP, treprostinil palmitil inhalation powder; Vd/F, apparent volume of distribution after nonintravenous drug administration.
^{a} AUC for the single-dose group = AUC from time 0 extrapolated to infinity;
^{b} n=5.
^{c} AUC for the multiple-dose group = AUC from time 0 to 24 hours at steady state.

Single- and multiple-TPIP-B dosing was generally well tolerated in healthy adults. An uptitration strategy in the multiple-dose group improved tolerability. Treatment-emergent adverse events (TEAEs) were dose related and generally mild (80.6%). No serious or severe TEAEs were observed. TEAEs are provided in Table 31A (single-dose group) and 31B (multiple-dose group).

| **Table 31A. TPIP-B single-dose groups (N=26)** | | | | | |
|---|---|---|---|---|---|
| | **112.5 µg (n=6)** | **225 µg (n=6)** | **450 µg (n=6)** | **675 µg (n=6)** | **Placebo (n=2)** |
| **Cough^{b}** | 2 (33.3) | 2 (33.3) | 3 (50.0) | 4 (66.7) | 0 |
| **Dizziness^{b}** | 1 (16.7) | 1 (16.7) | 2 (33.3) | 3 (50.0) | 0 |
| **Headache^{b}** | 0 | 0 | 1 (16.7) | 1 (16.7) | 0 |
| **Nausea^{b}** | 0 | 1 (16.7) | 2 (33.3) | 1 (16.7) | 0 |
| **Chest discomfort^{b}** | 1 (16.7) | 0 | 1 (16.7) | 1 (16.7) | 0 |
| **Throat irritation^{b}** | 2 (33.3) | 2 (33.3) | 1 (16.7) | 0 | 0 |
| **Hypotension^{b}** | 0 | 1 (16.7) | 1 (16.7) | 2 (33.3) | 0 |
| **Fatigue** | 0 | 0 | 0 | 2 (33.3) | 0 |
| **Feeling hot** | 0 | 0 | 0 | 2 (33.3) | 0 |
| **Hyperhidrosis** | 0 | 0 | 0 | 2 (33.3) | 0 |
| TPIP: treprostinil palmitil inhalation powder. | | | | | |
| ^{a}The safety population included all participants who were randomized and received ≥1 dose of assigned treatment. ^{b} AE of special interest. | | | | | |

| **Table 31B. TPIP-B single-dose groups (N=26)** | | | |
|---|---|---|---|
| | **225 µg QD** | **112.5 µg QD + 225 µg QD** | **Placebo** |
| | **(n=6)** | **(n=6)** | **(n=4)** |
| **Cough^{b}** | 6 (100.0) | 1 (16.7) | 2 (50.0) |
| **Dizziness^{b}** | 2 (33.3) | 1 (16.7) | 0 |
| **Headache^{b}** | 4 (66.7) | 2 (33.3) | 0 |
| **Nausea^{b}** | 3 (50.0) | 1 (16.7) | 0 |
| **Chest discomfort^{b}** | 2 (33.3) | 2 (33.3) | 0 |
| **Throat irritation^{b}** | 1 (16.7) | 0 | 0 |
| **Hypotension^{b}** | 0 | 0 | 0 |
| **Fatigue** | 0 | 0 | 0 |
| **Feeling hot** | 0 | 0 | 0 |
| **Hyperhidrosis** | 0 | 0 | 0 |
| QD: once daily; TPIP: treprostinil palmitil inhalation powder. | | | |
| ^{a} The safety population included all participants who were randomized and received ≥1 dose of assigned treatment. ^{b} AE of special interest. | | | |

While the described invention has been described with reference to the specific embodiments thereof it should be understood by those skilled in the art that various changes may be made and equivalents may be substituted without departing from the true spirit and scope of the invention. In addition, many modifications may be made to adopt a particular situation, material, composition of matter, process, process step or steps, to the objective spirit and scope of the described invention. All such modifications are intended to be within the scope of the claims appended hereto.

Patents, patent applications, patent application publications, journal articles and protocols referenced herein are incorporated by reference in their entireties, for all purposes.

### Embodiments

### Various embodiments of the invention are defined in the clauses below:

1. A dry powder composition comprising:
(a) from about 0.5 wt% to about 5 wt% of a compound of Formula (I): 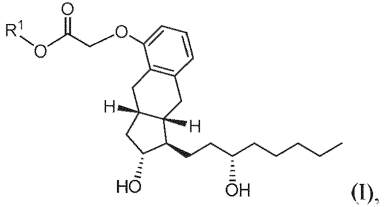 a stereoisomer thereof, or a pharmaceutically acceptable salt thereof, wherein R¹ is tetradecyl, pentadecyl, hexadecyl, heptadecyl, or octadecyl,
(b) from about 10 wt% to about 61 wt% of leucine, and

the balance being (c) a sugar selected from the group consisting of trehalose and mannitol,
wherein the entirety of (a), (b), and (c) is 100 wt%.
2. The dry powder composition of clause 1, wherein R¹ is hexadecyl.
3. The dry powder composition of clause 2. wherein R¹ is linear hexadecyl.
4. The dry powder composition of clause 1,. wherein R¹ is tetradecyl.
5. The dry powder composition of clause 4. wherein R¹ is linear tetradecyl.
6. The dry powder composition of clause 1, wherein R¹ is pentadecyl.
7. The dry powder composition of clause 6, wherein R¹ is linear pentadecyl.
8. The dry powder composition of clause 1, wherein R¹ is hexadecyl.
9. The dry powder composition of clause 8, wherein R¹ is linear hexadecyl.
10. The dry powder composition of clause 1, wherein R¹ is heptadecyl.
11. The dry powder composition of clause 10, wherein R¹ is linear heptadecyl.
12. The dry powder composition of clause 1, wherein R¹ is octadecyl.
13. The dry powder composition of clause 12, wherein R¹ is linear octadecyl.
14. The dry powder composition of any one of clauses 1-13. wherein the compound of Formula (I), or a pharmaceutically acceptable salt thereof, is present at from about 0.5 wt% to about 4.5 wt% of the total weight of the dry powder composition.
15. The dry powder composition of clauses 14, wherein the compound of Formula (I), or a pharmaceutically acceptable salt thereof. is present at from about 1 wt% to about 4.5 wt% of the total weight of the dry powder composition.
16. The dry powder composition of clause 14. wherein the compound of Formula (I), or a pharmaceutically acceptable salt thereof, is present at from about 1 wt% to about 4 wt% of the total weight of the dry powder composition.
17. The dry powder composition of clause 14, wherein the compound of Formula (I), or a pharmaceutically acceptable salt thereof, is present at from about 1 wt% to about 3.5 wt% of the total weight of the dry powder composition.
18. The dry powder composition of clause 14, wherein the compound of Formula (I), or a pharmaceutically acceptable salt thereof, is present at from about 1 wt% to about 3 wt% of the total weight of the dry powder composition.
19. The dry powder composition of clause 14. wherein the compound of Formula (I), or a pharmaceutically acceptable salt thereof, is present at from about 1 wt% to about 2.5 wt% of the total weight of the dry powder composition.
20. The dry powder composition of clause 14, wherein the compound of Formula (I), or a pharmaceutically acceptable salt thereof, is present at from about 1 wt% to about 2 wt% of the total weight of the dry powder composition.
21. The dry powder composition of clause 14, wherein the compound of Formula (I), or a pharmaceutically acceptable salt thereof, is present at from about 1 wt% to about 1.5 wt% of the total weight of the dry powder composition.
22. The dry powder composition of clause 14, wherein the compound of Formula (I), or a pharmaceutically acceptable salt thereof is present at from about 2 wt% to about 4 wt% of the total weight of the dry powder composition.
23. The dry powder composition of clause 14. wherein the compound of Formula (I), or a pharmaceutically acceptable salt thereof, is present at from about 2 wt% to about 3.5 wt% of the total weight of the dry powder composition.
24. The dry powder composition of clause 14, wherein the compound of Formula (I), or a pharmaceutically acceptable salt thereof, is present at from about 2 wt% to about 3 wt% of the total weight of the dry pow der composition.
25. The dry powder composition of clause 14. wherein the compound of Formula (I), or a pharmaceutically acceptable salt thereof, is present at from about 2 wt% to about 2.5 wt% of the total weight of the dry powder composition.
26. The dry powder composition of clause 14, wherein the compound of Formula (I), or a pharmaceutically acceptable salt thereof, is present at from about 3 wt% to about 4.5 wt% of the total weight of the dry powder composition.
27. The dry powder composition of clause 14, wherein the compound of Formula (I), or a pharmaceutically acceptable salt thereof, is present at from about 3 wt% to about 4 wt% of the total weight of the dry powder composition.
28. The dry powder composition of clause 14. wherein the compound of Formula (I), or a pharmaceutically acceptable salt thereof, is present at from about 3 wt% to about 3.5 wt% of the total weight of the dry powder composition.
29. The dry powder composition of clause 14, wherein the compound of Formula (I), or a pharmaceutically acceptable salt thereof, is present at from about 0.5 wt% to about 2 wt% of the total weight of the dry powder composition.
30. The dry powder composition of clause 14, wherein the compound of Formula (I), or a pharmaceutically acceptable salt thereof, is present at from about 0.5 wt% to about 1.5 wt% of the total weight of the dry powder composition.
31. The dry powder composition of clause 14, wherein the compound of Formula (I), or a pharmaceutically acceptable salt thereof, is present at from about 0.5 wt% to about 1 wt% of the total weight of the dry powder composition.
32. The dry powder composition of clause 14, wherein the compound of Formula (I), or a pharmaceutically acceptable salt thereof, is present at from about 3.5 wt% to about 4.5 wt% of the total weight of the dry powder composition.
33. The dry powder composition of clause 14, wherein the compound of Formula (I), or a pharmaceutically acceptable salt thereof, is present at from about 4 wt% to about 4.5 wt% of the total weight of the dry powder composition.
34. The dry powder composition of clause 14, wherein the compound of Formula (I), or a pharmaceutically acceptable salt thereof is present at from about 0 5 wt% of the total weight of the dry powder composition.
35. The dry powder composition of clause 14, wherein the compound of Formula (I), or a pharmaceutically acceptable salt thereof, is present at about 1 wt% of the total weight of the dry powder composition
36. The dry powder composition of clause 14, wherein the compound of Formula (I), or a pharmaceutically acceptable salt thereof, is present at about 1.5 wt% of the total weight of the dry powder composition.
37. The dry powder composition of clause 14, wherein the compound of Formula (I), or a pharmaceutically acceptable salt thereof, is present at about 2 wt% of the total weight of the dry powder composition.
38. The dry powder composition of clause 14, wherein the compound of Formula (I), or a pharmaceutically acceptable salt thereof, is present at about 2.5 wt% of the total weight of the dry powder composition.
39. The dry powder composition of clause 14, wherein the compound of Formula (I), or a pharmaceutically acceptable salt thereof, is present at about 3 wt% of the total weight of the dry powder composition.
40. The dry powder composition of clause 14, wherein the compound of Formula (I), or a pharmaceutically acceptable salt thereof, is present at about 3.5 wt% of the total weight of the dry powder composition.
41. The dry powder composition of clause 14, wherein the compound of Formula (I), or a pharmaceutically acceptable salt thereof, is present at about 4 wt% of the total weight of the dry powder composition.
42. The dry powder composition of any one of clauses 1-41. wherein the compound of Formula (I), or a pharmaceutically acceptable salt thereof, is a compound of Formula (I).
43. The dry powder composition of any one of clauses 1-42. wherein the leucine is present at from about 25 wt% to about 61 wt% of the total weight of the dry powder composition.
44. The dry powder composition of any one of clauses 1-42. wherein the leucine is present at from about 40 wt% to about 61 wt% of the total weight of the dry powder composition.
45. The dry powder composition of any one of clauses 1-42. wherein the leucine is present at from about 50 wt% to about 61 wt% of the total weight of the dry powder composition.
46. The dry powder composition of any one of clauses 1-42, wherein the leucine is present at from about 55 wt% to about 61 wt% of the total weight of the dry powder composition.
47. The dry powder composition of any one of clauses 1-42, wherein the leucine is present at from about 58 wt% to about 61 wt% of the total weight of the dry powder composition.
48. The dry powder composition of any one of clauses 1-42. wherein the leucine is present at from about 40 wt% to about 45 wt% of the total weight of the dry powder composition.
49. The dry powder composition of any one of clauses 1-42. wherein the leucine is present at from about 44 wt% to about 51 wt% of the total weight of the dry powder composition.
50. The dry powder composition of any one of clauses 1-42, wherein the leucine is present at from about 43 wt% to about 48 wt% of the total weight of the dry powder composition.
51. The dry powder composition of any one of clauses 1-42, wherein the leucine is present at from about 25 wt% to about 30 wt% of the total weight of the dry powder composition.
52. The dry powder composition of any one of clauses 1-42, wherein the leucine is present at from about 28 wt% to about 30 wt% of the total weight of the dry powder composition.
53. The dry powder composition of any one of clauses 1-42, wherein the leucine is present at from about 25 wt% to about 33 wt% of the total weight of the dry powder composition.
54. The dry powder composition of any one of clauses 1-42. wherein the leucine is present at from about 27 wt% to about 33 wt% of the total weight of the dry powder composition.
55. The dry powder composition of any one of clauses 1-42, wherein the leucine is present at from about 27 wt% to about 31 wt% of the total weight of the dry powder composition.
56. The dry powder composition of any one of clauses 1-42. wherein the leucine is present at from about 27 wt% to about 30 wt% of the total weight of the dry powder composition.
57. The dry powder composition of any one of clauses 1-42. wherein the leucine is present at from about 28 wt% to about 30 wt% of the total weight of the dry powder composition.
58. The dry powder composition of any one of clauses 1-42. wherein the leucine is present at about 30 wt% of the total weight of the dry powder composition.
59. The dry powder composition of any one of clauses 1-42. wherein the leucine is present at about 45 wt% of the total weight of the dry powder composition.
60. The dry powder composition of any one of clauses 1-42, wherein the leucine is present at about 60 wt% of the total weight of the dry powder composition.
61. The dry powder composition of any one of clauses 1-60, wherein the sugar is trehalose.
62. The dry powder composition of any one of clauses 1-60, wherein the sugar is mannitol.
63. The dry powder composition of clause 1, selected from one of the following compositions:

| **TPIP composition #** | **Treprostinil palmitil (%w)** | **Leucine (%w)** | **Mannitol (%w)** | **Total composition (%w)** |
|---|---|---|---|---|
| **1** | 0.5 | 60.0 | 39.5 | 100 |
| **2** | 2.0 | 61.2 | 36.8 | 100 |
| **3** | 3.0 | 60.7 | 36.3 | 100 |
| **4** | 4.0 | 60.0 | 36.0 | 100 |
| **5** | 0.4 | 45.0 | 54.6 | 100 |
| **6** | 1.5 | 44.4 | 54.1 | 100 |
| **7** | 2.0 | 45.0 | 53.0 | 100 |

| **TPIP composition #** | **Treprostinil palmitil (%w)** | **Leucine (%w)** | **Mannitol (%w)** | **Total composition (%w)** |
|---|---|---|---|---|
| **8** | 3.0 | 44.5 | 52.5 | 100 |
| **9** | 4.0 | 45.0 | 51.0 | 100 |
| **10** | 0.5 | 30.0 | 69.5 | 100 |
| **11** | 1.0 | 29.3 | 69.7 | 100 |
| **12** | 1.5 | 29.6 | 68.9 | 100 |
| **13** | 1.5 | 29.3 | 69.2 | 100 |
| **14** | 2.0 | 28.8 | 69.2 | 100 |
| **15** | 3.0 | 28.6 | 68.4 | 100 |
| **16** | 4.0 | 30.0 | 66.0 | 100 |

65. The dry powder composition of clause 1, having a leucine-to-mannitol weight ratio of about 0.40-to-1 (leucine-to-mannitol) to about 0.50-to-1 (leucine-to-mannitol).
66. The dry powder composition of clause 65. having a leucine-to-mannitol weight ratio of about 0.40-to-1 (leucine-to-mannitol) to about 0.45-to-1 (leucine-to-mannitol)
67. The dry powder composition of clause 1, having a leucine-to-mannitol weight ratio about 0.75-to-1 (leucine-to-mannitol) to about 0.90-to-1 (leucine-to-mannitol), of
68. The dry powder composition of clause 67. having a leucine-to-mannitol weight ratio of about 0.80-to-1 (leucine-to-mannitol) to about 0.90-to-1 (leucine-to-mannitol).
69. The dry powder composition of clause 1, having a leucine-to-mannitol weight ratio of about 1.5-to-1 (leucine-to-mannitol) to about 1.7-to-1 (leucine-to-mannitol).
70. The dry powder composition of clause 69, having a leucine-to-mannitol weight ratio of about 1.65-to-1 (leucine-to-mannitol) to about 1.7-to-1 (leucine-to-mannitol).
71. The dry powder composition of any one of clauses 65-70, comprising the compound of Formula (I) at from about 1 wt% to about 4 wt% of the total weight of the dry powder composition.
72. The dry powder composition of any one of clauses 65-70. comprising the compound of Formula (I) at from about 1 wt% to about 1.5 wt% of the total weight of the dry powder composition.
73. The dn powder composition of any one of clauses 65-70. comprising the compound Formula (I) at from about 2 wt% to about 4 wt% of the total weight of the dry powder composition.
74. The dry powder composition of any one of clauses 65-70. comprising the compound of Formula (I) at from about 3 wt% to about 4 wt% of the total weight of the dry powder composition.
75. The dry powder composition of any one of clauses 65-74. wherein R¹ is linear hexadecyl.
76. The dry powder composition of claim 1, comprising (a) about 1 wt% of the compound of Formula (I), wherein R¹ is linear hexadecyl, (b) about 29 wt% to about 30 wt% leucine, and the balance being (c) mannitol.
77. The dry powder composition of clause 1, comprising (a) about 1.5 wt% of the compound of Formula (I), wherein R¹ is linear hexadecyl, (b) about 29 wt% to about 30 wt% leucine, and the balance being (c) mannitol.
78. The dry powder composition of clause 1, comprising (a) about 2 wt% of the compound of Formula (I), wherein R¹ is linear hexadecyl. (b) about 29 wt% to about 30 wt% leucine, and the balance being (c) mannitol.
79. The dry powder composition of clause 1, comprising (a) about 3 wt% of the compound of Formula (I), wherein R¹ is linear hexadecyl. (b) about 29 wt% to about 30 wt% leucine, and the balance being (c) mannitol.
80. The dry powder composition of clause 1, comprising (a) about 4 wt% of the compound of Formula (I), wherein R¹ is linear hexadecyl, (b) about 29 wt% to about 30 wt% leucine, and the balance being (c) mannitol.
81. The dry powder composition of clause 1, comprising (a) about 1 wt% of the compound of Formula (I), wherein R¹ is linear hexadecyl, (b) about 60 wt% to about 61 wt% leucine, and the balance being (c) mannitol.
82. The dry powder composition of clause 1. comprising (a) about 2 wt% of the compound of Formula (I), wherein R¹ is linear hexadecyl. (b) about 60 wt% to about 61 wt% leucine, and the balance being (c) mannitol.
83. The dry powder composition of clause 1, comprising (a) about 3 wt% of the compound of Formula (I), wherein R¹ is linear hexadecyl. (b) about 60 wt% to about 61 wt% leucine, and the balance being (c) mannitol.
84. The dry powder composition of clause 1. comprising (a) about 4 wt% of the compound of Formula (I), wherein R¹ is linear hexadecyl. (b) about 60 wt% to about 61 wt% leucine, and the balance being (c) mannitol.
85. The dry powder composition of clause 1, comprising (a) about 1 wt% of the compound of Formula (I), wherein R¹ is linear hexadecyl. (b) about 43 wt% to about 45 wt% leucine, and the balance being (c) mannitol.
86. The dry powder composition of clause 1, comprising (a) about 1 wt% of the compound of Formula (I), wherein R¹ is linear hexadecyl. (b) about 43 wt% to about 45 wt% leucine, and the balance being (c) mannitol.
87. The dry powder composition of clause 1, comprising (a) about 1.5 wt% of the compound of Formula (I), wherein R¹ is linear hexadecyl, (b) about 43 wt% to about 45 wt% leucine, and the balance being (c) mannitol.
88. The dry powder composition of clause 1, comprising (a) about 2 wt% of the compound of Formula (I), wherein R¹ is linear hexadecyl, (b) about 43 wt% to about 45 wt% leucine, and the balance being (c) mannitol.
89. The dry powder composition of clause 1, comprising (a) about 3 wt% of the compound
of Formula (I), wherein R¹ is linear hexadecyl, (b) about 43 wt% to about 45 wt% leucine, and the balance being (c) mannitol.
90. The dry powder composition of clause 1, comprising (a) about 4 wt% of the compound of Formula (I), wherein R¹ is linear hexadecyl, (b) about 43 wt% to about 45 wt% leucine, and the balance being (c) mannitol.
91. The dry powder composition of any one of clauses 1-90. wherein the leucine is L-leucine.
92. The dry powder composition of any one of clauses 1-91, comprising from about 80 to about 675 µg of the compound of Formula (I). µg
93. The dry powder composition of clause 92, comprising from about 80 µg to about 640 µg of the compound of Formula (I).
94. The dry powder composition of clause 92. comprising from about 112.5 µg to about 675 µg of the compound of Formula (I).
95. The dry powder composition of clause 92. comprising about 80 µg of the compound of Formula (I),
96. The dry powder composition of clause 92. comprising about 160 µg of the compound of Formula (I),
97. The dry powder composition of clause 92. comprising about 240 µg of the compound of Formula (I).
98. The dry powder composition of clause 92. comprising about 320 µg of the compound of Formula (I).
99. The dry powder composition of clause 92. comprising about 480 µg of the compound of Formula (I)
100. The dry powder composition of clause 92, comprising about 640 µg of the compound of Formula (I).
101. The dry powder composition of any one of clauses 1-100, wherein the dry powder composition is in the form of an aerosol comprising aerosolized particles with a mass median aerodynamic diameter (MMAD) of from about 1 µm to about 4 µm, as measured by the Next Generation Impactor (NGI).
102. The dry powder composition of clause 101, wherein the MMAD is from about 1.5 µm to about 3.5 µm, as measured by the NGI.
103. The dry powder composition of clause 101, wherein the MMAD is from about 2 µm to about 3 µm, as measured by NGI.
104. The dry powder composition of any one of clauses 1-103. wherein the dry powder composition is in the form of an aerosol comprising aerosolized particles having a fine particle fraction (FPF) of from about 30% to about 60%, as measured by the NGI.
105. The dry powder composition of any one of clauses 1491, wherein the composition comprises from about 80 µg to about 675 µg of a compound of Formula (I), wherein R¹ is linear hexadecyl, and upon once daily inhalation administration via a dry powder inhaler, provides one of the following characteristics:
(a) a treprostinil maximum plasma concentration (Cₘₐₓ) ranging from about 80% to about 125% of the range of from about 17 pg/mL to about 1150 pg/mL; or
(b) a treprostinil area under the plasma concentration curve (AUC) from about 80% to about 125% of the range of about 475 pg*h/mL to about 8000 pg*h/mL.
106. The dry powder composition of clause 105. comprising about 80 µg of the compound of Formula (I), wherein R¹ is linear hexadecyl, and wherein upon once daily inhalation administration via a dry powder inhaler, provides a treprostinil Cₘₐₓ ranging from about 14 pg/mL to about 155 pg/mL.
107. The dry powder composition of clause 105. comprising about 80 µg of the compound of Formula (I), wherein R¹ is linear hexadecyl, and wherein upon once daily inhalation administration via a dry powder inhaler, provides a treprostinil Cₘₐₓ from about 80%-125% of a range from about 17 pg/mL to about 125 pg/mL.
108. The dry powder composition of clause 105, comprising about 80 µg of the compound of Formula (I), wherein R¹ is linear hexadecyl, and wherein upon once daily inhalation administration via a dry powder inhaler, provides a treprostinil Cₘₐₓ from about 80%-125% of a range from about 35 pg/mL to about 105 pg/mL.
109. The dry powder composition of clause 105, wherein the composition comprises about 112.5 µg of the compound of Formula (I), wherein R¹ is linear hexadecyl, and wherein upon once daily inhalation administration via a dry powder inhaler, provides a treprostinil Cₘₐₓ (CV%) ranging from about 80% to about 125% of about 78.4 (72.9) pg/mL.
110. The dry powder composition of clause 105, comprising about 160 µg of the compound of Formula (I), wherein R¹ is linear hexadecyl, and wherein upon once daily inhalation administration via a dry powder inhaler, provides a treprostinil Cₘₐₓ ranging from about 30 pg/mL to about 335 pg/mL.
111. The dry powder composition of clause 105, comprising about 160 µg of the compound of Formula (I), wherein R¹ is linear hexadecyl, and wherein upon once daily inhalation administration via a dry powder inhaler, provides a treprostinil Cₘₐₓ from about 80%-125% of a range from about 35 pg/mL to about 270 pg/mL.
112. The dry powder composition of clause 105. comprising about 160 µg of the compound of Formula (I), wherein R¹ is linear hexadecyl, and wherein upon once daily inhalation administration via a dry powder inhaler, provides a treprostinil Cₘₐₓ from 80%-125% of a range from about 76 pg/mL to about 230 pg/mL.
113. The dry powder composition of clause 105. comprising about 225 µg of the compound of Formula (I), wherein R¹ is linear hexadecyl, and wherein upon once daily inhalation administration via a dry powder inhaler, provides a treprostinil Cₘₐₓ (CV%) ranging from about 80% to about 125% of about 287 (46.6) pg/mL.
114. The dry powder composition of clause 105. comprising about 225 µg of the compound of Formula (I), wherein R¹ is linear hexadecyl, and wherein upon once daily inhalation administration via a dry powder inhaler, provides a treprostinil steady state Cₘₐₓ (CV%) ranging from about 80% to about 125% of about 193 (329) pg/mL.
115. The dry powder composition of clause 105. comprising about 225 µg of the compound
of Formula (I), wherein R¹ is linear hexadecyl, and wherein upon once daily inhalation administration via a dry powder inhaler, provides a treprostinil steady state Cₘₐₓ (CV%) ranging from about 80% to about 125% of about 228 (46.4) pg/mL.
116. The dry powder composition of clause 105, comprising about 240 µg of the compound of Formula (I), wherein R¹ is linear hexadecyl, and wherein upon once daily inhalation
administration via a dry powder inhaler, provides a treprostinil Cₘₐₓ ranging from about 45 pg/mL to about 520 pg/mL.
117. The dry powder composition of clause 105, comprising about 240 µg of the compound of Formula (I), wherein R¹ is linear hexadecyl, and wherein upon once daily inhalation administration via a dry powder inhaler, provides a treprostinil Cₘₐₓ from 80%-125% of a range from about 55 pg/mL to about 415 pg/mL.
118. The dry powder composition of clause 105. comprising about 240 µg of the compound of Formula (I), wherein R¹ is linear hexadecyl, and wherein upon once daily inhalation administration via a dry powder inhaler, provides a treprostinil Cₘₐₓ from 80%-125% of a range from about 115 pg/mL to about 355 pg/mL.
119. The dry powder composition of clause 105. comprising about 320 µg of the compound of Formula (I), wherein R¹ is linear hexadecyl, and wherein upon once daily inhalation administration via a dry powder inhaler, provides a treprostinil Cₘₐₓ ranging from about 60 pg/mL to about 700 pg/mL.
120. The dry pow der composition of clause 105. comprising about 320 µg of the compound of Formula (I), wherein R¹ is linear hexadecyl, and wherein upon once daily inhalation administration via a dry powder inhaler, provides a treprostinil Cₘₐₓ from 80%-125% of a range from about 80 pg/mL to about 560 pg/mL.
121. The dry powder composition of clause 105. comprising about 320 µg of the compound of Formula (I), wherein R¹ is linear hexadecyl, and wherein upon once daily inhalation administration via a dry powder inhaler, provides a treprostinil Cₘₐₓ from 80%-125% of a range from about 160 pg/mL to about 480 pg/mL.
122. The dry powder composition of clause 105. comprising about 400 µg of the compound of Formula (I), wherein R¹ is linear hexadecyl, and wherein upon once daily inhalation administration via a dry powder inhaler, provides a treprostinil Cₘₐₓ ranging from about 80 pg/mL to about 885 pg/mL.
123. The dry powder composition of clause 105, comprising about 400 µg of the compound of Formula (I), wherein R¹ is linear hexadecyl, and wherein upon once daily inhalation administration via a dry powder inhaler, provides a treprostinil Cₘₐₓ from 80%-125% of a range from about 100 pg/mL to about 705 pg/mL.
124. The dry powder composition of clause 105, comprising about 400 µg of the compound of Formula (I), wherein R¹ is linear hexadecyl, and wherein upon once daily inhalation administration via a dry powder inhaler, provides a treprostinil Cₘₐₓ from 80%-125% of a range from about 200 pg/mL to about 605 pg/mL.
125. The dry powder composition of clause 105. comprising about 450 µg of the compound of Formula (I), wherein R¹ is linear hexadecyl, and wherein upon once daily inhalation administration via a dry pow der inhaler, provides a treprostinil Cₘₐₓ (CV%) ranging from about 80% to about 125% of about 387 (38.6) pg/mL.
126. The dry powder composition of clause 105. comprising about 480 µg of the compound of Formula (I), wherein R¹ is linear hexadecyl, and wherein upon once daily inhalation administration via a dry powder inhaler, provides a treprostinil Cₘₐₓ ranging from about 95 pg/mL to about 1065 pg/mL.
127. The dry powder composition of clause 105 comprising about 480 µg of the compound of Formula (I), wherein R¹ is linear hexadecyl, and wherein upon once daily inhalation administration via a dry powder inhaler, provides a treprostinil Cₘₐₓ from 80%-125% of a range from about 120 pg/mL to about 855 pg/mL.
128. The dry powder composition of clause 105 comprising about 480 µg of the compound of Formula (I), wherein R¹ is linear hexadecyl, and wherein upon once daily inhalation administration via a dry powder inhaler, provides a treprostinil Cₘₐₓ from 80%-125% of a range from about 240 pg/mL to about 730 pg/mL.
129. The dry powder composition of clause 105. comprising about 640 µg of the compound of Formula (I), wherein R¹ is linear hexadecyl, and wherein upon once daily inhalation administration via a dry powder inhaler, provides a treprostinil Cₘₐₓ ranging from about 130 pg/mL to about 1430 pg/mL.
130. The dry powder composition of clause 105, comprising about 640 µg of the compound of Formula (I), wherein R¹ is linear hexadecyl, and wherein upon once daily inhalation administration via a dry powder inhaler, provides a treprostinil Cₘₐₓ from 80%-125% of a range about 160 pg/mL to about 1140 pg/mL.
131. The dry powder composition of clause 105. comprising about 640 µg of the compound of Formula (I), wherein R¹ is linear hexadecyl, and wherein upon once daily inhalation administration via a dry powder inhaler, provides a treprostinil Cₘₐₓ from 80%-125% of a range from about 325 pg/mL to about 980 pg/mL.
132. The dry pow der composition of clause 105. comprising about 675 µg of the compound of Formula (I), wherein R¹ is linear hexadecyl, and wherein upon once daily inhalation administration via a dry powder inhaler, provides a treprostinil Cₘₐₓ (CV%) ranging from about 80% to about 125% of about 717 (52 8) pg/mL.
133. The dry powder composition of clause 105. comprising about 80 µg of the compound of Formula (I), wherein R¹ is linear hexadecyl, and wherein upon once daily inhalation administration via a dry powder inhaler, provides a treprostinil AUC_{0-inf} ranging from about 375 pg*h/mL to about 1800 pg*h/mL.
134. The dry powder composition of clause 105 comprising about 80 µg of the compound of Formula (I), wherein R¹ is linear hexadecyl, and wherein upon once daily inhalation administration via a dry powder inhaler, provides a treprostinil AUC_{0-inf} from 80%- 125% of a range from about 475 pg*h/mL to about 1430 pg*h/mL.
135. The dry powder composition of clause 105. comprising about 80 µg of the compound of Formula (I), wherein R¹ is linear hexadecyl, and wherein upon once daily inhalation administration via a dry powder inhaler, provides a treprostinil AUC_{0-inf} from 80%-125% of a range from about 660 pg*h/mL to about 1240 pg*h/mL.
136. The dry powder composition of clause 105. comprising about 112.5 µg of the compound of Formula (I), wherein R¹ is linear hexadecyl, and wherein upon once daily inhalation administration via a dry powder inhaler, provides a treprostinil AUC_{0-inf} (CV%) ranging from about 80% to about 125% of about 1090 (19.8) pg*h/mL.
137. The dry powder composition of clause 105, comprising about 160 µg of the compound of Formula (I), wherein R¹ is linear hexadecyl, and wherein upon once daily inhalation administration via a dry powder inhaler, provides a treprostinil AUC_{0-inf} ranging from about 630 pg*h/mL to about 3000 pg*h/mL.
138. The dry powder composition of clause 105, comprising about 160 µg of the compound of Formula (I), wherein R¹ is linear hexadecyl, and wherein upon once daily inhalation administration via a dry pow der inhaler, provides a treprostinil AUC_{0-inf} from 80%-125% of a range from about 785 pg*h/mL to about 2370 pg*h/mL.
139. The dry powder composition of clause 105. comprising about 160 µg of the compound of Formula (I), wherein R¹ is linear hexadecyl, and wherein upon once daily inhalation administration via a dry powder inhaler, provides a treprostinil AUC_{0-inf} from 80%-125% of a range from about 1100 pg*h/mL to about 2050 pg*h/mL.
140. The dry powder composition of clause 105. comprising about 225 µg of the compound of Formula (I), wherein R¹ is linear hexadecyl, and wherein upon once daily inhalation administration via a dry powder inhaler, provides a treprostinil AUC_{0-inf} (CV%) ranging from about 80% to about 125% of about 2130 (30 0) pg*h/mL.
141. The dry powder composition of clause 105. comprising about 225 µg of the compound of Formula (I), wherein R¹ is linear hexadecyl, and wherein upon once daily inhalation administration via a dry powder inhaler, provides a treprostinil steady state AUC₀₋₂₄ (CV%) ranging from about 80% to about 125% of about 1680 (28 7) pg*h/mL.
142. The dry powder composition of clause 105. comprising about 225 µg of the compound of Formula (I), wherein R¹ is linear hexadecyl, and wherein upon once daily inhalation administration via a dry powder inhaler, provides a treprostinil steady state AUC₀₋₂₄ (CV%) ranging from about 80% to about 125% of about 1790 (39.6) pg*h/mL.
143. The dry powder composition of clause 105. comprising about 240 µg of the compound of Formula (I), wherein R¹ is linear hexadecyl, and wherein upon once daily inhalation administration via a dry powder inhaler, provides a treprostinil AUC_{0-inf} ranging from about 880 pg*h/mL to about 4130 pg*h/mL.
144. The dry powder composition of clause 105, comprising about 240 µg of the compound of Formula (I), wherein R¹ is linear hexadecyl, and wherein upon once daily inhalation
administration via a dry powder inhaler, provides a treprostinil AUC_{0-inf} from 80%-125% of a range of about of 1100 pg*h/mL to about 3305 pg*h/mL.
145. The dry powder composition of clause 105. comprising about 240 µg of the compound of Formula (I), wherein R¹ is linear hexadecyl, and wherein upon once daily inhalation administration via a dry powder inhaler, provides a treprostinil AUC_{0-inf} from 80%-125% of a range of about 1540 pg*h/mL to about 2865 pg*h/mL.
146. The dry powder composition of clause 105. comprising about 320 µg of the compound of Formula (I), wherein R¹ is linear hexadecyl, and wherein upon once daily inhalation administration via a dry powder inhaler, provides a treprostinil AUC_{0-inf} ranging from about 1 130 pg*h/mL to about 5310 pg*h/mL.
147. The dry powder composition of clause 105. comprising about 320 µg of the compound of Formula (I), wherein R¹ is linear hexadecyl, and wherein upon once daily inhalation administration via a dry powder inhaler, provides a treprostinil AUC_{0-inf} from 80%-125% of a range from about 1400 pg*h/mL to about 4250 pg*h/mL.
148. The dry powder composition of clause 105. comprising about 320 µg of the compound of Formula (I), wherein R¹ is linear hexadecyl, and wherein upon once daily inhalation administration via a dry powder inhaler, provides a treprostinil AUC_{0-inf} from 80%-125% of a range of about 1975 pg*h/mL to about 3680 pg*h/mL.
149. The dry powder composition of clause 105. comprising about 400 µg of the compound of Formula (I), wherein R¹ is linear hexadecyl, and wherein upon once daily inhalation administration via a dry powder inhaler, provides a treprostinil AUC_{0-inf} ranging from about 1380 pg*h/mL to about 6480 pg*h/mL.
150. The dry powder composition of clause 105. comprising about 400 µg of the compound of Formula (I), wherein R¹ is linear hexadecyl, and wherein upon once daily inhalation administration via a dry powder inhaler, provides a treprostinil AUC_{0-inf} from 80%-125% of a range about of 1725 pg*h/mL to about 5180 pg*h/mL.
151. The dry powder composition of clause 105, comprising about 400 µg of the compound of Formula (I), wherein R¹ is linear hexadecyl, and wherein upon once daily inhalation administration via a dry powder inhaler, provides a treprostinil AUC_{0-inf} from 80%-125% of a range from about 2415pg*h/mL to about 4490 pg*h/mL.
152. The dry powder composition of clause 105, comprising about 450 µg of the compound of Formula (I), wherein R¹ is linear hexadecyl, and wherein upon once daily inhalation administration via a dry powder inhaler, provides a treprostinil AUC_{0-inf} (CV%) ranging from about 80% to about 125% of about 4040 (27.4) pg*h/mL.
153. The dry powder composition of clause 105. comprising about 480 µg of the compound of Formula (I), wherein R¹ is linear hexadecyl, and wherein upon once daily inhalation administration via a dry powder inhaler, provides a treprostinil AUC_{0-inf} ranging from about 1 630 pg*h/mL to about 7650 pg*h/mL.
154. The dry pow der composition of clause 105. comprising about 480 µg of the compound of Formula (I), wherein R¹ is linear hexadecyl, and wherein upon once daily inhalation administration via a dry pow der inhaler, provides a treprostinil AUC_{0-inf} from 80%-125% of a range from about 2040 pg*h/mL to about 6120 pg*h/mL.
155. The dry powder composition of clause 105. comprising about 480 µg of the compound of Formula (I), wherein R¹ is linear hexadecyl, and wherein upon once daily inhalation administration via a dry powder inhaler, provides a treprostinil AUC_{0-inf} from 80%-125% of a range from about 2855 pg*h/mL to about 5310 pg*h/mL.
156. The dry powder composition of clause 105. comprising about 640 µg of the compound of Formula (I), wherein R¹ is linear hexadecyl, and wherein upon once daily inhalation administration via a dry powder inhaler, provides a treprostinil AUC_{0-inf} ranging from about 2130 pg*h/mL to about 10000 pg*h/mL.
157. The dry powder composition of clause 105. comprising about 640 µg of the compound of Formula (I), wherein R¹ is linear hexadecyl, and wherein upon once daily inhalation administration via a dry powder inhaler, provides a treprostinil AUC_{0-inf} from 80%-125% of a range from about of 2650 pg*h/mL to about 8000 pg*h/mL.
158. The dry powder composition of clause 105, comprising about 640 µg of the compound of Formula (I), wherein R¹ is linear hexadecyl, and wherein upon once daily inhalation administration via a dry powder inhaler, provides a treprostinil AUC_{0-inf} from 80%-125% of a range from about 3730 to about 6935 pg*h/mL.
159. The dry powder composition of clause 105 comprising about 675 µg of the compound of Formula (I), wherein R¹ is linear hexadecyl, and wherein upon once daily inhalation administration via a dry powder inhaler, provides a treprostinil AUC_{0-inf} (CV%) ranging from about 80% to about 125% of about 5480 (13.8) pg*h/mL.
160. A method for treating pulmonary hypertension (PH) in a patient in need thereof, comprising administering to the patient, an effective amount of the dry powder composition of any one of clauses 1-159 to the lungs of the patient via inhalation with a dry powder inhaler (DPI), once daily during an administration period.
161. The method of clause 160. wherein administering comprises (i) aerosolizing the dry powder composition via the DPI to provide an aerosolized dry powder composition, and (ii) administering the aerosolized dry powder composition to the lungs of the patient via inhalation with the DPI.
162. The method of clause 160 or 161, wherein the effective amount of the dry powder composition comprises about 80 µg to about 675 µg of the compound of Formula (I).
163. The method of any one of clause 160-162, wherein the patient is administered two or more different dosages of the compound of Formula (I) during the administration period.
164. The method of clause 163, wherein the patient is administered two different dosages of the compound of Formula (I) during the administration period.
165. The method of clause 163, wherein the patient is administered three different dosages of the compound of Formula (I) during the administration period.
166. The method of clause 163, wherein the patient is administered four different dosages of the compound of Formula (I) during the administration period.
167. The method of clause 163, wherein the patient is administered five different dosages of the compound of Formula (I) during the administration period.
168. The method of any one of clauses 163-167, wherein prior to receiving a higher dosage of the compound of Formula (I), or a pharmaceutically acceptable salt thereof, the patient is administered a lower dosage for two or more consecutive days.
169. The method of any one of clauses 163-167, wherein prior to receiving a higher dosage of the compound of Formula (I), or a pharmaceutically acceptable salt thereof, the patient is administered a lower dosage for three or more consecutive days.
170. The method of any one of clauses 160-169, wherein the PH is group 1 PH. as classified by the World Health Organization (WHO).
171. The method of any one of clauses 160-169, wherein the PH is group 2 PH, as classified bv the WHO.
172. The method of any one of clauses 160-169, wherein the PH is group 3 PH, as classified bv the WHO.
173. The method of any one of clauses 160-169, wherein the PH is group 4 PH. as classified bv the WHO.
174. The method of any one of clauses 160-169, wherein the PH is group 5 PH, as classified bv the WHO.
175. The method of any one of clauses 160-169. wherein the PH is pulmonary arterial hypertension (PAH).
176. The method of clause 175, wherein the pulmonary arterial hypertension is class I pulmonary arterial hypertension, as characterized bv the New York Heart Association (NYHA).
177. The method of clause 175, wherein the pulmonary arterial hypertension is class II pulmonary arterial hypertension, as characterized by the NYHA.
178. The method of clause 175, wherein the pulmonary arterial hypertension is class III pulmonary arterial hypertension, as characterized by the NYHA.
179. The method of clause 175, wherein the pulmonary arterial hypertension is class IV pulmonary arterial hypertension, as characterized by the NYHA.
180. The method of clause 172. wherein the PH is portopulmonary hypertension (PPH).
181. The method of clause 172. wherein the PH is PH associated with interstitial lung (ILD),
disease
182. The method of clause 181, wherein the ILD comprises one or more lung conditions selected from the group consisting of idiopathic pulmonary fibrosis (IPF), cryptogenic organizing pneumonia (COP), desquamative interstitial pneumonitis, nonspecific interstitial pneumonitis, hypersensitivity pneumonitis, acute interstitial pneumonitis, interstitial pneumonia, connective tissue disease, sarcoidosis or asbestosis.
183. The method of clause 181. wherein the ILD is idiopathic interstitial pneumonia (IIP).
184. The method of clause181, wherein the ILD is sarcoidosis.
185. The method of clause 181, wherein the ILD is connective tissue disease-associated interstitial lung disease (CTD-ILD).
186. The method of clause 181. wherein the ILD is idiopathic pulmonary fibrosis (IPF).
187. The method of any one of clauses 160-186, wherein treating comprises reducing the pulmonary vascular index (PVRI) of the patient during the administration period, compared to the patient's PVRI prior to the administration period.
188. The method of any one of clauses 160-187, wherein treating comprises reducing the mean pulmonary artery pressure of the patient during the administration period, compared to the mean pulmonary artery pressure of the patient prior to the administration period.
189. The method of any one of clauses 160-188, wherein treating comprises increasing hypoxemia score of the patient during the hypoxemia score of the patient prior to the administration period.
190. The method of any one of clauses 160-189, wherein decreasing the oxygenation index of the patient during the administration period, compared to the oxygenation index of the patient prior to the administration period.
191. The method of any one of clauses 160-190, wherein treating comprises improving the right heart function of the patient during the administration period, compared to the right heart function of the patient prior to the administration period.
192. The method of any one of clauses 160-191, wherein treating comprises improving exercise capacity of the patient during the administration period, compared to the exercise capacity of the patient prior to the administration period.
193. The method of clause 192. wherein exercise capacity is measured by the six-minute walk test (6MWT).
194. The method of clause 193, wherein improving exercise capacity comprises increasing the patient's distance walked in the 6MWT by at least about 5 meters, at least about 10 meters, at least about 20 meters, at least about 30 meters, at least about 40 meters, or at least about 50 meters during the administration period, compared to the patient's distance walked in the 6MWT prior to the administration period.
195. The method of clause 193. wherein improving exercise capacity comprises increasing the patient's distance walked in the 6MWT by from about 5 meters to about 60 meters, by from about 5 meters to about 50 meters, by from about 10 meters to about 50 meters, by from about 15 meters to about 50 meters, or by from about 20 meters to about 40 meters, during the administration period, compared to the patient's distance walked in the 6MWT prior to the administration period.
196. The method of anv one of clauses 160-195, wherein treating comprises improving the quality of life of the patient during the administration period, compared to the quality of life of the patient prior to the administration period.
197. The method of clause 196, wherein the quality of life of the patient is measured by the Cambridge Pulmonary Hypertension Outcome Review (CAMPHOR) Questionnaire.
198. The method of clause 197, wherein treating comprises decreasing the patient's CAMPHOR Questionnaire score during the administration period by from 1 to about 10, from 1 to about 9, from 1 to 8, from 1 to 7, from 1 to 6, from 1 to 5, from 1 to 4, from 1 to 3 or from 1 to 2, compared to the patient's CAMPHOR Questionnaire score prior to the administration period.
199. The method of any one of clauses 160-198, wherein treating comprises increasing the patient's saturation of peripheral capillary oxygenation (SpO₂) at rest assessed by pulse oximetry, during the administration period, compared to the patient's SpO₂ at rest assessed by pulse oximetry prior to the administration period.
200. The method of any one of clauses 160-198, wherein treating comprises improving the patient's lung function during the administration period, as compared to the patient's lung function prior to the administration period.
201. The method of clause 200. wherein improving the patient's lung function comprises increasing the patient's forced vital capacity (FVC) during the administration period, compared to the patient's PVC prior to the administration period.
202. The method of clause 200. wherein improving the patient's lung function comprises increasing the patient's percent predicted forced vital capacity (ppFVC) during the administration period, compared to the patient's ppFVC prior to the administration period.
203. The method of clause 200. wherein improving the patient's lung function comprises increasing the patient's the patient's forced expiratory volume in 1 second (FEV₁) during the administration period, compared to the patient's FEV₁ prior to the administration period.
204. The method of clause 203, wherein increasing the patient's the patient's forced expiratory volume in 1 second (FEV₁) during the administration period comprises increasing the patient's FEV1 by from about 5% to about 50%, from about 5% to about 40% or from about 5% to about 30%, compared to the patient's FEV₁ prior to the administration period.
205. The method of clause 203, wherein increasing the FEV₁ comprises increasing by 5%, about 10%, about 15%, by about 20%, by about 25%, by about 30%, by about 35%, by about 40%, by about 45% or by about 50%.
206. The method of clause 203, wherein increasing the FEV₁ comprises increasing by at least about 5%, at least about 10%, at least about 15%, by at least about 20%, by at least about 25%, by at least about 30%, by at least about 35%, by at least about 40%, by at least about 45% or by at least about 50%.
207. The method of clause 203, wherein increasing the FEV₁ comprises increasing by about 5% to about 50%, by about 5% to about 40%, by about 5% to about 30%, by about 5% to about 20%, by about 10% to about 50%, by about 15% to about 50%, by about 20% to about 50% or by about 25% to about 50%.
208. The method of clause 203, wherein increasing the FEV₁ comprises increasing by of at least about 5%.
209. The method of clause 203. wherein increasing the FEV₁ comprises increasing by from about 5% to about 50%, or from about 10% to about 50%, or from about 15% to about 50%.
210. The method of clause 203, wherein increasing the FEV₁ comprises increasing by about 25 mL to about 500 mL.
211. The method of clause 203, wherein increasing the FEV₁ comprises increasing by about 25 mL to about 250 mL.
212. The method of clause 201. wherein increasing the patient's forced vital capacity (FVC) during the administration period comprises increasing the patient's FVC by about 1%, by about 2%, by about 3%, by about 4%, by about 5%, by about 6%, by about 7%, by about 8%, by about 9%, by about 10%, by about 11%, by about 12%, by about 13%, by about 14%, by about 15%, by about 16%, by about 17%, by about 18%, by about 19%, by about 20%, by about 25%, by about 30%, by about 35%, by about 40%, by about 45%, by about 50%, by about 55%, by about 60%, by about 65%, by about 70%, by about 75%, by about 80%, by about 85% or by about 90%, as compared to a FVC of the patient prior to the administration period.
213. The method of clause 201, wherein increasing the patient's forced vital capacity (FVC) during the administration period comprises increasing the patient's FVC by from about 1% to about 20%, from about 1% to about 15%, from about 1% to about 10%, from about 1% to about 5%, from about 5% to about 50%, from about 5% to about 40%, from about 5% to about 30%, from about 5% to about 20%, from about 10% to about 50%, from about 15% to about 50%, from about 20% to about 50%, or from about 25% to about 50%, as compared to a FVC of the patient prior to the administration period.
214. The method of clause 200, wherein improving the patient's lung function comprises increasing the patient's total lung capacity (TLC) during the administration period, compared to the patient's TLC prior to the administration period.
215. The method of clause 214. wherein increasing the patient's total lung capacity (TLC) comprises increasing the patient's TLC by at least about 1%, at least about 2%, at least about 5%, at least about 10%, at least about 15%, at least about 20%, at least about 25%, at least about 30%, at least about 35%, at least about 40%, at least about 45%, or bv at least about 50% during the administration period, compared to the patient's TLC prior to the administration period.
216. The method of clause 214. wherein increasing the patient's total lung capacity (TLC) comprises increasing the patient s TLC by from about 1% to about 50%, by from about 5% to about 50%, by from about 5% to about 40%, by from about 5% to about 30%, by from about 5% to about 20%, by from about 10% to about 50%, bv from about 15% to about 50%, bv from about 20% to about 50%, or by from about 25% to about 50%during the administration period, compared to the patient's TLC prior to the administration period.
217. The method of any one of clauses 160-216, wherein the administration period is from about 1 year to about 30 years.
218. The method of clause 217. wherein the administration period is from about 1 year to about 25 years.
219. The method of clause 217. wherein the administration period is from about 5 years to about 30 years.
220. The method of clause 217. wherein the administration period is from about 1 year to about 2.0 years.
221. The method of clause 217. wherein the administration period is from about 1 year to about 15 years.
222. The method of clause 217, wherein the administration period is from about 1 year to about 10 years.
223. The method of clause 217, wherein the administration period is from about 1 year to about 5 years.
224. The method of any one of clauses 160-223, wherein the dry powder inhaler (DPI) is a capsule-based DPI, and the composition is present in a single DPI capsule.
225. The method of any one of clauses 160-223. wherein the dry powder inhaler (DPI) is a capsule-based DPI, and the composition is split between two DPI capsules.

## Claims

1. A dry powder composition comprising:
(a) from about 0.1 wt% to about 5 wt% of a compound of Formula (1): 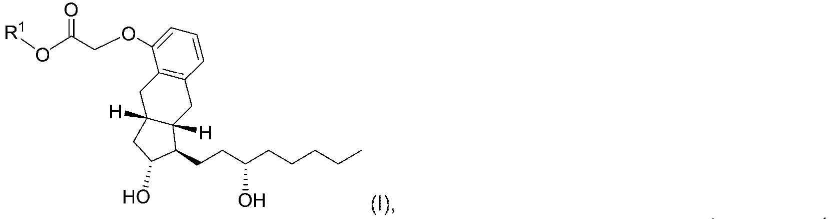 or an enantiomer, diastereomer, or a pharmaceutically acceptable salt thereof, wherein R¹ is tetradecyl, pentadecyl, hexadecyl, heptadecyl, or octadecyl,
(b) from about 25 wt% to about 61 wt% of leucine, and
(c) a sugar;
wherein the dry powder composition does not comprise DSPE-PEG2000.

2. The dry powder composition of claim 1, wherein R¹ is hexadecyl.

3. The dry powder composition of claim 2, wherein R¹ is linear hexadecyl.

4. The dry powder composition of any one of claims 1-3, wherein the compound of Formula (I), or an enantiomer, diastereomer, or a pharmaceutically acceptable salt thereof is present at about 0.5 wt%, 1.0 wt%, 1.5 wt%, 2.0 wt%, 2.5 wt%, 3.0 wt%, 3.5 wt%, or 4.0 wt% of the total weight of the dry powder composition.

5. The dry powder composition of any one of claims 1-4, comprising about 80 µg, about 160 µg, about 240 µg, about 320 µg, about 480 µg, or about 640 µg of the compound of Formula (1).

6. The dry powder composition of any one of claims 1-5, wherein the leucine is present at from about 45 wt% to about 61 wt% of the total weight of the dry powder composition.

7. The dry powder composition of any one of claims 1-6, wherein the leucine is present at about 55 wt% of the total weight of the dry powder composition.

8. The dry powder composition of any one of claims 1-7, wherein the sugar is selected from trehalose and mannitol.

9. The dry powder composition of claim 8, wherein the sugar is mannitol.

10. The dry powder composition of any one of claims 1-9, wherein the leucine is L-leucine.

11. The dry powder composition of any one of claims 1-10, wherein the dry powder composition is spray dried.

12. The dry powder composition of any one of claims 1-11, wherein the dry powder composition is in a capsule.

13. The dry powder composition of any one of claims 1-12, for use as a medicament.

14. The dry powder composition for the use of claim 13, wherein the dry powder composition is delivered to the lungs of a subject via inhalation using a dry powder inhaler (DPI).

15. The dry powder composition for the use of claim 13 or 14, wherein the dry powder composition is in the form of an aerosol comprising particles.

16. A method of preparing a dry powder composition according to any one of claims 1-10, wherein the method comprises spray drying a spray drying feed solution, wherein the spray drying feed solution comprises: i) a stock solution of the compound of Formula (I), or the enantiomer, diastereomer, or pharmaceutically acceptable salt thereof, and an organic solvent; ii) an aqueous stock solution of the sugar; iii) an aqueous stock solution of the leucine; and iv) a mixture of water and organic solvent.
